# EUROPEAN PATENT APPLICATION

(11) **EP 4 589 022 A1**
(43) Date of publication of application: **23.07.2025**
(21) Application number: 24152904.9
(22) Date of filing: 19.01.2024
(51) Int. Cl.: C12Q 1/6886

(54) **COMPOSITIONS AND METHODS RELATING TO COLORECTAL CANCER**

(71) Applicant: Royal College of Surgeons in Ireland, Dublin 2 (IE); University College Dublin, Belfield, Dublin 4 (IE)
(72) Inventor: Das, Sudipto, Dublin, Dublin 2 (IE); Moran, Bruce, Dublin, Dublin 4 (IE); O Connor, Darran, Dublin, Dublin 2 (IE); Finnegan, Ellen, Dublin, Dublin 2 (IE)
(74) Representative: Murgitroyd & Company

(57) **Abstract**

The present invention relates to diagnostic and prognostic methods and their use in diagnosing or predicting disease progression in a subject with colorectal cancer (CRC). More particularly, the present invention relates to a gene expression signature and the use thereof in determining the likelihood of progression of CRC to metastatic CRC (mCRC) in a subject, as well as compositions for the detection thereof. The present invention also extends to clinical markers and compositions for use in the treatment and/or prognosis of CRC and in particular mCRC.

## Description

### Field of the invention

The present invention relates to diagnostic and prognostic methods and their use in diagnosing or predicting disease progression in a subject with colorectal cancer (CRC). More particularly, the present invention relates to a gene expression signature and the use thereof in determining the likelihood of progression of CRC to metastatic CRC (mCRC) in a subject, as well as compositions for the detection thereof. The present invention also extends to clinical markers and compositions for use in the treatment and/or prognosis of CRC and in particular mCRC.

### Background to the Invention

Colorectal cancer is a disease in which cells in the colon or rectum grow out of control. Colorectal cancer often begins as a growth called a polyp inside the colon or rectum. Colorectal cancer (CRC) is the leading cause of cancer related deaths globally, when excluding gender-specific cancer types [1]. While survival rates of early stage (stage I-III) CRC are high (-50-80%), the 5-year rate of survival in patients with metastatic colorectal cancer (mCRC) is only 14% [2]. These low survival rates necessitate identification of novel diagnostic and therapeutic strategies that can allow better diagnosis and treatment of these mCRC patients.

It is widely reported that up to 50% of CRC patients develop metastases over the course of the disease. However, there is no definitive approach to predict if a CRC patient will develop metastases. In addition to this, from a treatment perspective only 10-20% of mCRC patients can be treated with surgery and chemotherapy with a likelihood of cure in some cases. This is worsened by the fact that mCRC patients who exhibit disease progression, despite treatment with conventional chemotherapeutic regimes such as FOLFOX, FOLFIRI and/or targeted biologic treatment, have refractory disease and thus further reduced survival rates [6-9].

Numerous studies in CRC and mCRC have reported that changes in DNA often impact disease progression and thus should be further investigated to establish their potential diagnostic/therapeutic role. One such change in DNA involves a chemical modification called "DNA methylation". DNA methylation is a biological process by which methyl groups are added to the DNA molecule. Methylation can change the activity of a DNA segment without changing the sequence. Although DNA methylation does not change the structure of the DNA, it can alter the information that the DNA codes for. This ultimately leads to aberrant switching on/off of genes that accelerate or stop tumour growth and progression.

DNA methylation alteration is a well-known epigenetic alteration associated with CRC. This is evidenced by the fact that CpG island methylator phenotype (CIMP) is a standard diagnostic assessment carried out for CRC patients with strong association with outcome in combination with microsatellite instability. However, the majority of studies involving DNA methylation in CRC limit the presence and impact of these alterations to conventional regulatory regions, e.g. promoters associated with genes. Moreover, recent studies demonstrate that alternative regulatory regions such as enhancers might in fact have a more profound role in mediating epigenetic regulation in the CRC epigenome than promoters. However, as such there is no information regarding the epigenetic state of alternative regulatory regions in mCRC. More importantly, from a disease progression perspective only limited routine diagnostic strategies allow prediction of CRC progression to metastasis.

The drastically poor survival rates together with a reduced response to standard of care treatment in mCRC patients presents a serious unmet clinical need that needs to be addressed urgently. In particular, there is a significant unmet clinical need to develop novel diagnostic and therapeutic strategies that can allow better diagnosis and treatment of mCRC patients. The development of a new diagnostic/prognostic assay would allow identification of CRC patients who are likely to progress in their disease. This would ultimately enable clinicians to introduce early interventions and save patients from treatment that would ultimately be ineffective and, together with the development of new treatments for CRC patients, would improve patient outcome and quality of life.

### Summary of Invention

Following extensive experimentation, the present inventors have surprisingly identified methods, and the use thereof, to predict disease progression in colorectal cancer (CRC) patients. More particularly, using a large cohort of metastatic colorectal cancer (mCRC) patients, the present inventors for the first time, have identified DNA methylation sites in DNA extracted from patients with CRC where tumour specific DNA methylation occurred and that are linked to genes that when turned on lead to CRC disease progression. Disease progression can be determined as colorectal cancer that has metastasised or that has a likelihood of metastasising. The DNA methylation signature of the present invention, or a subset thereof, demonstrates a high potential of predicting disease spread in CRC patients and in parallel certain genes can be used as therapeutic targets.

According to a first aspect of the present invention, there is provided a method for predicting the progression of colorectal cancer in a subject and/or screening for an increased likelihood of progression of colorectal cancer in a subject, the method comprising:
- providing a biological sample which has been obtained from the subject;
- testing the biological sample for the presence or absence of a gene expression signature, or a subset thereof;

wherein the gene expression signature, or a subset thereof, comprises or consists of one or more of the differentially methylated regions set forth in Table 1;
wherein the presence of the gene expression signature, or a subset thereof, indicates an increased likelihood of disease progression.

In embodiments, the one or more differentially methylated regions comprise or consist of the nucleic acid sequences of SEQ ID NO:1 to SEQ ID NO:367 or a functionally active fragment or variant thereof; or any combination thereof.

In embodiments, the one or more differentially methylated regions are hypomethylated.

In embodiments, the gene expression signature comprises or consists of one or more differentially methylated regions selected from the group comprising or consisting of DMR_240, DMR_236, DMR_148, DMR_276, DMR_33, DMR_312, DMR_302, DMR_75 or DMR_334 or any combination thereof.

In embodiments, the differentially methylated region DMR_240 comprises or consists of the nucleic acid sequence of SEQ ID NO:240 or a functionally active fragment or variant thereof. In embodiments, the differentially methylated region DMR_236 comprises or consists of the nucleic acid sequence of SEQ ID NO:236 or a functionally active fragment or variant thereof. In embodiments, the differentially methylated region DMR_148 comprises or consists of the nucleic acid sequence of SEQ ID NO:148 or a functionally active fragment or variant thereof. In embodiments, the differentially methylated region DMR_276 comprises or consists of the nucleic acid sequence of SEQ ID NO:276 or a functionally active fragment or variant thereof. In embodiments, the differentially methylated region DMR_33 comprises or consists of the nucleic acid sequence of SEQ ID NO:33 or a functionally active fragment or variant thereof. In embodiments, the differentially methylated region DMR_312 comprises or consists of the nucleic acid sequence of SEQ ID NO:312 or a functionally active fragment or variant thereof. In embodiments, the differentially methylated region DMR_302 comprises or consists of the nucleic acid sequence of SEQ ID NO:302 or a functionally active fragment or variant thereof. In embodiments, the differentially methylated region DMR_75 comprises or consists of the nucleic acid sequence of SEQ ID NO:75 or a functionally active fragment or variant thereof. In embodiments, the differentially methylated region DMR_334 comprises or consists of the nucleic acid sequence of SEQ ID NO:334 or a functionally active fragment or variant thereof.

In embodiments, the one or more differentially methylated region is defined by the coordinates chr3:32010001-32010500 (DMR_240), chr3:190041001-190041500 (DMR_236), chr17:39112501-39113000 (DMR_148), chr5:132576001-132576500 (DMR_276), chr10:11645501-11646000 (DMR_33), chr7:27147001-27147500 (DMR_312), chr7:130645001-130645500 (DMR_302), chr12:76035001-76035500 (DMR_75) or chr8:128235001-128235500 (DMR_334).

In embodiments, the one or more differentially methylated region is DMR_302. In embodiments, the differentially methylated region DMR_302 comprises or consists of the nucleic acid sequence of SEQ ID NO:302 or a functionally active fragment or variant thereof. In embodiments, the one or more differentially methylated region is defined by the coordinates chr7:130645001-130645500 (DMR_302).

In embodiments, the one or more differentially methylated region is DMR_75. In embodiments, the differentially methylated region DMR_75 comprises or consists of the nucleic acid sequence of SEQ ID NO:75 or a functionally active fragment or variant thereof. In embodiments, the one or more differentially methylated region is defined by the coordinates chr12:76035001-76035500 (DMR_75).

In embodiments, the one or more differentially methylated region is DMR_312. In embodiments, the differentially methylated region DMR_312 comprises or consists of the nucleic acid sequence of SEQ ID NO:312 or a functionally active fragment or variant thereof. In embodiments, the one or more differentially methylated region is defined by the coordinates chr7:27147001-27147500 (DMR_312).

In embodiments, there are three differentially methylated regions. In embodiments, the three differentially methylated regions are DMR_312, DMR_302 and DMR_75. In embodiments, there are two differentially methylated regions. In embodiments, the two differentially methylated regions are DMR_302 and DMR_75. In embodiments, the two differentially methylated regions are DMR_312 and DMR_75. In embodiments, the two differentially methylated regions are DMR_312 and DMR_302. In embodiments, the differentially methylated region DMR_302 comprises or consists of the nucleic acid sequence of SEQ ID NO:302 or a functionally active fragment or variant thereof. In embodiments, the differentially methylated region DMR_75 comprises or consists of the nucleic acid sequence of SEQ ID NO:75 or a functionally active fragment or variant thereof. In embodiments, the differentially methylated region DMR_312 comprises or consists of the nucleic acid sequence of SEQ ID NO:312 or a functionally active fragment or variant thereof. In embodiments, the one or more differentially methylated region is defined by the coordinates chr7:27147001-27147500 (DMR_312), chr7:130645001-130645500 (DMR_302) and chr12:76035001-76035500 (DMR_75).

In embodiments, the one or more differentially methylated regions comprise or consist of DMR_240, DMR_236, DMR_148, DMR_276 and/or DMR_33 or combinations thereof. In embodiments, the differentially methylated region DMR_240 comprises or consists of the nucleic acid sequence of SEQ ID NO:240 or a functionally active fragment or variant thereof. In embodiments, the differentially methylated region DMR_236 comprises or consists of the nucleic acid sequence of SEQ ID NO:236 or a functionally active fragment or variant thereof. In embodiments, the differentially methylated region DMR_148 comprises or consists of the nucleic acid sequence of SEQ ID NO:148 or a functionally active fragment or variant thereof.

In embodiments, the differentially methylated region DMR_276 comprises or consists of the nucleic acid sequence of SEQ ID NO:276 or a functionally active fragment or variant thereof. In embodiments, the differentially methylated region DMR_33 comprises or consists of the nucleic acid sequence of SEQ ID NO:33 or a functionally active fragment or variant thereof. In embodiments, the one or more differentially methylated region is defined by the coordinates chr3:32010001-32010500 (DMR_240), chr3:190041001-190041500 (DMR_236), chr17:39112501-39113000 (DMR_148), chr5:132576001-132576500 (DMR_276) and chr10:11645501-11646000 (DMR_33).

In embodiments, the one or more differentially methylated region is DMR_334. In embodiments, the differentially methylated region DMR_334 comprises or consists of the nucleic acid sequence of SEQ ID NO:334 or a functionally active fragment or variant thereof. In embodiments, the one or more differentially methylated region is defined by the coordinates chr8:128235001-128235500 (DMR_334).

In embodiments, the gene expression signature, or the subset thereof, of the present invention comprises or consists of at least 98%, at least 95%, at least 90%, at least 80%, at least 70%, at least 60%, at least 50%, at least 40%, at least 30%, at least 20%, at least 10%, at least 5% or at least 1% of the differentially methylated regions listed in Table 1. In embodiments, the gene expression signature, or a subset thereof, of the present invention comprises or consists of all of the hypomethylated differentially methylated regions in Table 1. In embodiments, the gene expression signature, or a subset thereof, of the present invention comprises or consists of all of the hypermethylated differentially methylated regions in Table 1.

**Table 1: Differentially Methylated Regions of the gene signature of the present invention.**

| **DMR_ID** | **SEQ ID NO** | **DMR _met hylati on** | **chr** | **start** | **end** | **coordinate** | **gene_wit hin** | **meth expre ss_co rrelat ed_ gene** | **correl ation** | **corre latio n_pv alue** |
|---|---|---|---|---|---|---|---|---|---|---|
| DMR_1 | SEQ ID NO: 1 | hyper | chr 1 | 147782 001 | 147782 500 | chr1:1477820 01-147782500 | RP11-495P10.4 | NA | NA | NA |
| DMR_2 | SEQ ID NO: 2 | hypo | chr 1 | 153540 001 | 153540 500 | chr1:1535400 01-153540500 | S100A2 | BX47 0102. 1 | -0.35 | 0.02 307 |
| DMR_3 | SEQ ID NO:3 | hypo | chr 1 | 153540 501 | 153541 000 | chr1:1535405 01-153541000 | S100A2 | NA | NA | NA |
| DMR_4 | SEQ ID NO: 4 | hypo | chr 1 | 169257 001 | 169257 500 | chr1:1692570 01-169257500 | NME7 | NA | NA | NA |
| DMR_5 | SEQ ID NO: 5 | hypo | chr 1 | 171973 501 | 171974 000 | chr1:1719735 01-171974000 | DNM3 | NA | NA | NA |
| DMR_6 | SEQ ID NO: 6 | hypo | chr 1 | 186464 501 | 186465 000 | chr1:1864645 01-186465000 | GS1-304P7.3 | NA | NA | NA |
| DMR_7 | SEQ ID NO: 7 | hypo | chr 1 | 199728 001 | 199728 500 | chr1:1997280 01-199728500 | RP11-2L13.1 | NA | NA | NA |
| DMR_8 | SEQ ID NO: 8 | hypo | chr 1 | 207921 001 | 207921 500 | chr1:2079210 01-207921500 | CD46 | NA | NA | NA |
| DMR_9 | SEQ ID NO: 9 | hypo | chr 1 | 207923 001 | 207923 500 | chr1:2079230 01-207923500 | CD46 | CD46 | - 0.413 | 0.00 658 |
| DMR_10 | SEQ ID NO: 10 | hypo | chr 1 | 210619 501 | 210620 000 | chr1:2106195 01-210620000 | HHAT | NA | NA | NA |
| DMR_11 | SEQ ID NO: 11 | hypo | chr 1 | 215756 501 | 215757 000 | chr1:2157565 01-215757000 | KCTD3 | NA | NA | NA |
| DMR_12 | SEQ ID NO: 12 | hypo | chr 1 | 219498 001 | 219498 500 | chrl:2194980 01-219498500 | RP11-392017. 1 | NA | NA | NA |
| DMR_13 | SEQ ID NO: 13 | hypo | chr 1 | 234700 501 | 234701 000 | chrl:2347005 01-234701000 | RP5-855F14.2 | NA | NA | NA |
| DMR_14 | SEQ ID NO: 14 | hypo | chr 1 | 234754 501 | 234755 000 | chr1:2347545 01-234755000 | IRF2BP2 | NA | NA | NA |
| DMR_15 | SEQ ID NO: 15 | hypo | chr 1 | 235493 501 | 235494 000 | chr1:2354935 01-235494000 | GGPS1 | NA | NA | NA |
| DMR_16 | SEQ ID NO: 16 | hypo | chr 1 | 241376 501 | 241377 000 | chr1:2413765 01-241377000 | RGS7 | NA | NA | NA |
| DMR_17 | SEQ ID NO: 17 | hyper | chr 1 | 244875 01 | 244880 00 | chrl:2448750 1-24488000 | IFNLR1 | NA | NA | NA |
| DMR_18 | SEQ ID NO: 18 | hyper | chr 1 | 252580 01 | 252585 00 | chr1:2525800 1-25258500 | RUNX3 | NA | NA | NA |
| DMR_19 | SEQ ID NO: 19 | hypo | chr 1 | 405730 01 | 405735 00 | chr1:4057300 1-40573500 | PPT1 | NA | NA | NA |
| DMR_20 | SEQ ID NO: 20 | hypo | chr 1 | 434160 01 | 434165 00 | chrl:4341600 1-43416500 | SLC2A1 | NA | NA | NA |
| DMR_21 | SEQ ID NO: 21 | hypo | chr 1 | 447930 01 | 447935 00 | chr1:4479300 1-44793500 | ERI3 | NA | NA | NA |
| DMR_22 | SEQ ID NO: 22 | hypo | chr 1 | 472950 1 | 473000 0 | chr1:4729501-4730000 | AJAP1 | NA | NA | NA |
| DMR_23 | SEQ ID NO: 23 | hyper | chr 1 | 479075 01 | 479080 00 | chrl:4790750 1-47908000 | FOXD2 | FOXD 2-AS1 | - 0.377 | 0.01 396 |
| DMR_24 | SEQ ID NO: 24 | hyper | chr 1 | 479080 01 | 479085 00 | chrl:4790800 1-47908500 | FOXD2 | NA | NA | NA |
| DMR_25 | SEQ ID NO: 25 | hyper | chr 1 | 479085 01 | 479090 00 | chrl:4790850 1-47909000 | FOXD2 | NA | NA | NA |
| DMR_26 | SEQ ID NO: 26 | hyper | chr 1 | 479090 01 | 479095 00 | chrl:4790900 1-47909500 | FOXD2 | NA | NA | NA |
| DMR_27 | SEQ ID NO: 27 | hyper | chr 1 | 479095 01 | 479100 00 | chrl:4790950 1-47910000 | FOXD2 | LINC0 1389 | 0.348 | 0.02 398 |
| DMR_28 | SEQ ID NO: 28 | hyper | chr 1 | 479110 01 | 479115 00 | chr1:4791100 1-47911500 | FOXD2 | FOXD 2-AS1 | 0.328 | 0.03 401 |
| DMR_29 | SEQ ID NO: 29 | hyper | chr 1 | 479115 01 | 479120 00 | chr1:4791150 1-47912000 | FOXD2 | FOXD 2 | 0.308 | 0.04 73 |
| DMR_30 | SEQ ID NO: 30 | hypo | chr 1 | 558950 01 | 558955 00 | chr1:5589500 1-55895500 | RNU6-830P | NA | NA | NA |
| DMR_31 | SEQ ID NO: 31 | hypo | chr 10 | 115718 001 | 115718 500 | chr10:115718 001-115718500 | NHLRC2 | NA | NA | NA |
| DMR_32 | SEQ ID NO: 32 | hypo | chr 10 | 116455 01 | 116460 00 | chr10:116455 01-11646000 | USP6NL | USP6 NL | - 0.553 | 0.00 015 |
| DMR_33 | SEQ ID NO: 33 | hypo | chr 10 | 124905 01 | 124910 00 | chr10:124905 01-12491000 | CAMK1D | NA | NA | NA |
| DMR_34 | SEQ ID NO: 34 | hyper | chr 10 | 131768 001 | 131768 500 | chr10:131768 001-131768500 | EBF3 | NA | NA | NA |
| DMR_35 | SEQ ID NO: 35 | hypo | chr 10 | 172580 01 | 172585 00 | chr10:172580 01-17258500 | VIM-AS1 | NA | NA | NA |
| DMR_36 | SEQ ID NO: 36 | hyper | chr 10 | 225425 01 | 225430 00 | chr10:225425 01-22543000 | RP11-573G6.6 | NA | NA | NA |
| DMR_37 | SEQ ID NO: 37 | hyper | chr 10 | 227660 01 | 227665 00 | chr10:227660 01-22766500 | SPAG6 | NA | NA | NA |
| DMR_38 | SEQ ID NO: 38 | hypo | chr 10 | 332505 01 | 332510 00 | chr10:332505 01-33251000 | RP11-462L8.1 | NA | NA | NA |
| DMR_39 | SEQ ID NO: 39 | hypo | chr 10 | 332510 01 | 332515 00 | chr10:332510 01-33251500 | RP11-462L8.1 | NA | NA | NA |
| DMR_40 | SEQ ID NO: 40 | hypo | chr 10 | 348170 01 | 348175 00 | chr10:348170 01-34817500 | PARD3 | NA | NA | NA |
| DMR_41 | SEQ ID NO: 41 | hypo | chr 10 | 378250 1 | 378300 0 | chr10:378250 1-3783000 | RP11-184A2.3 | NA | NA | NA |
| DMR_42 | SEQ ID NO: 42 | hypo | chr 10 | 379700 1 | 379750 0 | chr10:379700 1-3797500 | RP11-184A2.3 | NA | NA | NA |
| DMR_43 | SEQ ID NO: 43 | hypo | chr 10 | 597390 01 | 597395 00 | chr10:597390 01-59739500 | MRPS35 P3 | NA | NA | NA |
| DMR_44 | SEQ ID NO: 44 | hypo | chr 10 | 605805 01 | 605810 00 | chr10:605805 01-60581000 | BICC1 | NA | NA | NA |
| DMR_45 | SEQ ID NO: 45 | hypo | chr 10 | 605810 01 | 605815 00 | chr10:605810 01-60581500 | BICC1 | NA | NA | NA |
| DMR_46 | SEQ ID NO: 46 | hypo | chr 10 | 607540 01 | 607545 00 | chr10:607540 01-60754500 | LINC0084 4 | NA | NA | NA |
| DMR_47 | SEQ ID NO: 47 | hypo | chr 10 | 620430 01 | 620435 00 | chr10:620430 01-62043500 | ANK3 | NA | NA | NA |
| DMR_48 | SEQ ID NO: 48 | hypo | chr 10 | 720350 1 | 720400 0 | chr10:720350 1-7204000 | SFMBT2 | NA | NA | NA |
| DMR_49 | SEQ ID NO: 49 | hypo | chr 10 | 729950 1 | 730000 0 | chr10:729950 1-7300000 | SFMBT2 | NA | NA | NA |
| DMR_50 | SEQ ID NO: 50 | hypo | chr 10 | 901890 01 | 901895 00 | chr10:901890 01-90189500 | RNLS | NA | NA | NA |
| DMR_51 | SEQ ID NO: 51 | hypo | chr 10 | 919870 01 | 919875 00 | chr10:919870 01-91987500 | RN7SKP1 43 | NA | NA | NA |
| DMR_52 | SEQ ID NO: 52 | hypo | chr 11 | 101741 001 | 101741 500 | chr11:101741 001-101741500 | TRPC6 | NA | NA | NA |
| DMR_53 | SEQ ID NO: 53 | hypo | chr 11 | 104539 501 | 104540 000 | chr11:104539 501-104540000 | RP11-681H10. 1 | NA | NA | NA |
| DMR_54 | SEQ ID NO: 54 | hyper | chr 11 | 123301 501 | 123302 000 | chr11:123301 501-123302000 | AP00078 3.1 | NA | NA | NA |
| DMR_55 | SEQ ID NO: 55 | hypo | chr 11 | 126920 001 | 126920 500 | chr11:126920 001-126920500 | RP11-168K9.1 | NA | NA | NA |
| DMR_56 | SEQ ID NO: 56 | hyper | chr 11 | 128562 501 | 128563 000 | chr11:128562 501-128563000 | FLI1 | FLI1 | -0.41 | 0.00 699 |
| DMR_57 | SEQ ID NO: 57 | hypo | chr 11 | 206870 01 | 206875 00 | chr11:206870 01-20687500 | NA | NA | NA | NA |
| DMR_58 | SEQ ID NO: 58 | hypo | chr 11 | 222175 01 | 222180 00 | chr11:222175 01-22218000 | ANO5 | NA | NA | NA |
| DMR_59 | SEQ ID NO: 59 | hypo | chr 11 | 347070 01 | 347075 00 | chr11:347070 01-34707500 | RP11-350D17. 3 | NA | NA | NA |
| DMR_60 | SEQ ID NO: 60 | hypo | chr 11 | 762050 01 | 762055 00 | chr11:762050 01-76205500 | C11orf30 | NA | NA | NA |
| DMR_61 | SEQ ID NO: 61 | hypo | chr 11 | 856010 01 | 856015 00 | chr11:856010 01-85601500 | CCDC83 | NA | NA | NA |
| DMR_62 | SEQ ID NO: 62 | hypo | chr 11 | 942750 01 | 942755 00 | chr11:942750 01-94275500 | NA | NA | NA | NA |
| DMR_63 | SEQ ID NO: 63 | hypo | chr 11 | 950325 01 | 950330 00 | chr11:950325 01-95033000 | RP11-712B9.2 | NA | NA | NA |
| DMR_64 | SEQ ID NO: 64 | hypo | chr 11 | 984320 01 | 984325 00 | chr11:984320 01-98432500 | CTD-2342I9.1 | NA | NA | NA |
| DMR_65 | SEQ ID NO: 65 | hypo | chr 12 | 100889 001 | 100889 500 | chr12:100889 001-100889500 | NR1H4 | NA | NA | NA |
| DMR_66 | SEQ ID NO: 66 | hyper | chr 12 | 102177 501 | 102178 000 | chr12:102177 501-102178000 | GNPTAB | NA | NA | NA |
| DMR_67 | SEQ ID NO: 67 | hyper | chr 12 | 124448 001 | 124448 500 | chr12:124448 001-124448500 | CCDC92 | NA | NA | NA |
| DMR_68 | SEQ ID NO: 68 | hyper | chr 12 | 124448 501 | 124449 000 | chr12:124448 501-124449000 | CCDC92 | NA | NA | NA |
| DMR_69 | SEQ ID NO: 69 | hypo | chr 12 | 125073 501 | 125074 000 | chr12:125073 501-125074000 | NCOR2 | NA | NA | NA |
| DMR_70 | SEQ ID NO: 70 | hypo | chr 12 | 125118 001 | 125118 500 | chr12:125118 001-125118500 | NCOR2 | NA | NA | NA |
| DMR_71 | SEQ ID NO: 71 | hypo | chr 12 | 613145 01 | 613150 00 | chr12:613145 01-61315000 | RP11-471N19. 1 | NA | NA | NA |
| DMR_72 | SEQ ID NO: 72 | hypo | chr 12 | 659265 01 | 659270 00 | chr12:659265 01-65927000 | RP11-230G5.2 | NA | NA | NA |
| DMR_73 | SEQ ID NO: 73 | hypo | chr 12 | 760290 01 | 760295 00 | chr12:760290 01-76029500 | RP11-114H23. 1 | NA | NA | NA |
| DMR_74 | SEQ ID NO: 74 | hypo | chr 12 | 760345 01 | 760350 00 | chr12:760345 01-76035000 | RP11-114H23. 1 | NA | NA | NA |
| DMR_75 | SEQ ID NO: 75 | hypo | chr 12 | 760350 01 | 760355 00 | chr12:760350 01-76035500 | RP11-114H23. 1 | NA | NA | NA |
| DMR_76 | SEQ ID NO: 76 | hypo | chr 12 | 831340 01 | 831345 00 | chr12:831340 01-83134500 | TMTC2 | NA | NA | NA |
| DMR_77 | SEQ ID NO: 77 | hypo | chr 12 | 851325 01 | 851330 00 | chr12:851325 01-85133000 | RP11-701B6.1 | NA | NA | NA |
| DMR_78 | SEQ ID NO: 78 | hypo | chr 12 | 897430 01 | 897435 00 | chr12:897430 01-89743500 | DUSP6 | NA | NA | NA |
| DMR_79 | SEQ ID NO: 79 | hypo | chr 12 | 937095 01 | 937100 00 | chr12:937095 01-93710000 | RP11-486A14.1 | AC12 4947. 1 | - 0.392 | 0.01 016 |
| DMR_80 | SEQ ID NO: 80 | hypo | chr 13 | 102103 001 | 102103 500 | chr13:102103 001-102103500 | NA | NA | NA | NA |
| DMR_81 | SEQ ID NO: 81 | hypo | chr 13 | 103045 501 | 103046 000 | chr13:103045 501-103046000 | FGF14 | NA | NA | NA |
| DMR_82 | SEQ ID NO: 82 | hypo | chr 13 | 104200 001 | 104200 500 | chr13:104200 001-104200500 | ATP6V1G 1P7 | NA | NA | NA |
| DMR_83 | SEQ ID NO: 83 | hypo | chr 13 | 105792 001 | 105792 500 | chr13:105792 001-105792500 | DAOA-AS1 | NA | NA | NA |
| DMR_84 | SEQ ID NO: 84 | hypo | chr 13 | 105792 501 | 105793 000 | chr13:105792 501-105793000 | DAOA-AS1 | NA | NA | NA |
| DMR_85 | SEQ ID NO: 85 | hypo | chr 13 | 111100 501 | 111101 000 | chr13:111100 501-111101000 | COL4A2 | NA | NA | NA |
| DMR_86 | SEQ ID NO: 86 | hypo | chr 13 | 241860 01 | 241865 00 | chr13:241860 01-24186500 | TNFRSF1 9 | NA | NA | NA |
| DMR_87 | SEQ ID NO: 87 | hypo | chr 13 | 247220 01 | 247225 00 | chr13:247220 01-24722500 | SPATA13 | NA | NA | NA |
| DMR_88 | SEQ ID NO: 88 | hypo | chr 13 | 257965 01 | 257970 00 | chr13:257965 01-25797000 | MTM R6 | NUP5 8 | - 0.341 | 0.02 708 |
| DMR_89 | SEQ ID NO: 89 | hypo | chr 13 | 274885 01 | 274890 00 | chr13:274885 01-27489000 | FGFR1OP 2P1 | NA | NA | NA |
| DMR_90 | SEQ ID NO: 90 | hypo | chr 13 | 287785 01 | 287790 00 | chr13:287785 01-28779000 | PAN3 | PAN3 | - 0.368 | 0.01 647 |
| DMR_91 | SEQ ID NO: 91 | hypo | chr 13 | 287790 01 | 287795 00 | chr13:287790 01-28779500 | PAN3 | PAN3 -AS1 | - 0.377 | 0.01 383 |
| DMR_92 | SEQ ID NO: 92 | hypo | chr 13 | 337000 01 | 337005 00 | chr13:337000 01-33700500 | STARD13 | NA | NA | NA |
| DMR_93 | SEQ ID NO: 93 | hypo | chr 13 | 340690 01 | 340695 00 | chr13:340690 01-34069500 | RP11-141M1.3 | NA | NA | NA |
| DMR_94 | SEQ ID NO: 94 | hypo | chr 13 | 345945 01 | 345950 00 | chr13:345945 01-34595000 | RFC3 | NA | NA | NA |
| DMR_95 | SEQ ID NO: 95 | hypo | chr 13 | 360550 01 | 360555 00 | chr13:360550 01-36055500 | NBEA | NA | NA | NA |
| DMR_96 | SEQ ID NO: 96 | hypo | chr 13 | 363440 01 | 363445 00 | chr13:363440 01-36344500 | DCLK1 | NA | NA | NA |
| DMR_97 | SEQ ID NO: 97 | hypo | chr 13 | 363445 01 | 363450 00 | chr13:363445 01-36345000 | DCLK1 | NA | NA | NA |
| DMR_98 | SEQ ID NO: 98 | hypo | chr 13 | 367325 01 | 367330 00 | chr13:367325 01-36733000 | NA | NA | NA | NA |
| DMR_99 | SEQ ID NO: 99 | hypo | chr 13 | 367330 01 | 367335 00 | chr13:367330 01-36733500 | NA | NA | NA | NA |
| DMR_100 | SEQ ID NO: 100 | hypo | chr 13 | 367425 01 | 367430 00 | chr13:367425 01-36743000 | CCDC169 -SOHLH2 | NA | NA | NA |
| DMR_101 | SEQ ID NO: 101 | hypo | chr 13 | 367865 01 | 367870 00 | chr13:367865 01-36787000 | NA | NA | NA | NA |
| DMR_102 | SEQ ID NO: 102 | hypo | chr 13 | 421845 01 | 421850 00 | chr13:421845 01-42185000 | VWA8 | NA | NA | NA |
| DMR_103 | SEQ ID NO: 103 | hypo | chr 13 | 422220 01 | 422225 00 | chr13:422220 01-42222500 | VWA8 | NA | NA | NA |
| DMR_104 | SEQ ID NO: 104 | hypo | chr 13 | 436330 01 | 436335 00 | chr13:436330 01-43633500 | DNAJC15 | EPSTI 1 | - 0.381 | 0.01 277 |
| DMR_105 | SEQ ID NO: 105 | hypo | chr 13 | 448170 01 | 448175 00 | chr13:448170 01-44817500 | RP11-478K15.6 | NA | NA | NA |
| DMR_106 | SEQ ID NO: 106 | hypo | chr 13 | 448175 01 | 448180 00 | chr13:448175 01-44818000 | RP11-478K15.6 | AL58 9745. 1 | - 0.308 | 0.04 723 |
| DMR_107 | SEQ ID NO: 107 | hypo | chr 13 | 448545 01 | 448550 00 | chr13:448545 01-44855000 | RP11-478K15.6 | SERP 2 | - 0.508 | 0.00 059 |
| DMR_108 | SEQ ID NO: 108 | hypo | chr 13 | 448550 01 | 448555 00 | chr13:448550 01-44855500 | RP11-478K15.6 | SERP 2 | - 0.389 | 0.01 085 |
| DMR_109 | SEQ ID NO: 109 | hypo | chr 13 | 448870 01 | 448875 00 | chr13:448870 01-44887500 | SERP2 | NA | NA | NA |
| DMR_110 | SEQ ID NO: 110 | hypo | chr 13 | 449385 01 | 449390 00 | chr13:449385 01-44939000 | SERP2 | SERP 2 | - 0.365 | 0.01 74 |
| DMR_111 | SEQ ID NO: 111 | hypo | chr 13 | 449390 01 | 449395 00 | chr13:449390 01-44939500 | SERP2 | NA | NA | NA |
| DMR_112 | SEQ ID NO: 112 | hyper | chr 13 | 607390 01 | 607395 00 | chr13:607390 01-60739500 | DIAPH3 | NA | NA | NA |
| DMR_113 | SEQ ID NO: 113 | hypo | chr 13 | 642330 01 | 642335 00 | chr13:642330 01-64233500 | LINC0039 5 | NA | NA | NA |
| DMR_114 | SEQ ID NO: 114 | hypo | chr 13 | 705620 01 | 705625 00 | chr13:705620 01-70562500 | KLHL1 | NA | NA | NA |
| DMR_115 | SEQ ID NO: 115 | hypo | chr 13 | 713855 01 | 713860 00 | chr13:713855 01-71386000 | SOGA2P1 | NA | NA | NA |
| DMR_116 | SEQ ID NO: 116 | hyper | chr 13 | 791710 01 | 791715 00 | chr13:791710 01-79171500 | RNF219-AS1 | RNF2 19 | 0.353 | 0.02 178 |
| DMR_117 | SEQ ID NO: 117 | hyper | chr 13 | 791820 01 | 791825 00 | chr13:791820 01-79182500 | RNF219-AS1 | RNF2 19 | 0.408 | 0.00 735 |
| DMR_118 | SEQ ID NO: 118 | hypo | chr 13 | 866250 01 | 866255 00 | chr13:866250 01-86625500 | RP11-30L8.1 | NA | NA | NA |
| DMR_119 | SEQ ID NO: 119 | hypo | chr 13 | 866255 01 | 866260 00 | chr13:866255 01-86626000 | RP11-30L8.1 | NA | NA | NA |
| DMR_120 | SEQ ID NO: 120 | hypo | chr 13 | 979085 01 | 979090 00 | chr13:979085 01-97909000 | MBNL2 | NA | NA | NA |
| DMR_121 | SEQ ID NO: 121 | hypo | chr 13 | 999665 01 | 999670 00 | chr13:999665 01-99967000 | UBAC2 | NA | NA | NA |
| DMR_122 | SEQ ID NO: 122 | hypo | chr 13 | 999670 01 | 999675 00 | chr13:999670 01-99967500 | UBAC2 | NA | NA | NA |
| DMR_123 | SEQ ID NO: 123 | hyper meth ylate d | chr 14 | 100904 501 | 100905 000 | chr14:100904 501-100905000 | WDR25 | NA | NA | NA |
| DMR_124 | SEQ ID NO: 124 | hyper | chr 14 | 208435 01 | 208440 00 | chr14:208435 01-20844000 | TEP1 | NA | NA | NA |
| DMR_125 | SEQ ID NO: 125 | hyper | chr 14 | 369915 01 | 369920 00 | chr14:369915 01-36992000 | NKX2-1-AS1 | NA | NA | NA |
| DMR_126 | SEQ ID NO: 126 | hyper | chr 14 | 644195 01 | 644200 00 | chr14:644195 01-64420000 | SYNE2 | NA | NA | NA |
| DMR_127 | SEQ ID NO: 127 | hyper | chr 14 | 684420 01 | 684425 00 | chr14:684420 01-68442500 | RAD51B | NA | NA | NA |
| DMR_128 | SEQ ID NO: 128 | hypo | chr 15 | 422325 01 | 422330 00 | chr15:422325 01-42233000 | EHD4 | NA | NA | NA |
| DMR_129 | SEQ ID NO: 129 | hypo | chr 15 | 422330 01 | 422335 00 | chr15:422330 01-42233500 | EHD4 | NA | NA | NA |
| DMR_130 | SEQ ID NO: 130 | hypo | chr 15 | 860380 01 | 860385 00 | chr15:860380 01-86038500 | AKAP13 | NA | NA | NA |
| DMR_131 | SEQ ID NO: 131 | hypo | chr 15 | 895245 01 | 895250 00 | chr15:895245 01-89525000 | RP11-320A16.1 | NA | NA | NA |
| DMR_132 | SEQ ID NO: 132 | hyper | chr 16 | 116855 01 | 116860 00 | chr16:116855 01-11686000 | LITAF | NA | NA | NA |
| DMR_133 | SEQ ID NO: 133 | hypo | chr 16 | 144935 01 | 144940 00 | chr16:144935 01-14494000 | RP11-65J21.4 | MIR1 93BH G | - 0.325 | 0.03 568 |
| DMR_134 | SEQ ID NO: 134 | hypo | chr 16 | 232665 01 | 232670 00 | chr16:232665 01-23267000 | SCNN1B | NA | NA | NA |
| DMR_135 | SEQ ID NO: 135 | hyper | chr 16 | 276365 01 | 276370 00 | chr16:276365 01-27637000 | KIAA055 6 | NA | NA | NA |
| DMR_136 | SEQ ID NO: 136 | hyper | chr 16 | 276375 01 | 276380 00 | chr16:276375 01-27638000 | KIAA055 6 | NA | NA | NA |
| DMR_137 | SEQ ID NO: 137 | hyper | chr 16 | 277355 01 | 277360 00 | chr16:277355 01-27736000 | KIAA055 6 | NA | NA | NA |
| DMR_138 | SEQ ID NO: 138 | hyper | chr 16 | 277360 01 | 277365 00 | chr16:277360 01-27736500 | KIAA055 6 | NA | NA | NA |
| DMR_139 | SEQ ID NO: 139 | hypo | chr 16 | 665560 01 | 665565 00 | chr16:665560 01-66556500 | TK2 | CMT M3 | - 0.356 | 0.02 052 |
| DMR_140 | SEQ ID NO: 140 | hyper | chr 16 | 681020 01 | 681025 00 | chr16:681020 01-68102500 | DUS2 | SLC12 A4 | 0.388 | 0.01 111 |
| DMR_141 | SEQ ID NO: 141 | hypo | chr 16 | 695980 01 | 695985 00 | chr16:695980 01-69598500 | NA | NA | NA | NA |
| DMR_142 | SEQ ID NO: 142 | hypo | chr 16 | 704645 01 | 704650 00 | chr16:704645 01-70465000 | ST3GAL2 | NA | NA | NA |
| DMR_143 | SEQ ID NO: 143 | hypo | chr 16 | 729115 01 | 729120 00 | chr16:729115 01-72912000 | ZFHX3 | NA | NA | NA |
| DMR_144 | SEQ ID NO: 144 | hypo | chr 16 | 751725 01 | 751730 00 | chr16:751725 01-75173000 | RP11-252E2.2 | AC09 9508. 1 | 0.357 | 0.02 025 |
| DMR_145 | SEQ ID NO: 145 | hyper | chr 17 | 174295 01 | 174300 00 | chr17:174295 01-17430000 | PEMT | NA | NA | NA |
| DMR_146 | SEQ ID NO: 146 | hypo | chr 17 | 386725 01 | 386730 00 | chr17:386725 01-38673000 | RP5-1028K7.2 | TNS4 | - 0.329 | 0.03 342 |
| DMR_147 | SEQ ID NO: 147 | hypo | chr 17 | 391125 01 | 391130 00 | chr17:391125 01-39113000 | AC00423 1.2 | KRT3 9 | - 0.305 | 0.04 966 |
| DMR_148 | SEQ ID NO: 148 | hypo | chr 17 | 391130 01 | 391135 00 | chr17:391130 01-39113500 | AC00423 1.2 | KRT2 3 | - 0.345 | 0.02 506 |
| DMR_149 | SEQ ID NO: 149 | hypo | chr 17 | 554835 01 | 554840 00 | chr17:554835 01-55484000 | MSI2 | NA | NA | NA |
| DMR_150 | SEQ ID NO: 150 | hypo | chr 17 | 564570 01 | 564575 00 | chr17:564570 01-56457500 | RNF43 | RNF4 3 | - 0.434 | 0.00 405 |
| DMR_151 | SEQ ID NO: 151 | hypo | chr 17 | 564700 01 | 564705 00 | chr17:564700 01-56470500 | RNF43 | RNF4 3 | - 0.462 | 0.00 205 |
| DMR_152 | SEQ ID NO: 152 | hypo | chr 17 | 578615 01 | 578620 00 | chr17:578615 01-57862000 | VMP1 | VMP1 | - 0.434 | 0.00 407 |
| DMR_153 | SEQ ID NO: 153 | hypo | chr 17 | 579220 01 | 579225 00 | chr17:579220 01-57922500 | RNU6-450P | NA | NA | NA |
| DMR_154 | SEQ ID NO: 154 | hyper | chr 17 | 627745 01 | 627750 00 | chr17:627745 01-62775000 | hsa-mir-6080 | NA | NA | NA |
| DMR_155 | SEQ ID NO: 155 | hyper | chr 17 | 627750 01 | 627755 00 | chr17:627750 01-62775500 | hsa-mir-6080 | NA | NA | NA |
| DMR_156 | SEQ ID NO: 156 | hypo | chr 17 | 632250 01 | 632255 00 | chr17:632250 01-63225500 | RGS9 | NA | NA | NA |
| DMR_157 | SEQ ID NO: 157 | hypo | chr 17 | 694190 01 | 694195 00 | chr17:694190 01-69419500 | RNU6-305 P | NA | NA | NA |
| DMR_158 | SEQ ID NO: 158 | hypo | chr 17 | 784215 01 | 784220 00 | chr17:784215 01-78422000 | CTD-2526A2.2 | NA | NA | NA |
| DMR_159 | SEQ ID NO: 159 | hypo | chr 17 | 810065 01 | 810070 00 | chr17:810065 01-81007000 | B3GNTL1 | NA | NA | NA |
| DMR_160 | SEQ ID NO: 160 | hypo | chr 18 | 289780 01 | 289785 00 | chr18:289780 01-28978500 | DSG4 | DSG 1 -AS1 | - 0.317 | 0.04 078 |
| DMR_161 | SEQ ID NO: 161 | hypo | chr 18 | 524430 01 | 524435 00 | chr18:524430 01-52443500 | RAB27B | NA | NA | NA |
| DMR_162 | SEQ ID NO: 162 | hypo | chr 18 | 527645 01 | 527650 00 | chr18:527645 01-52765000 | CTD-2171N6. 1 | NA | NA | NA |
| DMR_163 | SEQ ID NO: 163 | hypo | chr 18 | 680030 01 | 680035 00 | chr18:680030 01-68003500 | RP11-4104.1 | NA | NA | NA |
| DMR_164 | SEQ ID NO: 164 | hyper | chr 18 | 911050 1 | 911100 0 | chr18:911050 1-9111000 | RP11-21J18.1 | AP00 5263. 1 | - 0.307 | 0.04 814 |
| DMR_165 | SEQ ID NO: 165 | hypo | chr 19 | 387895 01 | 387900 00 | chr19:387895 01-38790000 | CTB-102 L5.4 | NA | NA | NA |
| DMR_166 | SEQ ID NO: 166 | hyper | chr 19 | 455550 01 | 455555 00 | chr19:455550 01-45555500 | CLASRP | NA | NA | NA |
| DMR_167 | SEQ ID NO: 167 | hyper | chr 19 | 455555 01 | 455560 00 | chr19:455555 01-45556000 | CLASRP | NA | NA | NA |
| DMR_168 | SEQ ID NO: 168 | hyper | chr 19 | 469155 01 | 469160 00 | chr19:469155 01-46916000 | CCDC8 | CCDC 8 | - 0.345 | 0.02 52 |
| DMR_169 | SEQ ID NO: 169 | hypo | chr 19 | 475990 01 | 475995 00 | chr19:475990 01-47599500 | ZC3H4 | NA | NA | NA |
| DMR_170 | SEQ ID NO: 170 | hypo | chr 19 | 570395 01 | 570400 00 | chr19:570395 01-57040000 | ZNF471 | NA | NA | NA |
| DMR_171 | SEQ ID NO: 171 | hypo | chr 2 | 106746 501 | 106747 000 | chr2:1067465 01-106747000 | UXS1 | NA | NA | NA |
| DMR_172 | SEQ ID NO: 172 | hypo | chr 2 | 158102 501 | 158103 000 | chr2:1581025 01-158103000 | GALNT5 | NA | NA | NA |
| DMR_173 | SEQ ID NO: 173 | hyper | chr 2 | 162283 501 | 162284 000 | chr2:1622835 01-162284000 | SLC4A10 | NA | NA | NA |
| DMR_174 | SEQ ID NO: 174 | hypo | chr 2 | 163700 1 | 163750 0 | chr2:1637001-1637500 | PXDN | NA | NA | NA |
| DMR_175 | SEQ ID NO: 175 | hypo | chr 2 | 165771 001 | 165771 500 | chr2:1657710 01-165771500 | SLC38A1 1 | NA | NA | NA |
| DMR_176 | SEQ ID NO: 176 | hypo | chr 2 | 177755 01 | 177760 00 | chr2:1777550 1-17776000 | VSNL1 | NA | NA | NA |
| DMR_177 | SEQ ID NO: 177 | hypo | chr 2 | 180104 001 | 180104 500 | chr2:1801040 01-180104500 | SESTD1 | NA | NA | NA |
| DMR_178 | SEQ ID NO: 178 | hyper | chr 2 | 224414 001 | 224414 500 | chr2:2244140 01-224414500 | AC01344 8.2 | NA | NA | NA |
| DMR_179 | SEQ ID NO: 179 | hypo | chr 2 | 227292 001 | 227292 500 | chr2:2272920 01-227292500 | MIR5702 | NA | NA | NA |
| DMR_180 | SEQ ID NO: 180 | hypo | chr 2 | 232108 501 | 232109 000 | chr2:2321085 01-232109000 | ARMC9 | NA | NA | NA |
| DMR_181 | SEQ ID NO: 181 | hypo | chr 2 | 232109 001 | 232109 500 | chr2:2321090 01-232109500 | ARMC9 | NA | NA | NA |
| DMR_182 | SEQ ID NO: 182 | hypo | chr 2 | 232155 001 | 232155 500 | chr2:2321550 01-232155500 | ARMC9 | ARM C9 | -0.32 | 0.03 896 |
| DMR_183 | SEQ ID NO: 183 | hypo | chr 2 | 232156 501 | 232157 000 | chr2:2321565 01-232157000 | ARMC9 | NA | NA | NA |
| DMR_184 | SEQ ID NO: 184 | hypo | chr 2 | 233252 501 | 233253 000 | chr2:2332525 01-233253000 | ECEL1P2 | ECEL1 P2 | 0.319 | 0.03 922 |
| DMR_185 | SEQ ID NO: 185 | hypo | chr 2 | 233925 001 | 233925 500 | chr2:2339250 01-233925500 | INPP5D | INPP5 D | 0.373 | 0.01 505 |
| DMR_186 | SEQ ID NO: 186 | hypo | chr 2 | 235588 501 | 235589 000 | chr2:2355885 01-235589000 | AC01014 8.1 | NA | NA | NA |
| DMR_187 | SEQ ID NO: 187 | hypo | chr 2 | 242908 001 | 242908 500 | chr2:2429080 01-242908500 | AC13109 7.3 | NA | NA | NA |
| DMR_188 | SEQ ID NO: 188 | hyper | chr 2 | 281540 01 | 281545 00 | chr2:2815400 1-28154500 | BRE | NA | NA | NA |
| DMR_189 | SEQ ID NO: 189 | hyper | chr 2 | 666525 01 | 666530 00 | chr2:6665250 1-66653000 | MEIS1-AS3 | NA | NA | NA |
| DMR_190 | SEQ ID NO: 190 | hypo | chr 2 | 850340 01 | 850345 00 | chr2:8503400 1-85034500 | DNAH6 | NA | NA | NA |
| DMR_191 | SEQ ID NO: 191 | hyper | chr 2 | 870175 01 | 870180 00 | chr2:8701750 1-87018000 | CD8A | RMN D5A | 0.338 | 0.02 875 |
| DMR_192 | SEQ ID NO: 192 | hypo | chr 20 | 111885 01 | 111890 00 | chr20:111885 01-11189000 | RP4-734C18.1 | NA | NA | NA |
| DMR_193 | SEQ ID NO: 193 | hypo | chr 20 | 139650 1 | 139700 0 | chr20:139650 1-1397000 | FKBP1A | NA | NA | NA |
| DMR_194 | SEQ ID NO: 194 | hyper | chr 20 | 214925 01 | 214930 00 | chr20:214925 01-21493000 | NKX2-2-AS1 | NA | NA | NA |
| DMR_195 | SEQ ID NO: 195 | hypo | chr 20 | 261910 01 | 261915 00 | chr20:261910 01-26191500 | MIR663A HG | NA | NA | NA |
| DMR_196 | SEQ ID NO: 196 | hypo | chr 20 | 261930 01 | 261935 00 | chr20:261930 01-26193500 | MIR663A HG | NA | NA | NA |
| DMR_197 | SEQ ID NO: 197 | hypo | chr 20 | 339140 01 | 339145 00 | chr20:339140 01-33914500 | UQCC1 | EIF6 | - 0.305 | 0.04 959 |
| DMR_198 | SEQ ID NO: 198 | hypo | chr 20 | 341470 01 | 341475 00 | chr20:341470 01-34147500 | FER1L4 | ERGIC 3 | - 0.482 | 0.00 124 |
| DMR_199 | SEQ ID NO: 199 | hyper | chr 20 | 341890 01 | 341895 00 | chr20:341890 01-34189500 | FER1L4 | FER1L 4 | 0.532 | 0.00 029 |
| DMR_200 | SEQ ID NO: 200 | hypo | chr 20 | 384405 01 | 384410 00 | chr20:384405 01-38441000 | RP5-1031J8.1 | NA | NA | NA |
| DMR_201 | SEQ ID NO: 201 | hyper | chr 20 | 393190 01 | 393195 00 | chr20:393190 01-39319500 | MAFB | MAFB | - 0.335 | 0.03 023 |
| DMR_202 | SEQ ID NO: 202 | hypo | chr 20 | 406260 01 | 406265 00 | chr20:406260 01-40626500 | NA | AL04 9812. 2 | - 0.331 | 0.03 246 |
| DMR_203 | SEQ ID NO: 203 | hypo | chr 20 | 406315 01 | 406320 00 | chr20:406315 01-40632000 | NA | NA | NA | NA |
| DMR_204 | SEQ ID NO: 204 | hyper | chr 20 | 429735 01 | 429740 00 | chr20:429735 01-42974000 | R3HDML | NA | NA | NA |
| DMR_205 | SEQ ID NO: 205 | hypo | chr 20 | 551820 01 | 551825 00 | chr20:551820 01-55182500 | U3 | LINC0 1716 | - 0.335 | 0.03 021 |
| DMR_206 | SEQ ID NO: 206 | hypo | chr 20 | 563700 01 | 563705 00 | chr20:563700 01-56370500 | PMEPA1 | PMEP A1 | - 0.308 | 0.04 757 |
| DMR_207 | SEQ ID NO: 207 | hypo | chr 20 | 567060 01 | 567065 00 | chr20:567060 01-56706500 | C20orf85 | NA | NA | NA |
| DMR_208 | SEQ ID NO: 208 | hyper | chr 21 | 172100 01 | 172105 00 | chr21:172100 01-17210500 | USP25 | NA | NA | NA |
| DMR_209 | SEQ ID NO: 209 | hypo | chr 21 | 179050 01 | 179055 00 | chr21:179050 01-17905500 | LINC0047 8 | NA | NA | NA |
| DMR_210 | SEQ ID NO: 210 | hypo | chr 21 | 282885 01 | 282890 00 | chr21:282885 01-28289000 | NA | ADA MTS1 | 0.373 | 0.01 493 |
| DMR_211 | SEQ ID NO: 211 | hypo | chr 21 | 338355 01 | 338360 00 | chr21:338355 01-33836000 | EVA1C | URB1 | - 0.408 | 0.00 725 |
| DMR_212 | SEQ ID NO: 212 | hypo | chr 21 | 338360 01 | 338365 00 | chr21:338360 01-33836500 | EVA1C | NA | NA | NA |
| DMR_213 | SEQ ID NO: 213 | hypo | chr 21 | 338365 01 | 338370 00 | chr21:338365 01-33837000 | EVA1C | NA | NA | NA |
| DMR_214 | SEQ ID NO: 214 | hyper | chr 21 | 380690 01 | 380695 00 | chr21:380690 01-38069500 | AP00069 7.6 | AP00 0697. 1 | 0.346 | 0.02 495 |
| DMR_215 | SEQ ID NO: 215 | hyper | chr 21 | 406330 01 | 406335 00 | chr21:406330 01-40633500 | BRWD1 | NA | NA | NA |
| DMR_216 | SEQ ID NO: 216 | hyper | chr 22 | 183420 01 | 183425 00 | chr22:183420 01-18342500 | MICAL3 | NA | NA | NA |
| DMR_217 | SEQ ID NO: 217 | hyper | chr 22 | 209075 01 | 209080 00 | chr22:209075 01-20908000 | MED15 | NA | NA | NA |
| DMR_218 | SEQ ID NO: 218 | hyper | chr 22 | 320140 01 | 320145 00 | chr22:320140 01-32014500 | SFI1 | NA | NA | NA |
| DMR_219 | SEQ ID NO: 219 | hyper | chr 22 | 407635 01 | 407640 00 | chr22:407635 01-40764000 | ADSL | AL02 2238. 1 | - 0.307 | 0.04 786 |
| DMR_220 | SEQ ID NO: 220 | hypo | chr 3 | 112817 001 | 112817 500 | chr3:1128170 01-112817500 | RP11-572M11. 4 | NA | NA | NA |
| DMR_221 | SEQ ID NO: 221 | hyper | chr 3 | 114379 001 | 114379 500 | chr3:1143790 01-114379500 | ZBTB20 | NA | NA | NA |
| DMR_222 | SEQ ID NO: 222 | hypo | chr 3 | 124554 001 | 124554 500 | chr3:1245540 01-124554500 | ITGB5 | NA | NA | NA |
| DMR_223 | SEQ ID NO: 223 | hyper | chr 3 | 137489 001 | 137489 500 | chr3:1374890 01-137489500 | RP11-2A4.3 | SOX1 4 | 0.351 | 0.02 251 |
| DMR_224 | SEQ ID NO: 224 | hyper | chr 3 | 137489 501 | 137490 000 | chr3:1374895 01-137490000 | RP11-2A4.3 | SOX1 4 | 0.321 | 0.03 796 |
| DMR_225 | SEQ ID NO: 225 | hypo | chr 3 | 142569 001 | 142569 500 | chr3:1425690 01-142569500 | PCOLCE2 | NA | NA | NA |
| DMR_226 | SEQ ID NO: 226 | hypo | chr 3 | 143610 01 | 143615 00 | chr3:1436100 1-14361500 | RP11-536I6.2 | NA | NA | NA |
| DMR_227 | SEQ ID NO: 227 | hyper | chr 3 | 151692 501 | 151693 000 | chr3:1516925 01-151693000 | RP11-454C18.2 | NA | NA | NA |
| DMR_228 | SEQ ID NO: 228 | hypo | chr 3 | 152501 | 153000 | chr3:152501-153000 | AY26918 6.2 | NA | NA | NA |
| DMR_229 | SEQ ID NO: 229 | hypo | chr 3 | 161067 001 | 161067 500 | chr3:1610670 01-161067500 | SPTSSB | NA | NA | NA |
| DMR_230 | SEQ ID NO: 230 | hypo | chr 3 | 171594 501 | 171595 000 | chr3:1715945 01-171595000 | TMEM21 2 | NA | NA | NA |
| DMR_231 | SEQ ID NO: 231 | hypo | chr 3 | 177325 001 | 177325 500 | chr3:1773250 01-177325500 | LINC0057 8 | NA | NA | NA |
| DMR_232 | SEQ ID NO: 232 | hypo | chr 3 | 187607 501 | 187608 000 | chr3:1876075 01-187608000 | RP11-44H4.1 | NA | NA | NA |
| DMR_233 | SEQ ID NO: 233 | hypo | chr 3 | 190036 501 | 190037 000 | chr3:1900365 01-190037000 | CLDN1 | CLDN 1 | - 0.347 | 0.02 447 |
| DMR_234 | SEQ ID NO: 234 | hypo | chr 3 | 190038 501 | 190039 000 | chr3:1900385 01-190039000 | CLDN1 | CLDN 16 | - 0.361 | 0.01 898 |
| DMR_235 | SEQ ID NO: 235 | hypo | chr 3 | 190039 001 | 190039 500 | chr3:1900390 01-190039500 | CLDN1 | CLDN 1 | - 0.421 | 0.00 552 |
| DMR_236 | SEQ ID NO: 236 | hyper | chr 3 | 190041 001 | 190041 500 | chr3:1900410 01-190041500 | NA | CLDN 1 | - 0.385 | 0.01 181 |
| DMR_237 | SEQ ID NO: 237 | hypo | chr 3 | 192605 001 | 192605 500 | chr3:1926050 01-192605500 | MB21D2 | NA | NA | NA |
| DMR_238 | SEQ ID NO: 238 | hypo | chr 3 | 237810 01 | 237815 00 | chr3:2378100 1-23781500 | AC02062 6.1 | NA | NA | NA |
| DMR_239 | SEQ ID NO: 239 | hypo | chr 3 | 254650 01 | 254655 00 | chr3:2546500 1-25465500 | RARB | NA | NA | NA |
| DMR_240 | SEQ ID NO: 240 | hypo | chr 3 | 320100 01 | 320105 00 | chr3:3201000 1-32010500 | OSBPL10 | NA | NA | NA |
| DMR_241 | SEQ ID NO: 241 | hypo | chr 3 | 320105 01 | 320110 00 | chr3:3201050 1-32011000 | OSBPL10 | NA | NA | NA |
| DMR_242 | SEQ ID NO:242 | hypo | chr 3 | 451065 01 | 451070 00 | chr3:4510650 1-45107000 | CDCP1 | NA | NA | NA |
| DMR_243 | SEQ ID NO: 243 | hypo | chr 3 | 458475 01 | 458480 00 | chr3:4584750 1-45848000 | SLC6A20 | NA | NA | NA |
| DMR_244 | SEQ ID NO: 244 | hypo | chr 3 | 659105 01 | 659110 00 | chr3:6591050 1-65911000 | MAGI1-IT1 | NA | NA | NA |
| DMR_245 | SEQ ID NO: 245 | hypo | chr 4 | 106845 001 | 106845 500 | chr4:1068450 01-106845500 | NPNT | NA | NA | NA |
| DMR_246 | SEQ ID NO: 246 | hyper | chr 4 | 111539 001 | 111539 500 | chr4:1115390 01-111539500 | PITX2 | PITX2 | 0.434 | 0.00 409 |
| DMR_247 | SEQ ID NO: 247 | hyper | chr 4 | 111559 501 | 111560 000 | chr4:1115595 01-111560000 | NA | PITX2 | 0.522 | 0.00 039 |
| DMR_248 | SEQ ID NO: 248 | hyper | chr 4 | 114277 001 | 114277 500 | chr4:1142770 01-114277500 | ANK2 | NA | NA | NA |
| DMR_249 | SEQ ID NO: 249 | hypo | chr 4 | 114880 001 | 114880 500 | chr4:1148800 01-114880500 | ARSJ | NA | NA | NA |
| DMR_250 | SEQ ID NO: 250 | hypo | chr 4 | 116160 01 | 116165 00 | chr4:1161600 1-11616500 | RP11-281P23.2 | NA | NA | NA |
| DMR_251 | SEQ ID NO: 251 | hyper | chr 4 | 165878 001 | 165878 500 | chr4:1658780 01-165878500 | TRIM61 | AC10 6872. 7 | 0.346 | 0.02 463 |
| DMR_252 | SEQ ID NO: 252 | hypo | chr 4 | 169020 001 | 169020 500 | chr4:1690200 01-169020500 | ANXA10 | NA | NA | NA |
| DMR_253 | SEQ ID NO: 253 | hypo | chr 4 | 187729 001 | 187729 500 | chr4:1877290 01-187729500 | AC10886 5.1 | NA | NA | NA |
| DMR_254 | SEQ ID NO: 254 | hypo | chr 4 | 187766 501 | 187767 000 | chr4:1877665 01-187767000 | AC10886 5.1 | NA | NA | NA |
| DMR_255 | SEQ ID NO: 255 | hypo | chr 4 | 239325 01 | 239330 00 | chr4:2393250 1-23933000 | PPARGC1 A | NA | NA | NA |
| DMR_256 | SEQ ID NO: 256 | hypo | chr 4 | 312385 01 | 312390 00 | chr4:3123850 1-31239000 | RP11-617I14.1 | NA | NA | NA |
| DMR_257 | SEQ ID NO: 257 | hypo | chr 4 | 314340 01 | 314345 00 | chr4:3143400 1-31434500 | RP11-665I14.1 | NA | NA | NA |
| DMR_258 | SEQ ID NO: 258 | hyper | chr 4 | 715001 | 715500 | chr4:715001-715500 | PCGF3 | AC13 9887. 2 | -0.39 | 0.01 078 |
| DMR_259 | SEQ ID NO: 259 | hypo | chr 4 | 721645 01 | 721650 00 | chr4:7216450 1-72165000 | SLC4A4 | NA | NA | NA |
| DMR_260 | SEQ ID NO: 260 | hypo | chr 4 | 748720 01 | 748725 00 | chr4:7487200 1-74872500 | RN7SL21 8P | NA | NA | NA |
| DMR_261 | SEQ ID NO: 261 | hypo | chr 4 | 751865 01 | 751870 00 | chr4:7518650 1-75187000 | EPGN | NA | NA | NA |
| DMR_262 | SEQ ID NO: 262 | hypo | chr 4 | 752300 01 | 752305 00 | chr4:7523000 1-75230500 | EREG | AREG | - 0.353 | 0.02 204 |
| DMR_263 | SEQ ID NO: 263 | hypo | chr 4 | 752370 01 | 752375 00 | chr4:7523700 1-75237500 | EREG | NA | NA | NA |
| DMR_264 | SEQ ID NO: 264 | hypo | chr 4 | 754025 01 | 754030 00 | chr4:7540250 1-75403000 | AC14229 3.3 | NA | NA | NA |
| DMR_265 | SEQ ID NO: 265 | hypo | chr 4 | 754030 01 | 754035 00 | chr4:7540300 1-75403500 | AC14229 3.3 | NA | NA | NA |
| DMR_266 | SEQ ID NO: 266 | hypo | chr 4 | 754060 01 | 754065 00 | chr4:7540600 1-75406500 | AC14229 3.3 | NA | NA | NA |
| DMR_267 | SEQ ID NO: 267 | hypo | chr 4 | 754080 01 | 754085 00 | chr4:7540800 1-75408500 | AC14229 3.3 | AC23 9584. 1 | - 0.345 | 0.02 514 |
| DMR_268 | SEQ ID NO: 268 | hypo | chr 4 | 755505 01 | 755510 00 | chr4:7555050 1-75551000 | AC14229 3.3 | NA | NA | NA |
| DMR_269 | SEQ ID NO: 269 | hypo | chr 4 | 755535 01 | 755540 00 | chr4:7555350 1-75554000 | AC14229 3.3 | AC23 9584. 1 | - 0.512 | 0.00 052 |
| DMR_270 | SEQ ID NO: 270 | hypo | chr 4 | 885785 01 | 885790 00 | chr4:8857850 1-88579000 | RP11-742B18.1 | NA | NA | NA |
| DMR_271 | SEQ ID NO: 271 | hypo | chr 5 | 103416 001 | 103416 500 | chr5:1034160 01-103416500 | RP11-138J23.1 | LINC0 2163 | - 0.646 | 3.89 E-06 |
| DMR_272 | SEQ ID NO: 272 | hypo | chr 5 | 103848 501 | 103849 000 | chr5:1038485 01-103849000 | RP11-6N13.1 | NA | NA | NA |
| DMR_273 | SEQ ID NO: 273 | hypo | chr 5 | 106673 501 | 106674 000 | chr5:1066735 01-106674000 | EFNA5 | NA | NA | NA |
| DMR_274 | SEQ ID NO: 274 | hypo | chr 5 | 112499 001 | 112499 500 | chr5:1124990 01-112499500 | MCC | NA | NA | NA |
| DMR_275 | SEQ ID NO: 275 | hypo | chr 5 | 116512 501 | 116513 000 | chr5:1165125 01-116513000 | RPL35AP 15 | NA | NA | NA |
| DMR_276 | SEQ ID NO: 276 | hypo | chr 5 | 132576 001 | 132576 500 | chr5:1325760 01-132576500 | FSTL4 | NA | NA | NA |
| DMR_277 | SEQ ID NO: 277 | hyper | chr 5 | 142454 001 | 142454 500 | chr5:1424540 01-142454500 | ARHGAP 26 | NA | NA | NA |
| DMR_278 | SEQ ID NO: 278 | hypo | chr 5 | 157030 501 | 157031 000 | chr5:1570305 01-157031000 | AC00869 4.2 | NA | NA | NA |
| DMR_279 | SEQ ID NO: 279 | hypo | chr 5 | 174806 001 | 174806 500 | chr5:1748060 01-174806500 | DRD1 | NA | NA | NA |
| DMR_280 | SEQ ID NO: 280 | hypo | chr 5 | 439980 01 | 439985 00 | chr5:4399800 1-43998500 | RNU6-381P | NA | NA | NA |
| DMR_281 | SEQ ID NO: 281 | hypo | chr 5 | 735375 01 | 735380 00 | chr5:7353750 1-73538000 | AC10673 2.1 | NA | NA | NA |
| DMR_282 | SEQ ID NO: 282 | hypo | chr 5 | 739295 01 | 739300 00 | chr5:7392950 1-73930000 | ENC1 | NA | NA | NA |
| DMR_283 | SEQ ID NO: 283 | hypo | chr 5 | 816850 01 | 816855 00 | chr5:8168500 1-81685500 | ATP6AP1 L | NA | NA | NA |
| DMR_284 | SEQ ID NO: 284 | hypo | chr 5 | 918875 01 | 918880 00 | chr5:9188750 1-91888000 | RP11-133F8.2 | NA | NA | NA |
| DMR_285 | SEQ ID NO: 285 | hypo | chr 6 | 107860 501 | 107861 000 | chr6:1078605 01-107861000 | SOBP | PDSS 2 | - 0.318 | 0.04 042 |
| DMR_286 | SEQ ID NO: 286 | hyper | chr 6 | 111801 001 | 111801 500 | chr6:1118010 01-111801500 | REV3L | NA | NA | NA |
| DMR_287 | SEQ ID NO: 287 | hypo | chr 6 | 134339 001 | 134339 500 | chr6:1343390 01-134339500 | SLC2A12 | NA | NA | NA |
| DMR_288 | SEQ ID NO: 288 | hypo | chr 6 | 138339 001 | 138339 500 | chr6:1383390 01-138339500 | RPSAP42 | LINC0 2528 | - 0.353 | 0.02 168 |
| DMR_289 | SEQ ID NO: 289 | hypo | chr 6 | 148762 501 | 148763 000 | chr6:1487625 01-148763000 | SASH1 | NA | NA | NA |
| DMR_290 | SEQ ID NO: 290 | hypo | chr 6 | 208775 01 | 208780 00 | chr6:2087750 1-20878000 | CDKAL1 | NA | NA | NA |
| DMR_291 | SEQ ID NO: 291 | hypo | chr 6 | 213830 01 | 213835 00 | chr6:2138300 1-21383500 | RP1-135L22.1 | NA | NA | NA |
| DMR_292 | SEQ ID NO: 292 | hypo | chr 6 | 213845 01 | 213850 00 | chr6:2138450 1-21385000 | RP1-135L22.1 | NA | NA | NA |
| DMR_293 | SEQ ID NO: 293 | hypo | chr 6 | 454095 01 | 454100 00 | chr6:4540950 1-45410000 | RUNX2 | NA | NA | NA |
| DMR_294 | SEQ ID NO: 294 | hypo | chr 6 | 470435 01 | 470440 00 | chr6:4704350 1-47044000 | GPR110 | NA | NA | NA |
| DMR_295 | SEQ ID NO: 295 | hypo | chr 6 | 535790 01 | 535795 00 | chr6:5357900 1-53579500 | RP11-79N23.1 | LRRC 1 | - 0.365 | 0.01 749 |
| DMR_296 | SEQ ID NO: 296 | hyper | chr 6 | 641075 01 | 641080 00 | chr6:6410750 1-64108000 | RP3-407E4.3 | NA | NA | NA |
| DMR_297 | SEQ ID NO: 297 | hypo | chr 6 | 642375 01 | 642380 00 | chr6:6423750 1-64238000 | PTP4A1 | NA | NA | NA |
| DMR_298 | SEQ ID NO: 298 | hypo | chr 6 | 744140 01 | 744145 00 | chr6:7441400 1-74414500 | CD109 | NA | NA | NA |
| DMR_299 | SEQ ID NO: 299 | hypo | chr 7 | 101360 501 | 101361 000 | chr7:1013605 01-101361000 | MYL10 | NA | NA | NA |
| DMR_300 | SEQ ID NO: 300 | hypo | chr 7 | 114869 501 | 114870 000 | chr7:1148695 01-114870000 | AC06861 0.5 | NA | NA | NA |
| DMR_301 | SEQ ID NO: 301 | hypo | chr 7 | 114870 001 | 114870 500 | chr7:1148700 01-114870500 | AC06861 0.5 | NA | NA | NA |
| DMR_302 | SEQ ID NO: 302 | hypo | chr 7 | 130645 001 | 130645 500 | chr7:1306450 01-130645500 | LINC-PINT | NA | NA | NA |
| DMR_303 | SEQ ID NO: 303 | hypo | chr 7 | 144606 501 | 144607 000 | chr7:1446065 01-144607000 | RN7SKP1 74 | NA | NA | NA |
| DMR_304 | SEQ ID NO: 304 | hyper | chr 7 | 155596 501 | 155597 000 | chr7:1555965 01-155597000 | SHH | NA | NA | NA |
| DMR_305 | SEQ ID NO: 305 | hypo | chr 7 | 157660 001 | 157660 500 | chr7:1576600 01-157660500 | PTPRN2 | NA | NA | NA |
| DMR_306 | SEQ ID NO: 306 | hypo | chr 7 | 261950 01 | 261955 00 | chr7:2619500 1-26195500 | NFE2L3 | NFE2 L3 | - 0.307 | 0.04 796 |
| DMR_307 | SEQ ID NO: 307 | hyper | chr 7 | 271420 01 | 271425 00 | chr7:2714200 1-27142500 | NA | HOXA 13 | 0.329 | 0.03 337 |
| DMR_308 | SEQ ID NO: 308 | hyper | chr 7 | 271425 01 | 271430 00 | chr7:2714250 1-27143000 | NA | NA | NA | NA |
| DMR_309 | SEQ ID NO: 309 | hyper | chr 7 | 271430 01 | 271435 00 | chr7:2714300 1-27143500 | HOXA2 | HOXA 13 | 0.327 | 0.03 477 |
| DMR_310 | SEQ ID NO: 310 | hyper | chr 7 | 271460 01 | 271465 00 | chr7:2714600 1-27146500 | HOXA3 | NA | NA | NA |
| DMR_311 | SEQ ID NO: 311 | hyper | chr 7 | 271465 01 | 271470 00 | chr7:2714650 1-27147000 | HOXA3 | NA | NA | NA |
| DMR_312 | SEQ ID NO: 312 | hyper | chr 7 | 271470 01 | 271475 00 | chr7:2714700 1-27147500 | HOXA3 | NA | NA | NA |
| DMR_313 | SEQ ID NO: 313 | hyper | chr 7 | 271475 01 | 271480 00 | chr7:2714750 1-27148000 | HOXA-AS2 | NA | NA | NA |
| DMR_314 | SEQ ID NO: 314 | hyper | chr 7 | 271480 01 | 271485 00 | chr7:2714800 1-27148500 | HOXA-AS2 | NA | NA | NA |
| DMR_315 | SEQ ID NO: 315 | hyper | chr 7 | 271485 01 | 271490 00 | chr7:2714850 1-27149000 | HOXA-AS2 | AC00 4080. 4 | 0.351 | 0.02 282 |
| DMR_316 | SEQ ID NO: 316 | hyper | chr 7 | 271520 01 | 271525 00 | chr7:2715200 1-27152500 | HOXA-AS2 | NA | NA | NA |
| DMR_317 | SEQ ID NO: 317 | hypo | chr 7 | 384220 01 | 384225 00 | chr7:3842200 1-38422500 | NA | TRGV 5P | 0.305 | 0.04 919 |
| DMR_318 | SEQ ID NO: 318 | hypo | chr 7 | 503410 01 | 503415 00 | chr7:5034100 1-50341500 | NA | NA | NA | NA |
| DMR_319 | SEQ ID NO: 319 | hypo | chr 7 | 542370 01 | 542375 00 | chr7:5423700 1-54237500 | RP11-436F9.1 | NA | NA | NA |
| DMR_320 | SEQ ID NO: 320 | hyper | chr 7 | 552555 01 | 552560 00 | chr7:5525550 1-55256000 | EGFR | NA | NA | NA |
| DMR_321 | SEQ ID NO: 321 | hyper | chr 7 | 552560 01 | 552565 00 | chr7:5525600 1-55256500 | EGFR | NA | NA | NA |
| DMR_322 | SEQ ID NO: 322 | hypo | chr 7 | 611650 1 | 611700 0 | chr7:6116501-6117000 | AC00489 5.4 | NA | NA | NA |
| DMR_323 | SEQ ID NO: 323 | hyper | chr 7 | 768280 01 | 768285 00 | chr7:7682800 1-76828500 | CCDC146 | NA | NA | NA |
| DMR_324 | SEQ ID NO: 324 | hypo | chr 7 | 834350 1 | 834400 0 | chr7:8343501-8344000 | RP4-594A5.1 | AC00 7009. 1 | - 0.305 | 0.04 954 |
| DMR_325 | SEQ ID NO: 325 | hypo | chr 7 | 836850 01 | 836855 00 | chr7:8368500 1-83685500 | SEMA3A | NA | NA | NA |
| DMR_326 | SEQ ID NO: 326 | hypo | chr 7 | 883850 01 | 883855 00 | chr7:8838500 1-88385500 | NA | NA | NA | NA |
| DMR_327 | SEQ ID NO: 327 | hyper | chr 7 | 906501 | 907000 | chr7:906501-907000 | SUN1 | NA | NA | NA |
| DMR_328 | SEQ ID NO: 328 | hyper | chr 8 | 103266 001 | 103266 500 | chr8:1032660 01-103266500 | UBR5 | NA | NA | NA |
| DMR_329 | SEQ ID NO: 329 | hypo | chr 8 | 103918 501 | 103919 000 | chr8:1039185 01-103919000 | KB-1507C5.2 | AZIN1 -AS1 | - 0.346 | 0.02 488 |
| DMR_330 | SEQ ID NO: 1 | hypo | chr 8 | 106328 001 | 106328 500 | chr8:1063280 01-106328500 | ZFPM2 | NA | NA | NA |
| DMR_331 | SEQ ID NO: 1 | hypo | chr 8 | 106334 501 | 106335 000 | chr8:1063345 01-106335000 | ZFPM2 | NA | NA | NA |
| DMR_332 | SEQ ID NO: 332 | hypo | chr 8 | 121787 501 | 121788 000 | chr8:1217875 01-121788000 | SNTB1 | AC10 4958. 2 | - 0.498 | 0.00 079 |
| DMR_333 | SEQ ID NO: 333 | hypo | chr 8 | 126714 001 | 126714 500 | chr8:1267140 01-126714500 | RP11-697B24.1 | NA | NA | NA |
| DMR_334 | SEQ ID NO: 334 | hypo | chr 8 | 128235 001 | 128235 500 | chr8:1282350 01-128235500 | CASC19 | CASC 19 | - 0.355 | 0.02 122 |
| DMR_335 | SEQ ID NO: 335 | hypo | chr 8 | 128256 001 | 128256 500 | chr8:1282560 01-128256500 | PCAT1 | NA | NA | NA |
| DMR_336 | SEQ ID NO: 336 | hypo | chr 8 | 128256 501 | 128257 000 | chr8:1282565 01-128257000 | PCAT1 | AC01 8714. 1 | - 0.361 | 0.01 897 |
| DMR_337 | SEQ ID NO: 337 | hypo | chr 8 | 128257 501 | 128258 000 | chr8:1282575 01-128258000 | PCAT1 | AC01 8714. 1 | - 0.351 | 0.02 279 |
| DMR_338 | SEQ ID NO: 338 | hypo | chr 8 | 128259 001 | 128259 500 | chr8:1282590 01-128259500 | PCAT1 | CASC 19 | - 0.409 | 0.00 711 |
| DMR_339 | SEQ ID NO: 339 | hypo | chr 8 | 128260 001 | 128260 500 | chr8:1282600 01-128260500 | PCAT1 | CASC 19 | - 0.391 | 0.01 039 |
| DMR_340 | SEQ ID NO: 340 | hypo | chr 8 | 128808 001 | 128808 500 | chr8:1288080 01-128808500 | PVT1 | NA | NA | NA |
| DMR_341 | SEQ ID NO: 341 | hypo | chr 8 | 128808 501 | 128809 000 | chr8:1288085 01-128809000 | PVT1 | CASC 11 | - 0.307 | 0.04 819 |
| DMR_342 | SEQ ID NO: 342 | hypo | chr 8 | 132050 501 | 132051 000 | chr8:1320505 01-132051000 | ADCY8 | NA | NA | NA |
| DMR_343 | SEQ ID NO: 343 | hypo | chr 8 | 132057 001 | 132057 500 | chr8:1320570 01-132057500 | NA | NA | NA | NA |
| DMR_344 | SEQ ID NO: 344 | hypo | chr 8 | 132855 001 | 132855 500 | chr8:1328550 01-132855500 | EFR3A | NA | NA | NA |
| DMR_345 | SEQ ID NO: 345 | hypo | chr 8 | 140227 001 | 140227 500 | chr8:1402270 01-140227500 | CTD-2534J5.1 | NA | NA | NA |
| DMR_346 | SEQ ID NO: 346 | hyper | chr 8 | 140757501 | 140758000 | chr8:1407575 01-140758000 | TRAPPC9 | KCNK9 | 0.361 | 0.019 |
| DMR_347 | SEQ ID NO: 347 | hypo | chr 8 | 143316 501 | 143317 000 | chr8:1433165 01-143317000 | TSNARE1 | NA | NA | NA |
| DMR_348 | SEQ ID NO: 348 | hypo | chr 8 | 280565 01 | 280570 00 | chr8:2805650 1-28057000 | ELP3 | NA | NA | NA |
| DMR_349 | SEQ ID NO: 349 | hypo | chr 8 | 548575 01 | 548580 00 | chr8:5485750 1-54858000 | RGS20 | TCEA 1 | -0.32 | 0.03 895 |
| DMR_350 | SEQ ID NO: 350 | hyper | chr 8 | 642000 1 | 642050 0 | chr8:6420001-6420500 | MCPH1 | NA | NA | NA |
| DMR_351 | SEQ ID NO: 351 | hypo | chr 8 | 655010 01 | 655015 00 | chr8:6550100 1-65501500 | CYP7B1 | NA | NA | NA |
| DMR_352 | SEQ ID NO: 352 | hypo | chr 8 | 655025 01 | 655030 00 | chr8:6550250 1-65503000 | CYP7B1 | AC09 0136. 3 | 0.365 | 0.01 739 |
| DMR_353 | SEQ ID NO: 353 | hyper | chr 8 | 673445 01 | 673450 00 | chr8:6734450 1-67345000 | ADHFE1 | NA | NA | NA |
| DMR_354 | SEQ ID NO: 354 | hypo | chr 8 | 688685 01 | 688690 00 | chr8:6886850 1-68869000 | PREX2 | NA | NA | NA |
| DMR_355 | SEQ ID NO: 355 | hyper | chr 8 | 709830 01 | 709835 00 | chr8:7098300 1-70983500 | PRDM14 | NA | NA | NA |
| DMR_356 | SEQ ID NO: 356 | hyper | chr 8 | 724685 01 | 724690 00 | chr8:7246850 1-72469000 | RP11-1102P16. 1 | NA | NA | NA |
| DMR_357 | SEQ ID NO: 357 | hypo | chr 8 | 765280 01 | 765285 00 | chr8:7652800 1-76528500 | NA | NA | NA | NA |
| DMR_358 | SEQ ID NO: 358 | hypo | chr 8 | 819630 01 | 819635 00 | chr8:8196300 1-81963500 | PAG1 | NA | NA | NA |
| DMR_359 | SEQ ID NO: 359 | hypo | chr 8 | 948925 01 | 948930 00 | chr8:9489250 1-94893000 | PDP1 | NA | NA | NA |
| DMR_360 | SEQ ID NO: 360 | hypo | chr 8 | 960785 01 | 960790 00 | chr8:9607850 1-96079000 | NDUFAF 6 | NA | NA | NA |
| DMR_361 | SEQ ID NO: 361 | hyper | chr 8 | 971575 01 | 971580 00 | chr8:9715750 1-97158000 | GDF6 | NA | NA | NA |
| DMR_362 | SEQ ID NO: 362 | hypo | chr 8 | 975100 01 | 975105 00 | chr8:9751000 1-97510500 | SDC2 | NA | NA | NA |
| DMR_363 | SEQ ID NO: 363 | hypo | chr 9 | 110856 501 | 110857 000 | chr9:1108565 01-110857000 | CHCHD4 P2 | NA | NA | NA |
| DMR_364 | SEQ ID NO: 364 | hyper | chr 9 | 124132 001 | 124132 500 | chr9:1241320 01-124132500 | STOM | NA | NA | NA |
| DMR_365 | SEQ ID NO: 365 | hyper | chr 9 | 124988 501 | 124989 000 | chr9:1249885 01-124989000 | LHX6 | MRRF | 0.359 | 0.01 963 |
| DMR_366 | SEQ ID NO: 366 | hypo | chr 9 | 129748 501 | 129749 000 | chr9:1297485 01-129749000 | RALGPS1 | NA | NA | NA |
| DMR_367 | SEQ ID NO: 367 | hypo | chr 9 | 131317 001 | 131317 500 | chr9:1313170 01-131317500 | SPTAN1 | NA | NA | NA |
| DMR_368 | SEQ ID NO: 368 | hypo | chr 9 | 218305 01 | 218310 00 | chr9:2183050 1-21831000 | RP11-145E5.5 | NA | NA | NA |
| DMR_369 | SEQ ID NO: 369 | hyper | chr 9 | 274420 01 | 274425 00 | chr9:2744200 1-27442500 | MOB3B | MOB 3B | - 0.399 | 0.00 877 |
| DMR_370 | SEQ ID NO: 370 | hyper | chr 9 | 318100 1 | 318150 0 | chr9:3181001-3181500 | RP11-32F11.2 | NA | NA | NA |
| DMR_371 | SEQ ID NO: 371 | hypo | chr 9 | 331605 01 | 331610 00 | chr9:3316050 1-33161000 | B4GALT1 | NA | NA | NA |
| DMR_372 | SEQ ID NO: 372 | hypo | chr X | 147584 501 | 147585 000 | chrX:1475845 01-147585000 | AFF2 | NA | NA | NA |
| DMR_373 | SEQ ID NO: 373 | hypo | chr X | 148476 001 | 148476 500 | chrX:1484760 01-148476500 | NA | NA | NA | NA |
| DMR_374 | SEQ ID NO: 374 | hypo | chr X | 160425 01 | 160430 00 | chrX:1604250 1-16043000 | GRPR | NA | NA | NA |
| DMR_375 | SEQ ID NO: 375 | hypo | chr X | 239025 01 | 239030 00 | chrX:2390250 1-23903000 | APOO | NA | NA | NA |
| DMR_376 | SEQ ID NO: 376 | hypo | chr X | 339740 01 | 339745 00 | chrX:3397400 1-33974500 | RP11-305F18.1 | NA | NA | NA |

| | | | | | | | | | | |
|---|---|---|---|---|---|---|---|---|---|---|
| Table 1 demonstrates the nucleic acid sequence ID number, the methylation status, the chromosome each DMR resides in, the start and end coordinates of each DMR, the gene the DMR is located in and the correlation values for each DMR. | | | | | | | | | | |

According to a second aspect of the present invention, there is provided one or more biomarkers for use in vitro diagnosis of colorectal cancer or for predicting the likelihood of colorectal cancer progressing to metastatic colorectal cancer, wherein the biomarkers comprise or consist of the nucleic acid sequences of the one or more differentially methylated regions listed in Table 1. In embodiments, the biomarkers comprise or consist of one or more of the nucleic acid sequences of SEQ ID NO:1 to 376 or a functionally active fragment or variant thereof.

In embodiments, the one or more differentially methylated region is DMR_334, DMR_312, DMR_302, DMR_75 or combinations thereof. In embodiments, the differentially methylated region DMR_312 comprises or consists of the nucleic acid sequence of SEQ ID NO:312 or a functionally active fragment or variant thereof. In embodiments, the differentially methylated region DMR_302 comprises or consists of the nucleic acid sequence of SEQ ID NO:302 or a functionally active fragment or variant thereof. In embodiments, the differentially methylated region DMR_75 comprises or consists of the nucleic acid sequence of SEQ ID NO:75 or a functionally active fragment or variant thereof. In embodiments, the one or more differentially methylated region is defined by the coordinates chr8:128235001-128235500 (DMR_334), chr7:27147001-27147500 (DMR_312), chr7:130645001-130645500 (DMR_302) or chr12:76035001-76035500 (DMR_75).

In embodiments, the differentially methylated region is DMR_334. In embodiments, the differentially methylated region DMR_334 comprises or consists of the nucleic acid sequence of SEQ ID NO:334 or a functionally active fragment or variant thereof.

In embodiments, the one or more differentially methylated region is defined by the coordinates chr8:128235001-128235500 (DMR_334).

In embodiments, the differentially methylated region is DMR_302. In embodiments, the differentially methylated region DMR_302 comprises or consists of the nucleic acid sequence of SEQ ID NO:302 or a functionally active fragment or variant thereof. In embodiments, the one or more differentially methylated region is defined by the coordinates chr7:130645001-130645500 (DMR_302).

In embodiments, the differentially methylated region is DMR_75. In embodiments, the differentially methylated region DMR_75 comprises or consists of the nucleic acid sequence of SEQ ID NO:75 or a functionally active fragment or variant thereof. In embodiments, the one or more differentially methylated region is defined by the coordinates chr12:76035001-76035500 (DMR_75).

In embodiments, the differentially methylated region is DMR_312. In embodiments, the differentially methylated region DMR_312 comprises or consists of the nucleic acid sequence of SEQ ID NO:312 or a functionally active fragment or variant thereof. In embodiments, the one or more differentially methylated region is defined by the coordinates chr7:27147001-27147500 (DMR_312).

In embodiments, there are three differentially methylated regions. In embodiments, the three differentially methylated regions are DMR_312, DMR_302 and DMR_75. In embodiments, there are two differentially methylated regions. In embodiments, the two differentially methylated regions are DMR_302 and DMR_75. In embodiments, the two differentially methylated regions are DMR_312 and DMR_75. In embodiments, the two differentially methylated regions are DMR_312 and DMR_302. In embodiments, the differentially methylated region DMR_302 comprises or consists of the nucleic acid sequence of SEQ ID NO:302 or a functionally active fragment or variant thereof. In embodiments, the differentially methylated region DMR_75 comprises or consists of the nucleic acid sequence of SEQ ID NO:75 or a functionally active fragment or variant thereof. In embodiments, the differentially methylated region DMR_312 comprises or consists of the nucleic acid sequence of SEQ ID NO:312 or a functionally active fragment or variant thereof. In embodiments, the one or more differentially methylated region is defined by the coordinates chr7:27147001-27147500 (DMR_312), chr7:130645001-130645500 (DMR_302) and chr12:76035001-76035500 (DMR_75).

In embodiments, the differentially methylated region is hypomethylated in the primary tumour of mCRC patients in comparison to normal colon samples. In embodiments, the nucleic acid sequence of one or more differentially methylated regions is hypomethylated. In embodiments, the one or more biomarkers comprise or consist of all of the hypomethylated differentially methylated regions in Table 1.

In embodiments, the nucleic acid sequence of one or more differentially methylated regions is hypermethylated. In embodiments, the one or more biomarkers comprises or consists of all of the hypermethylated differentially methylated regions. In Table 1.

The markers listed in Table 1 have been developed and validated as a DNA methylation signature, allowing the detection of colorectal cancer or metastatic colorectal cancer by analysis of the DNA methylation pattern.

Accordingly a further aspect of the present invention, provides a method of screening for the presence of or diagnosing colorectal cancer in a subject, comprising
(a) obtaining a biological sample from a subject; and
(b) determining a methylation status of one or more differential methylated regions (DMR) selected from the group listed in Table 1;
wherein an alteration in methylation of said DMR in said sample relative to the level of methylation of said DMR in normal colorectal cells in indicative of colorectal cancer in said subject.

In embodiments, the alteration in methylation is increased or decreased methylation. A decrease in methylation can mean hypomethylation. In embodiments, the one or more DMRs are hypomethylated. An increase in methylation can mean hypermethylation. In embodiments, the one or more DMRs are hypermethylated.

In embodiments, the one or more differentially methylated regions comprise or consist of the nucleic acid sequences of SEQ ID Nos 1-376 or a functionally active fragment or variant thereof.

In embodiments, the one or more differentially methylated regions comprise or consist of DMR_240, DMR_236, DMR_148, DMR_276, DMR_33, DMR312, DMR_302 and/or DMR_75 or combinations thereof. In embodiments, the differentially methylated region DMR_240 comprises or consists of the nucleic acid sequence of SEQ ID NO:240 or a functionally active fragment or variant thereof. In embodiments, the differentially methylated region DMR_236 comprises or consists of the nucleic acid sequence of SEQ ID NO:236 or a functionally active fragment or variant thereof. In embodiments, the differentially methylated region DMR_148 comprises or consists of the nucleic acid sequence of SEQ ID NO:148 or a functionally active fragment or variant thereof. In embodiments, the differentially methylated region DMR_276 comprises or consists of the nucleic acid sequence of SEQ ID NO:276 or a functionally active fragment or variant thereof. In embodiments, the differentially methylated region DMR_33 comprises or consists of the nucleic acid sequence of SEQ ID NO:33 or a functionally active fragment or variant thereof. In embodiments, the differentially methylated region DMR_312 comprises or consists of the nucleic acid sequence of SEQ ID NO:312 or a functionally active fragment or variant thereof. In embodiments, the differentially methylated region DMR_302 comprises or consists of the nucleic acid sequence of SEQ ID NO:302 or a functionally active fragment or variant thereof. In embodiments, the differentially methylated region DMR_75 comprises or consists of the nucleic acid sequence of SEQ ID NO:75 or a functionally active fragment or variant thereof.

In embodiments, the one or more differentially methylated regions are DMR_240, DMR_236, DMR_148, DMR_276 and/or DMR_33. In embodiments, the differentially methylated region DMR_240 comprises or consists of the nucleic acid sequence of SEQ ID NO:240 or a functionally active fragment or variant thereof. In embodiments, the differentially methylated region DMR_236 comprises or consists of the nucleic acid sequence of SEQ ID NO:236 or a functionally active fragment or variant thereof. In embodiments, the differentially methylated region DMR_148 comprises or consists of the nucleic acid sequence of SEQ ID NO:148 or a functionally active fragment or variant thereof. In embodiments, the differentially methylated region DMR_276 comprises or consists of the nucleic acid sequence of SEQ ID NO:276 or a functionally active fragment or variant thereof. In embodiments, the differentially methylated region DMR_33 comprises or consists of the nucleic acid sequence of SEQ ID NO:33 or a functionally active fragment or variant thereof.

In embodiments, the one or more differentially methylated regions are DMR_302, DMR_312 and/or DMR_75. In embodiments, the differentially methylated region DMR_302 comprises or consists of the nucleic acid sequence of SEQ ID NO:302 or a functionally active fragment or variant thereof. In embodiments, the differentially methylated region DMR_312 comprises or consists of the nucleic acid sequence of SEQ ID NO:312 or a functionally active fragment or variant thereof. In embodiments, the differentially methylated region DMR_75 comprises or consists of the nucleic acid sequence of SEQ ID NO:75 or a functionally active fragment or variant thereof.

In embodiments, there are three differentially methylated regions. In embodiments, the differentially methylated regions are DMR_302, DMR_312 and DMR_75. In embodiments, there are two differentially methylated regions. In embodiments, the differentially methylated regions are DMR_302 and DMR_75. In embodiments, the differentially methylated regions are DMR_302 and DMR_312. In embodiments, the differentially methylated regions are DMR_312 and DMR_75. In embodiments, the differentially methylated region DMR_302 comprises or consists of the nucleic acid sequence of SEQ ID NO:302 or a functionally active fragment or variant thereof. In embodiments, the differentially methylated region DMR_75 comprises or consists of the nucleic acid sequence of SEQ ID NO:75 or a functionally active fragment or variant thereof.

In embodiments, the differentially methylated region is DMR_312. In embodiments, the differentially methylated region DMR_312 comprises or consists of the nucleic acid sequence of SEQ ID NO:312 or a functionally active fragment or variant thereof.

The DMRs listed in Table 1 can be used as therapeutic targets in the treatment of colorectal cancer.

Accordingly, a further aspect of the present invention provides one or more biomarkers for use in the treatment of colorectal cancer, wherein the biomarker comprises or consists of one or more differential methylated regions (DMR) selected from the group listed in Table 1.

In embodiments, the one or more differentially methylated regions comprise or consist of the nucleic acid sequences of SEQ ID Nos 1-376 or a functionally active fragment or variant thereof.

In embodiments, the nucleic acid sequence of one or more differentially methylated regions is hypomethylated.

In embodiments, the one or more biomarkers comprise or consist of all of the hypomethylated differentially methylated regions in Table 1

In embodiments, the differentially methylated region is DMR_334. In embodiments, the differentially methylated region DMR_334 comprises or consists of the nucleic acid sequence of SEQ ID NO:334 or a functionally active fragment or variant thereof. In embodiments, the one or more differentially methylated region is defined by the coordinates chr8:128235001-128235500 (DMR_334).

According to a further aspect of the present invention, there is provided a method for the treatment and/or prophylaxis of colorectal cancer, said method comprising the step of:
(i) administering to a subject in need thereof a therapeutically effective amount of a biomarker, wherein the biomarker comprises or consists of one or more differential methylated regions (DMR) selected from the group listed in Table 1.

In embodiments, the one or more differentially methylated regions comprise or consist of the nucleic acid sequences of SEQ ID Nos 1-376 or a functionally active fragment or variant thereof; or any combination thereof.

In embodiments, the differentially methylated region is DMR_334. In embodiments, the differentially methylated region DMR_334 comprises or consists of the nucleic acid sequence of SEQ ID NO:334 or a functionally active fragment or variant thereof. In embodiments, the one or more differentially methylated region is defined by the coordinates chr8:128235001-128235500 (DMR_334).

According to a further aspect of the present invention, there is provided a composition for use in the treatment and/or prophylaxis of colorectal cancer, said composition comprising a therapeutically effective amount of a biomarker, wherein the biomarker comprises or consists of one or more differential methylated regions (DMR) selected from the group listed in Table 1.

In embodiments, the one or more differentially methylated regions comprise or consist of the nucleic acid sequences of SEQ ID Nos 1-376 or a functionally active fragment or variant thereof; or any combination thereof.

In embodiments, the differentially methylated region is DMR_334. In embodiments, the differentially methylated region DMR_334 comprises or consists of the nucleic acid sequence of SEQ ID NO:334 or a functionally active fragment or variant thereof. In embodiments, the one or more differentially methylated region is defined by the coordinates chr8:128235001-128235500 (DMR_334).

According to a further aspect of the present invention, there is provided a pharmaceutical composition for the treatment and/or prophylaxis of colorectal cancer, wherein the composition comprises a biomarker, wherein the biomarker comprises or consists of one or more differential methylated regions (DMR) selected from the group listed in Table 1, along with a pharmaceutically acceptable excipient, diluent or carrier.

In embodiments, the one or more differentially methylated regions comprise or consist of the nucleic acid sequences of SEQ ID Nos 1-376 or a functionally active fragment or variant thereof; or any combination thereof.

In embodiments, the differentially methylated region is DMR_334. In embodiments, the differentially methylated region DMR_334 comprises or consists of the nucleic acid sequence of SEQ ID NO:334 or a functionally active fragment or variant thereof. In embodiments, the one or more differentially methylated region is defined by the coordinates chr8:128235001-128235500 (DMR_334).

In embodiments of the aspects of the present invention, the methylation is detected in the enhancer region of said gene.

In embodiments of the aspects of the present invention, the colorectal cancer is metastatic colorectal cancer.

In embodiments of the aspects of the present invention, the method or biomarkers or gene expression signature of the present invention can be used to predict the progression of colorectal cancer or the increased likelihood of progression of colorectal cancer from one stage to another. In embodiments of the aspects of the present invention, the stage is stage 0, stage 1, stage 2, stage 3 or stage 4 colorectal cancer and the colorectal cancer can progress from any one of these stages to any other stage.

In embodiments of the aspects of the present invention, the method or biomarkers or gene expression signature of the present invention can be used to diagnose if the subject has a particular stage of colorectal cancer. In embodiments of the aspects of the present invention, the stage is stage 0, stage 1, stage 2, stage 3 or stage 4 colorectal cancer.

In embodiments of the aspects of the present invention, the step of determining a methylation status is performed by one or more of the techniques selected from the group consisting of or comprising, nucleic acid amplification, polymerase chain reaction (PCR), methylation-specific PCR, real-time methylation-specific PCR, PCR assay using a methylation DNA-specific binding protein, quantitative PCR, DNA chip-based assay, pyrosequencing or bisulfate sequencing.

According to a further aspect of the present invention, there is provided a set of nucleic acid sequences, wherein the nucleic acid sequences are located in the enhancer region of a gene, wherein the nucleic acid sequences are differentially methylated in colorectal cancer and wherein the nucleic acid sequences are selected from the differentially methylated regions set forth in Table 1. In embodiments, the differentially methylated sequences comprise or consist of the nucleic acid sequences of SEQ ID NO:1-376 or a functionally active fragment or variant thereof.

According to a further aspect of the present invention, there is provided a set of nucleic acid primers and/or probes suitable for performing the inventive method, which primers and/or probes are specific for targeting one or more of the differentially methylated regions selected from Table 1. Such a set can be a set of PCR primers or a microarray comprising the probes that are well known to the person skilled in the art. In embodiments, the sgRNA sequences used to target DMR_334 are TGAGTCACGGAGTTGTCTAC (sgrna_1) (SEQ ID NO: 377) and GAAGGAAAGAAGAATCACTG (sgRNA_2) (SEQ ID NO: 378) (top strand sequences from double stranded DNA oligos).

According to a further aspect of the present invention, there is provided a kit for determining a methylation status of one or more differentially methylated regions of the invention.

In embodiments of the above aspects of the present invention, the biological sample contains DNA or RNA from the subject. In embodiments, the biological sample comprises nucleic acids.

In embodiments of the above aspects of the present invention, the biological sample is selected from the group consisting of tissue, blood, plasma, serum, stool, urine, urine supernatant, urine cell pellet, semen, colorectal secretions and colorectal cells. In embodiments, the biological sample of the subject is a colorectal tissue sample, preferably of a colorectal polyp, or a blood or serum sample taken from the circulatory system of the subject.

In embodiments of the above aspects of the present invention the subject is typically a human but also can be any mammal, including, but not limited to, a dog, cat, rabbit, cow, bird, rat, horse, pig, or monkey.

In embodiments of the above aspects of the present invention, the methods describes are in vitro methods.

It is understood that the present invention may be practiced using each differentially methylated region listed in Table 1 separately as a diagnostic or prognostic marker or a few marker DMRs/genes combined into a panel display format so that several marker DMRs/genes may be detected for overall pattern or listing of DMRs/genes that are methylated to increase reliability and efficiency. Furthermore, any of the DMRs identified in the present invention may be used individually or as a set of DMRs in any combination with any of the other DMRs that are recited herein. Alternatively, DMRs may be ranked according to their importance and weighted and together with the number of DMRs that are methylated, a level of likelihood of developing colorectal cancer or progressing to metastatic colorectal cancer may be assigned.

### Description

The present disclosure provides genetic signatures, comprising genomic CpG dinucleotide sequences, genes, and/or genomic regions, which are differentially methylated in individuals with colorectal cancer (CRC). The inventors of the present invention have surprisingly discovered a gene marker panel based on DNA methylation analysis to develop a tool for diagnosing CRC and preferably for predicting the likelihood of CRC progression to mCRC. The inventors have developed a tumour specific DNA methylation signature that primarily encompasses enhancer regions from mCRC patients. The DNA methylation signature of the present invention comprises or consists of 376 differentially methylated regions (DMRs), or a subset thereof. The DNA methylation signature can be used for prognosis of cancer, in particular a prediction of the progression of colorectal cancer. The DNA methylation signature can be used to predict the likelihood of a patient with CRC progressing to metatastic CRC (mCRC).

It is widely reported that up to 50% of CRC patients develop metastases over the course of the disease. mCRC development can occur in a patient with previously treated CRC (stage III) or *de novo* at stage IV, characterized by development of metastatic lesions at sites including the liver, lymph nodes and lungs. The molecular landscape of CRC includes features such as microsatellite instability and mismatch repair deficiency and sequential variants in genes such as *KRAS, NRAS* and *BRAF* [2, 3]. These molecular changes are included within molecular profiling panels applied to CRC patients and routinely used as clinical prognostic/diagnostic measures, which ultimately predicts patient survival and response to standard-of-care treatment. In contrast to CRC, until recently the genetic landscape of mCRC was poorly understood. However, combining patient-derived multi-*omic* data with *in vivo* models, the present inventors have identified novel copy number alteration-based clusters across large cohorts of mCRC patients, which predict outcome in these patients receiving bevacizumab and combination therapy [4, 5]. Despite these concerted efforts to molecularly characterize mCRC, there is a significant deficit in the understanding of the mechanisms that underpin development of metastasis in a CRC patient.

Several studies strongly indicate that epigenetic alterations, such as DNA methylation, play a significant role in regulating molecular processes in CRC. While, these studies involved the investigation of conventional regulatory regions, e.g. gene promoters [3], recent evidence suggests that altered methylation of alternative regulatory sites, such as gene enhancers, can significantly impact gene expression [10].

Despite the clinical relevance of DNA methylation in CRC, very little is known about the functional impact of this epigenetic modification in mCRC. Moreover, most DNA methylation signatures across various cancer types typically include conventional regulatory regions such as promoters. Therefore, identifying tumour-specific DNA methylation alterations in enhancers within mCRC particularly with the intent of using such alterations for predicting metastatic progression is not obvious.

DNA methylation is an attractive target for diagnosis because cell free methylated DNA can be detected in body-fluids like blood, plasma, serum, sputum, and urine from patients with cancerous neoplastic conditions and disease. It may also be detected in non-invasive samples, including stool. Stool may comprise cellular material from intestinal villi and tumor tissue allowing the detection of the inventive methylation markers due to the presence of tumor DNA in this tissue material. Preferably the inventive method is performed on such colon tissue found in stool. It may or may not be performed on stool blood or on samples comprising stool blood. Also, it is also possible to perform the inventive method on tumour tissue obtained from the colorectal wall, e.g. biopsy tissue.

Cancer specific DNA methylation patterns can be found in cell free DNA derived from serum, as tumours release substantial amounts of their DNA into the bloodstream. In particular, tumours which are flushed thoroughly by blood release considerable amounts of tumour DNA into the blood stream. Thus DNA methylation is well suited for noninvasive detection in materials like serum, plasma (blood plasma) and blood.

Hypomethylation of a DMR is present when there is a measurable decrease in methylation of the DMR. In some embodiments, a DMR can be determined to be hypomethylated when less than 50% of the methylation sites analysed are not methylated. Hypermethylation of a DMR is present when there is a measurable increase in methylation of the DMR. In some embodiments, a DMR can be determined to be hypermethylated when more than 50% of the methylation sites analyzed are methylated. Methods for determining methylation states are provided herein and are known in the art.

The methylation levels of non-amplified or amplified nucleic acids can be detected by any conventional means. For example, in some embodiments, PCR, methylation-specific PCR, real-time methylation-specific PCR, PCR assay using a methylation DNA-specific binding protein, quantitative PCR, DNA chip-based assay, pyrosequencing, and bisulfite sequencing can be used. In some embodiments, methylation levels are detected using targeted bisulfite sequencing. In some embodiments, methylation levels are detected using bisulfite sequencing using PCR followed by Sanger sequencing or droplet digital PCR. In some embodiments, methylation levels are detected using bisulfite sequencing accompanied by PCR and Next Generation Sequencing (NGS). Additional detection methods include, but are not limited to, bisulfite modification followed by any number of detection methods (e.g., probe binding, sequencing, amplification, mass spectrometry, antibody binding, etc.) methylation-sensitive restriction enzymes and physical separation by methylated DNA-binding proteins or antibodies against methylated DNA.

In one embodiment of the present invention, the method for detecting the methylation of a gene may comprise: (a) preparing a clinical sample containing DNA; (b) isolating DNA from the clinical sample; (c) amplifying the isolated DNA using primers capable of amplifying a fragment comprising the DMR of one or more of the genes of the signature of the present invention; and (d) determining whether the DMR was methylated.

In some embodiments, a computer-based analysis program is used to translate the raw data generated by the detection assay (e.g., the presence, absence, or amount of methylation of a given marker or markers) into data of predictive value for a clinician. The clinician can access the predictive data using any suitable means. Thus, in some preferred embodiments, the present invention provides the further benefit that the clinician, who is not likely to be trained in genetics or molecular biology, need not understand the raw data. The data is presented directly to the clinician in its most useful form. The clinician is then able to immediately utilise the information in order to optimize the care of the subject.

Nucleic acids isolated from a subject are obtained in a biological sample from the subject. If it is desired to detect colorectal cancer or stages of colorectal cancer progression, the nucleic acid may be isolated from colorectal tissue by scraping or biopsy. Such samples may be obtained by various medical procedures known to those of skill in the art.

In one aspect of the invention, the state of methylation in nucleic acids of the sample obtained from a subject is hypomethylation compared with the same regions of the nucleic acid in a subject not having colorectal cancer or metastatic colorectal cancer. Hypomethylation as used herein refers to the absence of methylated alleles in one or more nucleic acids. Nucleic acids from a subject not having a colorectal cancer or metastatic colorectal cancer contain detectable methylated alleles when the same nucleic acids are examined.

From a disease progression perspective, only limited routine diagnostic strategies allow prediction of CRC progression to metastasis thus presenting a significant unmet clinical need. Molecular signatures such as the one identified in the present invention, provide a novel insight into the role of epigenetic alterations at alternative regulatory regions which regulate key processes underpinning metastatic progression in CRC. In parallel, the differentially methylated signature of the present invention can also prove to be a vital diagnostic strategy to predict progression of CRC to metastasis.

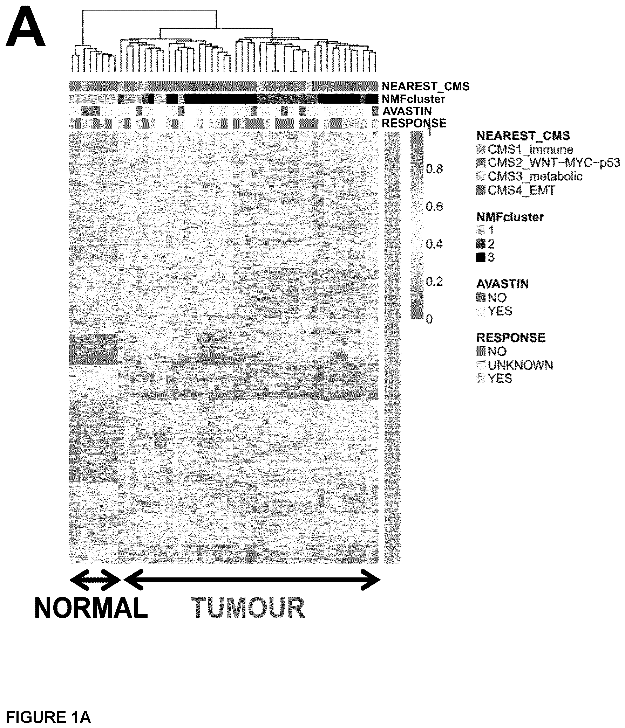

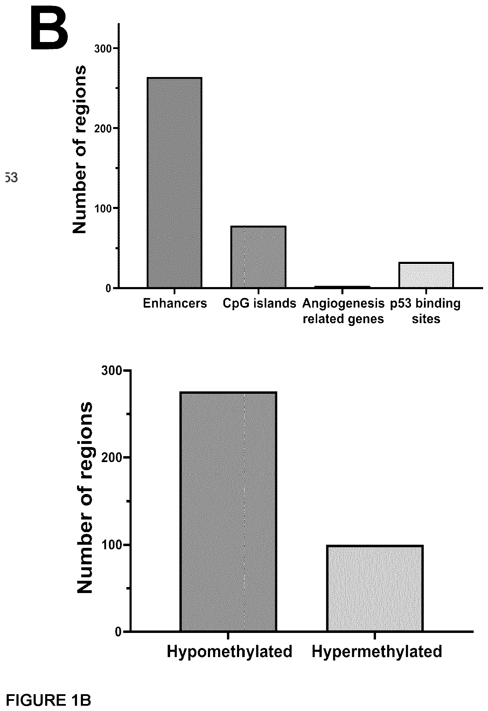

The inventors of the present invention investigated the role of enhancers in mCRC pathogenesis and identified a robust tumour-specific DNA methylation signature comprising or consisting of 376-differentially methylated loci (DMLs), or a subset thereof, which consisted predominantly (>90%) of gene enhancer regions (Figure 1).

The signature was further stratified into three distinct clusters (NMF1,2,3) and the present inventors determined the consensus molecular sub-types (CMS) present within this cohort. Interestingly, the present inventors showed that NMF1 and NMF2 clusters, as defined in the signature of the present invention, significantly overlapped (p=0.008, Fisher's-exact test) with CMS2 (Wnt-MYC-p53 signalling pathway subtype) and CMS4 (Epithelial mesenchymal transition subtype), respectively (Figure 1). This data thus strongly suggests the likely impact of the novel DNA methylation signature of the present invention, or a subset thereof, on processes underpinning mCRC development.

Surprisingly, the inventors have identified a novel 376-loci differentially methylated signature identified in mCRC tumours that encompasses enhancer regions and which likely plays a functional role in regulating expression of disease-associated genes, which ultimately impact molecular/cellular processes involved in mCRC pathogenesis.

The gene expression signature of the present invention comprises or consists of at least 98%, at least 95%, at least 90%, at least 80%, at least 70%, at least 60%, at least 50%, at least 40%, at least 30%, at least 20%, at least 10%, at least 5%, at least 1% or at least less than 1% of the DMRs listed in Table 1.

Given that the signature of the present invention was identified in stage IV metastatic CRC patients and together with the fact that genes associated with the loci within the signature appear to have an oncogenic potential, the inventors submit that this signature has a predictive potential in terms of predicting progression of CRC to mCRC. To this end, the inventors have assembled two unique and highly valuable clinical cohorts consisting of primary tumour material derived from III CRC clinical cohorts. These cohorts include stage III CRC patients who exhibit stable disease - "Non-progressors" (n=100) and stage III CRC patients who develop metastasis - "Progressors" (n=100). Through a proposed study, inventors applied a targeted bisulfite sequence capture panel which encompassed all the 376-loci contained within the DNA methylation signature. The small size of this capture (~2 Mb) allowed the inventors to carry out high-throughput sequencing, while maintaining a high sequence coverage (>100x). The inventors applied this capture panel to bisulfite converted DNA libraries generated from all tumour samples extracted from the two aforementioned cohorts using well-established experimental pipelines [17, 18]. Data generated was analysed using established bioinformatic pipeline, which allows generation of single-base pair resolution, high-confidence methylation calls across both strands of the DNA [17, 18, 20].

The inventor's original discovery data set allowed them to ascertain the precise methylation status of each loci in stage IV mCRC patients (Figure 1). Thus, in order to identify which of these loci are indeed specific to mCRC, the inventors compared the methylation status of all 376 loci between the stage IV (discovery cohort) (Figure 1) and stage III progressors. The loci exhibiting similar methylation profile across both groups, was compared to their corresponding methylation status determined in the stage III non-progressors group. The loci which demonstrated a different methylation pattern following this comparison, was thus deemed as *"methylation loci which likely predict mCRC development'.*

Next, the inventors assessed these loci against a range of clinical parameters and statistically examined using Fishers exact test to determine correlation of each loci to clinical variables including but not limited to TNM staging, grade, response to chemotherapy, neutrophil/T-lymphocyte count, BMI, survival.

Recent studies have shown strong utility of circulating tumour DNA (ctDNA) methylation in early detection of colorectal cancer [22-25] and a preferable route towards a non-invasive diagnostic strategy. With that in mind, the inventors evaluated the methylation status of the methylation sensitive loci/genes identified in ctDNA isolated from a sub-set (n=50) of the stage III progressor cohort. To this purpose, ctDNA was isolated from fresh blood collected from the patients using the widely published ctDNA extraction kit (Norgen). To examine the methylation status of the methylation sensitive loci, the inventors first carried out bisulfite conversion of the ctDNA (Zymo Gold bisulfite conversion kit). The bisulfite converted DNA was amplified using primers specific to the methylation sensitive loci and subsequently applied to EPITYPER mass spectrometry platform, which allows quantitative measurement of individual CpG sites [18]. Pearson's correlation analysis between methylation status determined from sequencing of primary tumours vs. methylation status of ctDNA derived from each patient, allowed the inventors to identify methylation sensitive loci that can be evaluated in a liquid biopsy collected from a mCRC patient.

Ultimately, the inventors have evaluated the metastatic predictive potential of the unique signature of the present invention with the likelihood of assessing this signature using ctDNA derived through non-invasive procedure such as routine blood draw.

As a whole, the 376-loci methylation signature of the present invention displayed high accuracy in differentiating normal colon samples from mCRC. Without being bound by theory, the inventors submit that methylation sequencing of all 376 DMRs or preferably a subset of DMRS would indicate that a patient with CRC would progress to mCRC. As methylation sequencing of all 376 DMRs may not be realistic in all clinical settings, the inventors used machine learning approaches to identify the most predictive DMRs. In total, five DMRs were identified as having a role in driving metastatic progression in CRC and having the greatest diagnostic and/or prognostic utility. These DMRs are DMR_240, DMR_236, DMR_148, DMR_276, DMR_33, four of which were hypomethylated enhancers. The inventors surprisingly discovered that differential methylation at these DMRs in particular showed the highest tumor-specificity and hence potential diagnostic value. The AUC of the 5-DMR diagnostic model in the Angiopredict cohort was 0.92 in comparison to 0.85 and 0.82 for the 376 loci in the training and test cohort respectively. Further to this, the reported AUC of the most widely known methylation biomarker for CRC, *SEPT9,* is 0.87. Therefore, for the first time the inventors present previously unidentified DMRs with prognostic utility that are not covered by known methylation microarrays. As *SEPT9* is currently a plasma-based non-invasive methylation biomarker, the methylation status of the DMRs in ctDNA/cfDNA warrants investigation in a larger CRC cohort.

The inventor's investigation into the prognostic utility of the 376 DMRs of the differentially methylated signature of the present invention revealed that the hypomethylated enhancers, DMR_302 and DMR_75, were associated with worse progression-free survival (PFS) in mCRC patients. Development of a prognostic risk score based on the two DMRs was associated with greater risk compared to factors such as age, tumour grade and location. DMR_302 is located within the gene body of *LINC-PINT,* which is a *p*53-transcript and tumour suppressor downregulated in many cancer types. On the other hand, the inventors found that DMR_75 forms chromatin loops with novel transcript *AC078820.1* (*Lnc-KRR1-4*) in HCT116 Hi-C data, which is a IncRNA associated with poor prognosis in CRC. Based on these findings, the inventors conclude that the two DMRs potentially control expression of IncRNAs with oncogenic functions and acts as a 2-DMR signature. Therefore, the inventors have surprisingly found that this 2-DMR signature of DMR_302 and DMR_75 acts as a beneficial diagnostic/prognostic tool or therapeutic target for CRC. In particular, the presence of this 2-DMR signature of DMR_302 and DMR_75 in a patient sample indicates that the patient with CRC has a likelihood of progressing to mCRC. In addition, the inventors submit that inhibition of these precise loci impedes metastasis of CRC and that DMR_302 and DMR_75 effectively are targets against which suitable targeted therapies can be effectively designed.

The inventor's findings from subsequent prognostic evaluation of individual CpGs in the FOCUS cohort revealed that low methylation levels at one particular CpG site: *cg01175550,* was associated with significantly lower overall survival (OS) and disease free survival (DFS). Moreover, in the GEO cohort utilized in this chapter, *cg01175550* is significantly hypermethylated in CRC compared to normal, and the DMR containing this CpG in the Angiopredict cohort, which is a CpG shore, is also hypermethylated. Thus, it is surprising that lower methylation in FOCUS mCRC patients confers worse prognosis. It is possible that hypermethylation at this loci in CRC may provide a protective effect, potentially through TSG or oncogene modulation and should hence be further examined to elucidate its precise mechanism.

Having evaluated the 376 DMRs in stage IV mCRC patients, the inventors next turned to an additional CRC cohort which included tubular adenoma patients. Advanced adenomas are often regarded as precursors for CRC, thus development of biomarkers to aid early diagnosis or predict progression is required. In total, the inventors identified four CpGs differentially methylated between normal, adenoma and CRC patients. As with previous diagnostic markers identified in this chapter, the methylation status of these loci should be validated in cfDNA/ctDNA for non-invasive biomarker discovery going forward. Strikingly, of the 201 CpGs overlapping with the 376 DMRs of the present inveniton, 82% of these were differentially methylated between the adenoma-high and adenoma-low methylation clusters identified. As clustering between adenoma-H and CRC was observed, longitudinal studies are needed to examine whether any of the DMPs in patients with adenoma-H polyps confer risk for future CRC development. Furthermore, examination of the methylation status of these CpGs in villous and serrated adenomas is warranted, given that patients with these have an increased risk of CRC.

In conclusion, this additional data provides critical insights into the prognostic, diagnostic and potential therapeutic utility of various DMRs within the 376-DMR signature, CpG sites and enhancer gene targets in CRC.

In particular, the present invention provides a method for predicting the progression of colorectal cancer in a subject and/or screening for an increased likelihood of progression of colorectal cancer in a subject, the method comprising:
- providing a biological sample which has been obtained from the subject;
- testing the biological sample for the presence or absence of a gene expression signature or a subset thereof;

wherein the gene expression signature, or a subset thereof, comprises or consists of one or more of the genes set forth in Table 1;
wherein the presence of the gene expression signature, or a subset thereof, indicates an increased likelihood of disease progression.

The present invention also provides a method of screening for the presence of or diagnosing colorectal cancer in a subject, comprising
(a) obtaining a biological sample from a subject; and
(b) determining a methylation status of one or more genes selected from the group listed in Table 1;
wherein a decreased degree of methylation of said gene in said sample relative to the level of methylation of said gene in normal colorectal cells in indicative of colorectal cancer in said subject.

### Colorectal cancer biomarker

The inventors submit that certain DMRs are very useful as targets for therapeutics for use in the treatment of CRC and mCRC, for example the DMR_334 enhancer. As the second ranking depleted hit from the CRISPRi screen was identified as an enhancer DMR derived from the original 376-DMR methylation signature (DMR_334), which was the only depleted DMR in the screen, the inventors focused on this for further investigation. Through literature search the inventors found that the 500bp hypomethylated enhancer is likely part of the *CCAT1-L* super-enhancer *(CCAT1-L* SE) gained in CRC, based on its location on chromosome 8. Furthermore, the 500bp DMR contains two SNPs, one of which (rs35813501) is associated with breast cancer risk in Asian women. However, given as CRISPRi induces silencing as opposed to knockout of target regions, it is unlikely that the anti-proliferative effect was associated with this mutation.

The *CCAT1-L* SE is well studied in CRC, however, for the first time the inventors show that this specific 500bp DMR is uniquely essential for HCT116 proliferation in comparison to five other hypomethylated DMRs spanning this super-enhancer region, which were also included in the screen however did not demonstrate any impact on the CRC-associated phenotype. Furthermore, the inventors submit that this is the first report of differential methylation at this SE in CRC patient tumors. While DNA methylation is known to affect enhancer loci, we do not yet know if loss of methylation at the essential DMR drives enhancer activity or target gene expression. Nevertheless, correlation analysis revealed that DMR_334 hypomethylation is associated with increased expression of CCAT1 and *CASC19.* Both IncRNAs are reportedly elevated in CRC, consistent with the knowledge that the chromosome 8.q24.21 locus is frequently amplified in CRC.

Comparison of stage IV CRC patients with high or low levels of DMR_334 methylation revealed that the majority of NMF3 patients from the Angiopredict cohort had low levels of enhancer methylation. As discussed above, it was identified that the majority of NMF3 patients are CMS4, the subtype with worse overall survival rates predominantly associated with CIMP negativity. While the inventors observed a trend of patients with lower methylation at DMR_334 having worse overall survival (OS) and CMS4, these did not reach significance. Ultimately, continued recruitment of stage IV CRC validation cohort patients will allow us to better understand the impact of DMR_334 methylation on molecular and clinical characteristics, given that methylation at this region is unfortunately not covered by illumina microarrays.

Through investigation of HCT116 Hi-C data, interactions between the essential enhancer and *PVT1, TMEM75* and CCDC26 were identified. *PVT1* is a well-studied oncogene in numerous cancers, while *TMEM75* can promote CRC by activating SIM2. While it is possible that the enhancer may interact with or regulate these IncRNAs, further validation is required given that the interactions were only identified in one replicate. Moreover, as previously mentioned, use of publicly available Hi-C data is also limited given that it is not targeted directly to our regions of interest. With this in mind, the inventors have carried out 4C-Seq to identify all genomic regions interacting specifically with this 500bp enhancer in HCT116 cells. As correlation between the enhancer methylation and *CCAT1* and *CASC19* expression was identified, it will be interesting to determine if interactions are evident between these loci, or potentially interactions between the DMR and *MYC,* given that loop formation between *MYC* and the *CCAT1-L* super-enhancer, which the essential DMR is located in, has been reported.

Motif analysis subsequently revealed that the YY1 TF motif had highest predicted enrichment in the essential enhancer. YY1 is highly expressed In CRC, with conflicting roles depending on chromatin state and binding partners. Additionally, a study previously reported that methylation of the CpG site present in the YY1 binding site results in abrogation of YY1 binding *in vitro.* Thus, the inventors have assessed if a similar mechanism occurs at DMR_334 by performing CRISPRi-mediated methylation of this binding site within the enhancer followed by ChIP-Seq pulldown of the YY1 TF.

The inventors found that enhancer repression resulted in abrogation of CCAT1 expression and strikingly reduced *MYC* expression by -75%. One study has previously reported that knockdown of *CCAT1-L,* transcribed by the SE, reduced *MYC* expression by -20% in HCT116 cells. Thus, it is possible that the 500bp DMR is highly important for the interaction between *CCAT1-L* and *MYC,* or potentially contains binding sites for transcriptional activity between these regions.

To the inventors' knowledge, whole transcriptome analysis of the *CCAT1-L* SE has not been documented. 12 genes commonly regulated by both sgRNAs were identified including two SLC family genes involved in glycometabolism and *ST6Gal1* and *ISPD,* both involved in glycosylation. This suggests that the enhancer is strongly linked to genes involved in various metabolic processes such as glycolysis, which cancer cells are historically known to be heavily dependent on for growth. This may explain the profound decrease in HCT116 growth observed upon enhancer repression. Remarkably, gene ontology analysis showed that numerous ontology terms associated with ribosomal pathways were downregulated upon enhancer repression with sgRNA1. This is consistent with the downregulation of the translation elongation reactome pathway also seen upon enhancer repression, which involves structural rearrangement of ribosomes. *MYC* is widely known as a key regulator of ribosomal biogenesis. Potentially, the profound downregulation of *MYC* observed upon enhancer repression in both the qPCR and RNA-Seq data, may explain the downregulation of these pathways, suggesting that the enhancer is crucial for the physiological functions of *MYC.*

However, given the pleotropic regulatory nature of this enhancer (as seen from initial HiC-based interactions, further validation required), while MYC indeed appears to be a target gene, it is likely that these gene expression alterations as well as phenotypic changes are driven by other critical mediators of molecular functions. This thus needs to be explored further through in-depth in vitro perturbation and rescue experiments.

Upregulation of barrier function pathways such as those involving cellular and anchoring junctions was identified in enhancer knockdown CRC cells. In cancer, loss of adhesion ability is a hallmark of EMT, underpinned by the downregulation of cell adhesion molecules. This can result in anchoring junctions, such as desmosomes, with reduced integrity and adhesion, which is associated with an invasive phenotype. As such, upregulation of these pathways upon enhancer repression may be beneficial in restoring HCT116 adhesion ability.

Subsequent gene set enrichment analysis (GSEA) indicated downregulation of the oxidative phosphorylation (oxphos) hallmark pathway in addition to a trend in *MYC* target downregulation upon enhancer repression. It is noteworthy that Oxphos was also identified as the top negatively regulated pathway in the KEGG pathway analysis. This is interesting given that *MYC* is known to coordinate metabolic pathways including oxphos and glycolysis eluded to earlier. Accumulating evidence has shown that CRC cells can switch their metabolism, which is usually reliant on aerobic glycolysis, to oxphos to support increased proliferation and migration. Thus, downregulation of these pathways, either *via MYC* and its target genes or other molecular mediators are potentially regulated by the essential enhancer, may be favorable in targeting CRC cell metabolism.

Finally, CRISPRi-mediated enhancer silencing also revealed that chromatin organization was the top upregulated reactome pathway in HCT116 cells. This suggests that silencing of the enhancer, which prevents TF binding and transcriptional activity, may have altered the chromatin architecture at this region, particularly because the enhancer potentially interacts with multiple genes. Whether or not the upregulation of chromatin organization is beneficial in altering interactions with oncogenes remains unknown and can only be confirmed by performing 4C-Seq before and after enhancer repression.

In conclusion, the additional data shown herein identifies novel gene enhancers that are essential for HCT116 CRC cell proliferation, identified using a CRISPRi-LOF screen. The inventors identified one essential differentially methylated enhancer derived from the 376-DMR methylation signature that is part of a larger super-enhancer know to regulate *MYC* expression. Uniquely, the inventors found that targeting this 500bp region results in significant inhibition of CRC cell proliferation compared to other areas of the super-enhancer and for the first time showed that hypomethylation along the super-enhancer is associated with increased expression of *CCAT1.* Without being bound by theory, the inventors submit that any hypomethylated gene from the 376-DMR methylation signature of the present invention could be used as a novel therapeutic target for CRC or mCRC.

### Definitions

Unless otherwise defined, all technical and scientific terms used herein have the meaning commonly understood by a person who is skilled in the art in the field of the present invention.

The term 'disease progression' as used herein can be determined as colorectal cancer that has metastasised or has the likelihood of metastasising. Disease progression can mean that colorectal cancer progresses from any one stage to another, namely from stage 0, stage 1, stage 2, stage 3 or stage 4.

The term 'gene expression signature' as used herein is a single or combined group of genes with a uniquely characteristic pattern of gene expression that occurs as a result of an altered or unaltered biological process or pathogenic medical condition. The phenotypes that may theoretically be defined by a gene expression signature range from those that predict the survival or prognosis of an individual with a disease, those that are used to differentiate between different subtypes of a disease, to those that predict activation of a particular pathway. Ideally, gene signatures can be used to select a group of patients for whom a particular treatment will be effective.

The term 'differentially methylated regions (DMRs) as used herein can be determined as genomic regions with different DNA methylation status across different biological samples and regarded as possible functional regions involved in gene transcriptional regulation. DNA is mostly methylated at a CpG site, which is a cytosine followed by a guanine. The "p" refers to the phosphate linker between them. DMR usually involves adjacent sites or a group of sites close together that have different methylation patterns between samples. CpG islands appear to be unmethylated in most of the normal tissues, however, are highly methylated in cancer tissues. There are several different types of DMRs. These include tissue-specific DMR (tDMR), cancer-specific DMR (cDMR), development stages (dDMRs), reprogramming-specific DMR (rDMR), allele-specific DMR (AMR), and aging-specific DMR (aDMR). DNA methylation is associated with cell differentiation and proliferation.

The term 'hpomethylation' as used herein describes the unmethylated state of most CpG sites in a specific sequence that is normally methylated. Hypomethylation is an epigenetic change in human cancer were losses of DNA methylation (m⁵C residues replaced by unmethylated C residues) occur. Metastases are even more susceptible to cancer-linked DNA hypomethylation than primary tumors. Hypomethylation of a DMR is present when there is a measurable decrease in methylation of the DMR. In some embodiments, a DMR can be determined to be hypomethylated when less than 50% of the methylation sites analysed are not methylated.

"Hypermethylation" of a DMR is present when there is a measurable increase in methylation of the DMR. In some embodiments, a DMR can be determined to be hypermethylated when more than 50% of the methylation sites analyzed are methylated. Methods for determining methylation states are provided herein and are known in the art.

The term "methylation status" means the level of methylation of cytosine residues (found in CpG pairs) in the gene of interest. When used in reference to a CpG site, the methylation status may be methylated or unmethylated. When used in reference to a CpG island or to any stretch of residues, the methylation status refers to the level of methylation, which is the relative or absolute concentration of methylated C at the particular CpG island or stretch of residues in a biological sample. Methylation of a CpG site or island at a promoter usually prevents expression of the gene. The sites or islands can also surround the 5' region of the coding region of the gene as well as the 3' region of the coding region. Thus, CpG sites or islands can be found in multiple regions of a nucleic acid sequence including upstream of coding sequences in a regulatory region including a promoter region, in the coding regions (e.g., exons), downstream of coding regions in, for example, enhancer regions, and in introns. All of these regions can be assessed to determine their methylation status, as appropriate. The levels of methylation of the gene of interest are determined by any suitable means in order to reflect whether the gene is likely to be downregulated or not.

The term "gene" refers to a nucleic acid (e.g., DNA) sequence that comprises coding sequences necessary for the production of a polypeptide, precursor, or RNA (e.g., rRNA, tRNA). The polypeptide can be encoded by a full length coding sequence or by any portion of the coding sequence so long as the desired activity or functional properties (e.g., enzymatic activity, ligand binding, signal transduction, immunogenicity, etc.) of the full-length or fragments are retained. The term also encompasses the coding region of a structural gene and the sequences located adjacent to the coding region on both the 5' and 3' ends for a distance of about 1 kb or more on either end such that the gene corresponds to the length of the full-length mRNA. Sequences located 5' of the coding region and present on the mRNA are referred to as 5' non-translated sequences. Sequences located 3' or downstream of the coding region and present on the mRNA are referred to as 3' non-translated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

The term "nucleic acid" is used broadly herein to mean a sequence of deoxyribonucleotides or ribonucleotides that are linked together by a phosphodiester bond. As such, the term "nucleic acid" or "nucleic acid sequence" is meant to include DNA and RNA, which can be single stranded or double stranded, as well as DNA/RNA hybrids. Furthermore, the term "nucleic acid" as used herein includes naturally occurring nucleic acid molecules, which can be isolated from a cell, as well as synthetic molecules, which can be prepared, for example, by methods of chemical synthesis or by enzymatic methods such as by the polymerase chain reaction (PCR), and, in various embodiments, can contain nucleotide analogs or a backbone bond other than a phosphodiester bond.

Any nucleic acid may be used in the present invention, given the presence of differently methylated CpG islands can be detected therein. The "CpG island" is a CpG-rich region in a nucleic acid sequence. A CpG island is capable of being differentially methylated. CpG islands have an average G*C content of about 60%, compared with the 40% average in bulk DNA. The islands take the form of stretches of DNA typically about one to two kilobases long. There are about 45,000 islands in the human genome. In many genes, the CpG islands begin just upstream of a promoter and extend downstream into the transcribed region. Methylation of a CpG island at a promoter usually suppresses expression of the gene. The islands can also surround the 5' region of the coding region of the gene as well as the 3' region of the coding region. Thus, CpG islands can be found in multiple regions of a nucleic acid sequence including upstream of coding sequences in a regulatory region including a promoter region, in the coding regions (e.g., exons), downstream of coding regions in, for example, enhancer regions, and in introns. Nucleic acids contained in a sample used for detection of methylated CpG islands may be extracted by a variety of techniques known to the person skilled in the art.

The terms "polynucleotide" and "oligonucleotide" also are used herein to refer to nucleic acid molecules. Although no specific distinction from each other or from "nucleic acid" is intended by the use of these terms, the term "polynucleotide" is used generally in reference to a nucleic acid molecule that encodes a polypeptide, or a peptide portion thereof, whereas the term "oligonucleotide" is used generally in reference to a nucleotide sequence useful as a probe, a PCR primer, an antisense molecule, or the like. Of course, it will be recognised that an "oligonucleotide" also can encode a peptide.

By `functionally active' is meant an nucleic acid sequence of the DMRs of the present invention wherein the administration of DMR to a subject or expression of DMR in a subject promotes suppression of colorectal cancer. Further, functional activity may be indicated by the ability of a DMR of the present invention to suppress an immune response to a colorectal cancer. Further functionally active may mean a nucleic acid sequence of the DMR of the present invention that is capable of diagnosing colorectal cancer or predicting the likelihood of colorectal cancer progressing to metastatic colorectal cancer.

A 'fragment' can comprise at least 50, preferably 100 and more preferably 150 or greater contiguous amino acids from SEQ ID NOs:1-376 and which is functionally active. Suitably, a fragment may be determined using, for example, C-terminal serial deletion of cDNA. Said deletion constructs may then be cloned into suitable plasmids. The activity of these deletion mutants may then be tested for biological activity as described herein. Fragments may be generated using suitable molecular biology methods as known in the art.

By 'variant' is meant an amino acid sequence which is at least 90% homologous to SEQ ID NOs:1-376, even more preferably at least 95% homologous to SEQ ID NOs:1-376, even more preferably at least 96% homologous to SEQ ID NOs:1-376, even more preferably at least 97% homologous to SEQ ID NOs:1-376, and most preferably at least 98% homology with SEQ ID NOs:1-376. A variant encompasses a polypeptide sequence of SEQ ID NOs:1-376 which includes substitution of amino acids, especially a substitution(s) which is/are known for having a high probability of not leading to any significant modification of the biological activity or configuration, or folding, of the protein. These substitutions, typically known as conserved substitutions, are known in the art. For example the group of arginine, lysine and histidine are known interchangeable basic amino acids. Suitably, in embodiments amino acids of the same charge, size or hydrophobicity may be substituted with each other. Suitably, any substitution may be selected based on analysis of amino acid sequence alignments of interferon alpha subtypes to provide amino acid substitutions to amino acids which are present in other alpha subtypes at similar or identical positions when the sequences are aligned. Variants may be generated using suitable molecular biology methods as known in the art.

The term "diagnosed," as used herein, refers to the recognition of a disease by its signs and symptoms, or genetic analysis, pathological analysis, histological analysis, and the like. "Diagnosing" may or may not be used to provide information of a 100% sure cancer presence, but is usually used to describe a risk or likelihood of cancer presence. Such a risk or likelihood may be at least 60%, at least 70%, at least 80% at least 90% at least 95% or at least 98% probability.

In the context of the present invention "prognosis", "prediction" or "predicting" should not be understood in an absolute sense, as in a certainty that an individual will develop cancer (including cancer progression), but as an increased risk or likelihood to develop cancer or of cancer progression. "Prognosis" is also used in the context of the invention for predicting cancer progression, in particular to predict therapeutic results of a certain therapy of the colorectal cancer. The prognosis of a therapy can e.g. be used to predict a chance of success (e.g. treating cancer with cancerous DNA methylation marker states being below detection levels) or chance of reducing the severity of the disease to a certain level. The inventive marker sets may also be used to monitor a patient for the emergence of therapeutic results or positive disease progressions.

The term "treatment" is used herein to refer to any regimen that can benefit a human or non-human animal. The term "treatment" and associated terms such as "treat" and "treating" means the reduction of the progression, severity and/or duration of colorectal cancer or at least one symptom thereof, wherein said reduction or amelioration results from the administration of the biomarker or composition of the present invention. The treatment may be in respect of colorectal cancer and the treatment may be prophylactic (preventative treatment). Treatment may include curative or alleviative effects. Reference herein to "therapeutic" and "prophylactic" treatment is to be considered in its broadest context. The term "therapeutic" does not necessarily imply that a subject is treated until total recovery. Similarly, "prophylactic" does not necessarily mean that the subject will not eventually contract a disease condition. Accordingly, therapeutic and/or prophylactic treatment includes amelioration of the symptoms of colorectal cancer or preventing or otherwise reducing the risk of developing colorectal cancer or metastatic colorectal cancer. The term "prophylactic" may be considered as reducing the severity or the onset of a particular condition. "Therapeutic" may also reduce the severity of an existing condition.

'Pharmaceutical compositions' according to the present invention, and for use in accordance with the present invention, may comprise, in addition to an active ingredient, a pharmaceutically acceptable excipient, carrier, buffer stabiliser or other materials well known to those skilled in the art. Such materials should be non-toxic and should not interfere with the efficacy of the active ingredient. The precise nature of the carrier or other material will depend on the route of administration, which may be, for example, oral, intravenous, intranasal or via oral or nasal inhalation. The formulation may be a liquid, for example, a physiologic salt solution containing non-phosphate buffer at pH 6.8-7.6, or a lyophilised or freeze-dried powder.

The composition of the invention is typically administered to a subject in a "therapeutically effective amount", this being an amount sufficient to show benefit to the subject to whom the composition is administered. The actual dose administered, and rate and time-course of administration, will depend on, and can be determined with due reference to, the nature and severity of the condition which is being treated, as well as factors such as the age, sex and weight of the subject being treated, as well as the route of administration. Further due consideration should be given to the properties of the composition, for example, its binding activity and in-vivo plasma life, the concentration of the antibody or binding member in the formulation, as well as the route, site and rate of delivery.

Dosage regimens can include a single administration of the composition, or multiple administrative doses of the composition. The compositions can further be administered sequentially or separately with other therapeutics and medicaments which are used for the treatment of the condition for which the composition of the present invention is being administered to treat.

Examples of dosage regimens which can be administered to a subject can be selected from the group comprising, but not limited to; 1µg/kg/day through to 20mg/kg/day, 1µg/kg/day through to 10mg/kg/day, 10µg/kg/day through to 1mg/kg/day. In certain embodiments, the dosage will be such that a plasma concentration of from 1µg/ml to 100µg/ml of the DMR is obtained. However, the actual dose of the composition administered, and rate and time-course of administration, will depend on the nature and severity of the condition being treated. Prescription of treatment, e.g. decisions on dosage etc, is ultimately within the responsibility and at the discretion of general practitioners and other medical doctors, and typically takes account of the disorder to be treated, the condition of the individual patient, the site of delivery, the method of administration and other factors known to practitioners.

As used herein, the term "subject" refers to any organisms that are screened using the diagnostic methods described herein. Such organisms preferably include, but are not limited to, mammals (e.g., murines, simians, equines, bovines, porcines, canines, felines, and the like), and most preferably includes humans. The subject may have had a colorectal cancer or he may have had no history of colorectal cancer, in particular if the subject is suspected of having colorectal cancer. The invention may be used as a routine test even without any suspect of the subject having colorectal cancer. If the subject has colorectal cancer, the invention may be used to determine the likelihood of the subject progressing to metastatic colorectal cancer.

As used herein, the term "sample" is used in its broadest sense. In one sense, it is meant to include a specimen or culture obtained from any source, as well as biological and environmental samples. Biological samples may be obtained from animals (including humans) and encompass fluids, solids, tissues, and gases. Biological samples include blood products, such as plasma, serum and the like. Such examples are not however to be construed as limiting the sample types applicable to the present invention.

In the present invention, "normal" cells refer to those that do not show any abnormal morphological or cytological changes. "Tumor" cells are cancer cells. "Non-tumor" cells are those cells that are part of the diseased tissue but are not considered to be the tumor portion.

As used herein, the terms "detect", "detecting" or "detection" may describe either the general act of discovering or discerning or the specific observation of a detectably labeled composition.

Throughout the specification, unless the context demands otherwise, the terms 'comprise' or 'include', or variations such as 'comprises' or 'comprising', 'includes' or 'including' will be understood to imply the inclusion of a stated integer or group of integers, but not the exclusion of any other integer or group of integers.

### Brief description of the Figures

The invention will be more clearly understood from the following description of some embodiments thereof, given by way of example only, with reference to the following figure in which:
Figure 1A-B demonstrates a heatmap which shows a tumour specific differentially methylated signature derived mathylcapture sequencing of 55 mCRC patients.

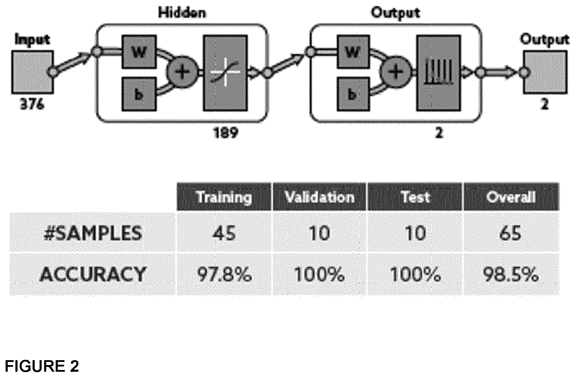

Figure 2 demonstrates implementation of a shallow neural network to classify tumour vs normal patients. The network was trained using scale conjugate gradient backpropagation. The 65 (55 mCRC tumours + 10 normal samples) were randomly divided into 70% training data, 15% validation and 15% test data. The overall accuracy of the model was 98.5%.

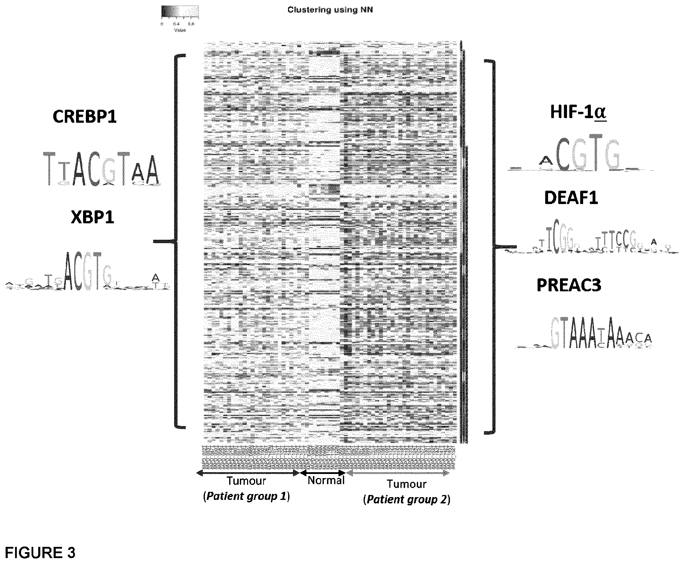

Figure 3 demonstrates clustering methylation data using ANN. Figure 3 shows a heatmap generated using the 378 DML signature of the present invention which shows two distinct patient tumour clusters, with normal samples clustered in the centre as indicated. TFBS motif analysis shows distinct enrichments for TF's between the two clusters.

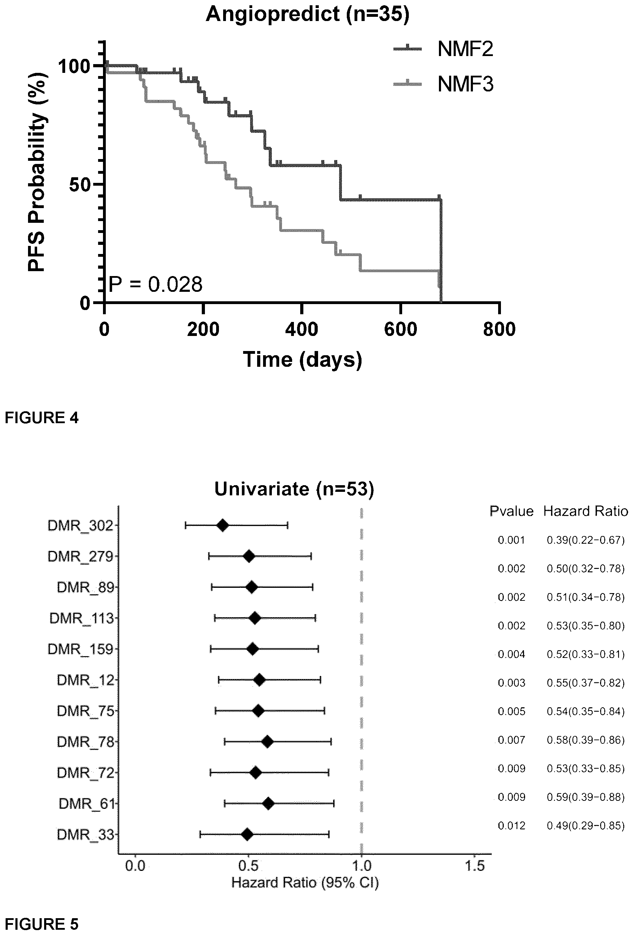

Figure 4 is a graph demonstrating that the NMF3 patient methylation cluster have significantly worse progression-free survival (PFS) compared to NMF2.
Figure 5 demonstrates DMRs associated with PFS in the Angiopredict cohort depicted in Figure 4. The forest plot shows univariate Cox analyses of DMRS significantly associated with PFS on the y-axis and the Hazard Ratio (HR) on the x-axis. The dotted line indicates a HR=1, meaning no effect. P-values are also show in addition to HR values with 95% confidence intervals.

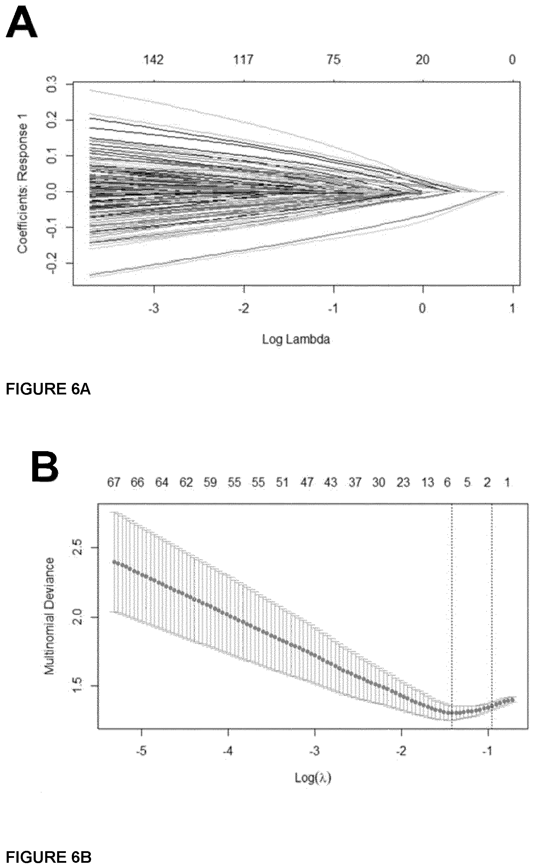

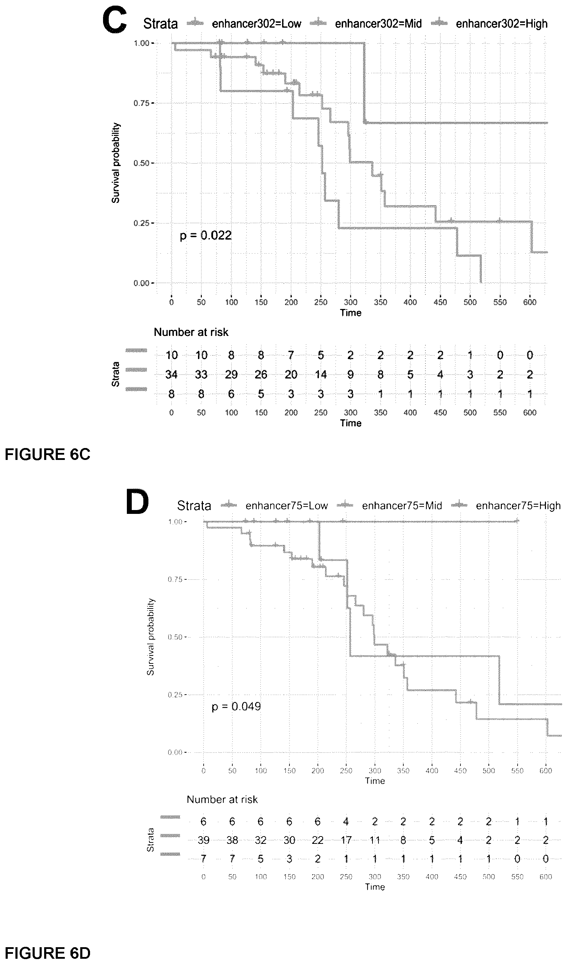

Figure 6A-D demonstrates stratification of prognostic DMRs in mCRC patients. Figure 6A demonstrates a spectrum of lasso coefficients for the 376 DMRs shown on the y-axis against the log (lambda) sequence generated by a single-fold LASSO-penalised model shown on the x-axis. The coloured curves denote the 376 DMRs. Those with non-zero coefficients correspond to DMRs with potential use for feature selection. Figure 6B demonstrates 10-fold cross-validation for candidate DMR selection. The red dotted lines denote the cross-validation curve with every red point depicting deviance with upper and lower standard error. Dashed vertical lines indicate the log(lambda) values corresponding to lambda.min and lambda.1se. Numbers on top of the plot represent the number of DMR non-zero coefficients in the model. As lambda increases, fewer DMRs remain. Figure 6C and 6D demonstrate Kaplan-Meier survival curves of mCRC patients stratified with low, medium or high methylation at DMR302 (Figure 6C) and DMR_75 (Figure 6D).

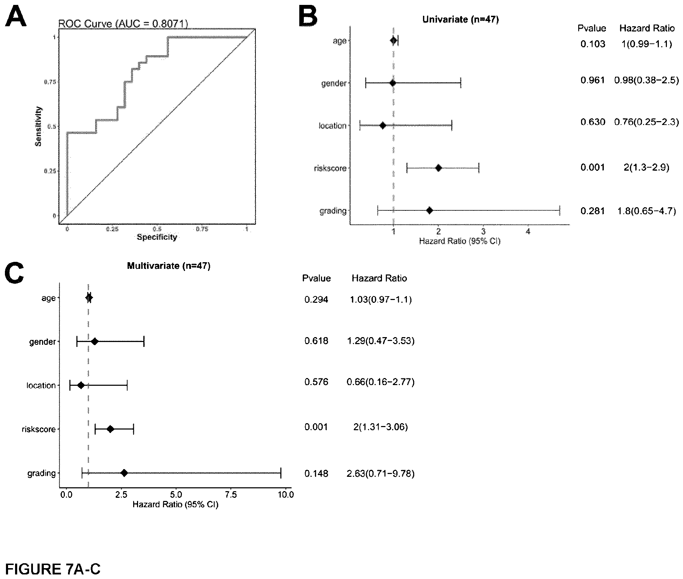

Figure 7A-C demonstrates utility of the prognostic risk model in predicting PFS in mCRC patients. Figure 7A is a ROC curve of the prognostic model constructed with two hypomethylated DMRs: DMR_302 and DMR_75. AUC: Area Under Curve. Figure 7B is a forest plot of univariate Cox analysis of the DMR methylation risk score and other clinical risk factors. Figure 7C is a forest plot of multivariate Cox analysis of the DMR methylation risk score and other clinical risk factors.

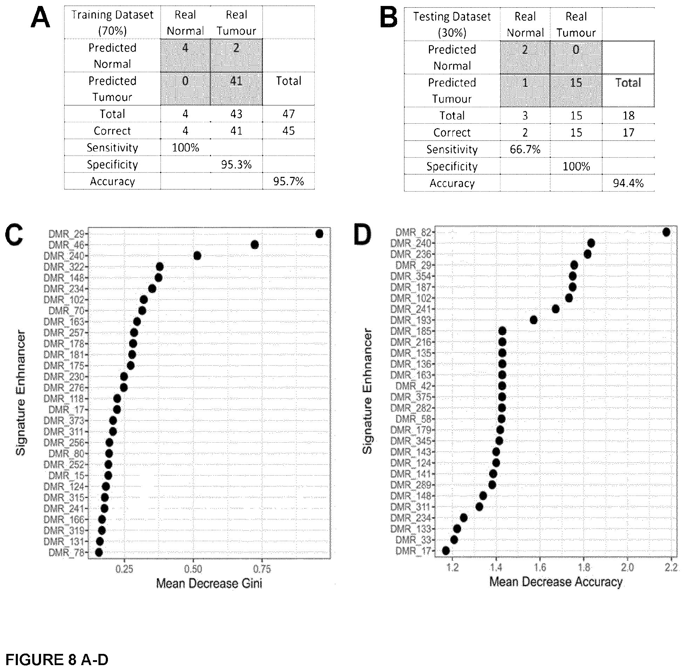

Figure 8A-D demonstrates evaluation of the methylation signature's diagnostic utility. Figure 8A and Figure 8B demonstrate confusion matrices showing the classifier's predictions in differentiating normal from mCRC samples in the split training and testing groups. Figure 8C and Figure 8D demonstrate importance plots show the top 30 DMRs with highest mean decrease Gini scores and highest mean decrease accuracy scores.

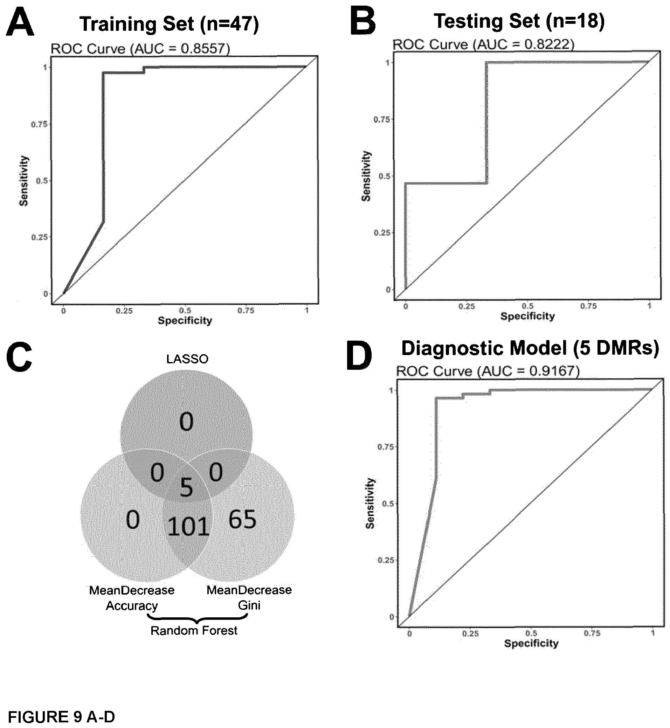

Figure 9A-D demonstrates construction of a 5-DMR diagnostic model. Figures 9A and 9B are ROC curves showing the accuracy of the full 376-loci methylation signature in classifying normal vs tumour samples in the split (A) training and (B) testing groups of Angiopredict patients. Figure 9C is an intersection between potential diagnostic predictors in the random forest and LASSO penalised regression analysis. Figure 9D is a ROC curve of the diagnostic model constructed with five DMRs; DMR_240, DMR_236, DMR_148, DMR_276, DMR_33. AUC: Area Under Curve.

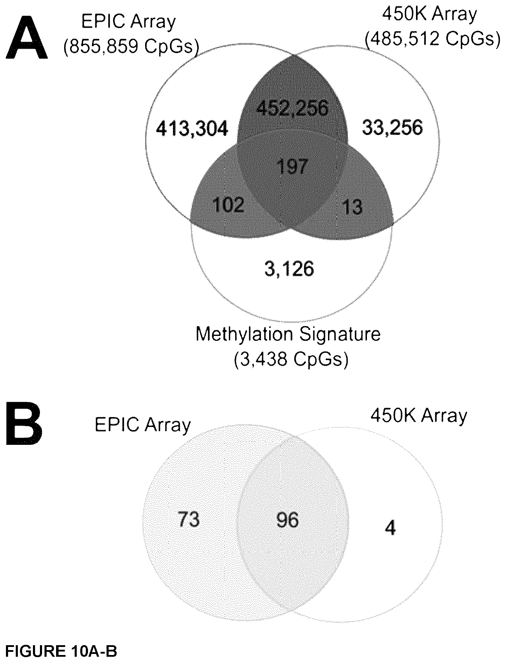

Figure 10A-B demonstrates the evaluation of the signature of the present invention in publically available CRC datasets. Figure 10A shows the number of CpG sites present in the 376-DMR signature overlapping with the 450K and EPIC array CpGs. Figure 10B shows the number of signature loci that contain at least one CpG from the EPIC and 450K array.

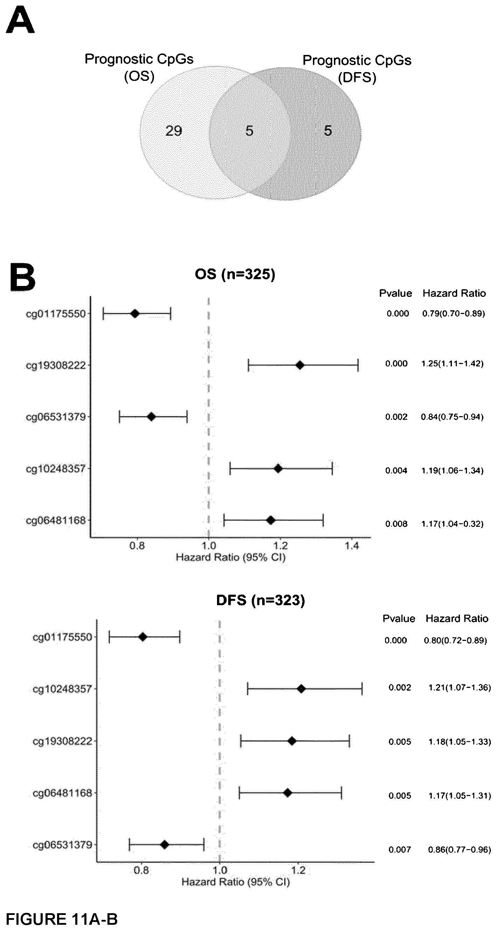

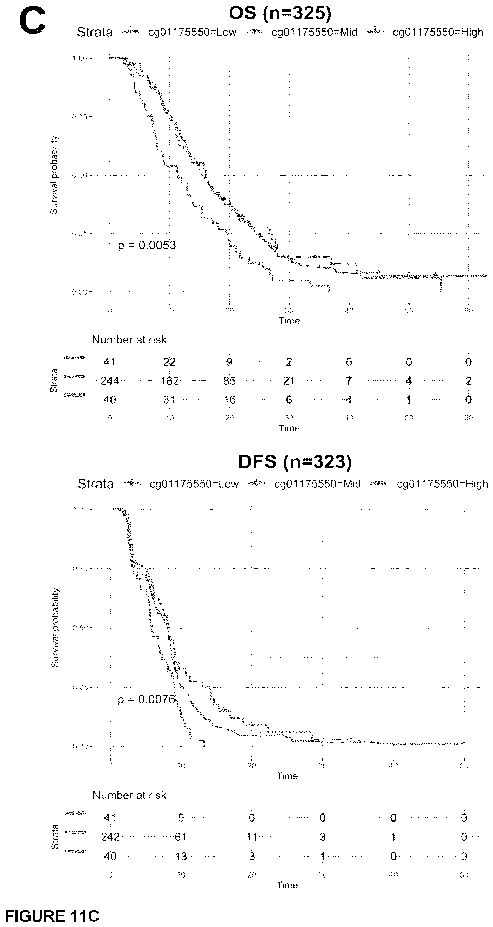

Figure 11A-C demonstrates stratification of prognostic CpGs in the FOCUS mCRC cohort. Figure 11A shows overlaps between CpGs identified in Cox univariate analysis with OS (overall survival) and DFS (disease free survival) as endpoints. Figure 11B shows a forest plot showing the five CpG sites identified in Cox univariate analysis commonly associated with OS and DF. Figure 11C shows Kaplan-Meier survival curves of mCRC patients stratified with low, medium or high methylation at cg01175550 with OS and DFS as endpoints.

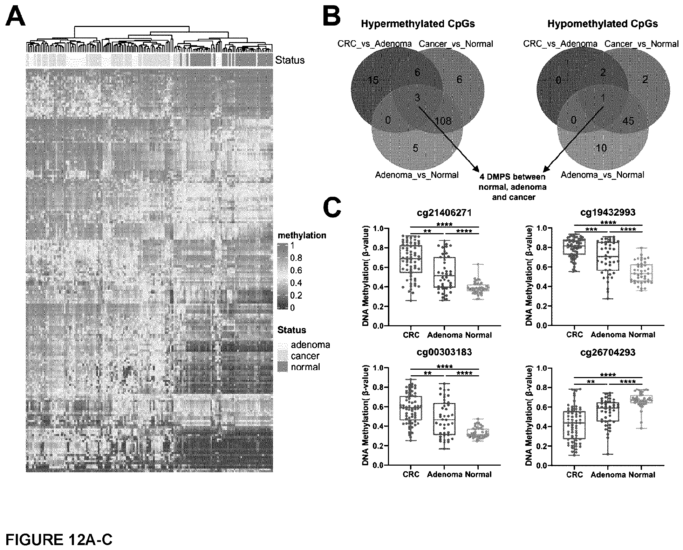

Figure 12A-C demonstrates a differential methylation analysis between normal, adenoma and CRC patients. Figure 12A shows a heatmap of methylation values of the 201 CpGs overlapping with the 376 DMRs in the GEO cohort (n=147). No other clinical or molecular variables were available for heatmap annotations. Figure 12B shows the number of significantly hypermethylated and hypomethylation CpGs in CRC vs normal, CRC vs adenoma and adenoma vs normal. Figure 12C shows boxplots of methylation at the four DMPs common to all three differential methylation analyses (p.adj ≤ 0.05).

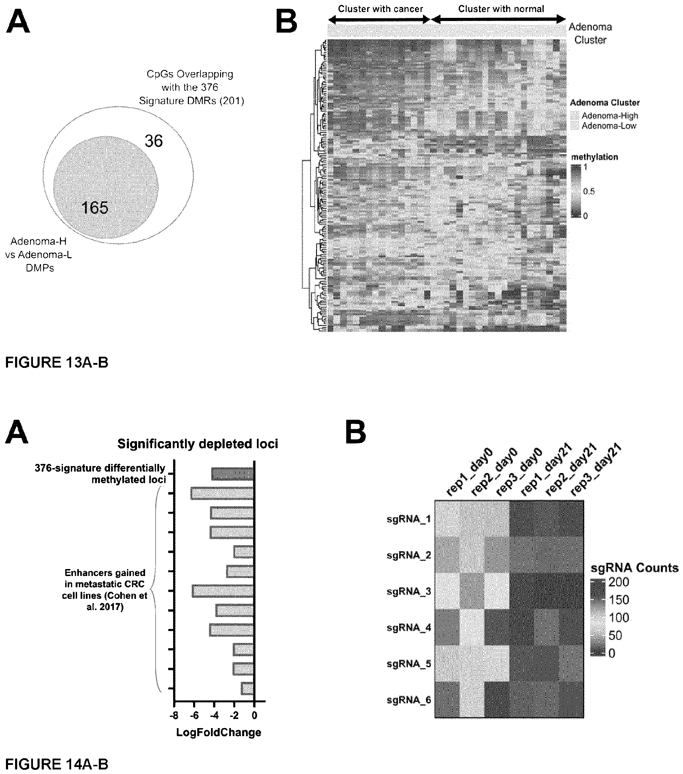

Figure 13A-B demonstrates differential methylation analysis between two adenoma subgroups. Figure 13A is an euler diagram showing of the number of CpGs differentially methylated between the two adenoma clusters which were identified through hierarchal clustering analysis. Figure 13B shows a heatmap for visualization of the DMPs. The cluster with predominantly hypermethylated DMPs was termed 'adenoma-H' while the second cluster was named 'adenoma-L'.
Figure 14A-B demonstrates the identification of regions of interest with the strongest effect on HCT116 proliferation. Figure 14A shows CRISPRi screen hits with a log2foldchange threshold of +/-1. The region shown in red (top bar) represents the only signature DMR with a log2foldchange within the specified threshold. Figure 14B shows a heatmap of all six sgRNAs targeting the depleted signature DMR on day 0 and day 21 of the screen.

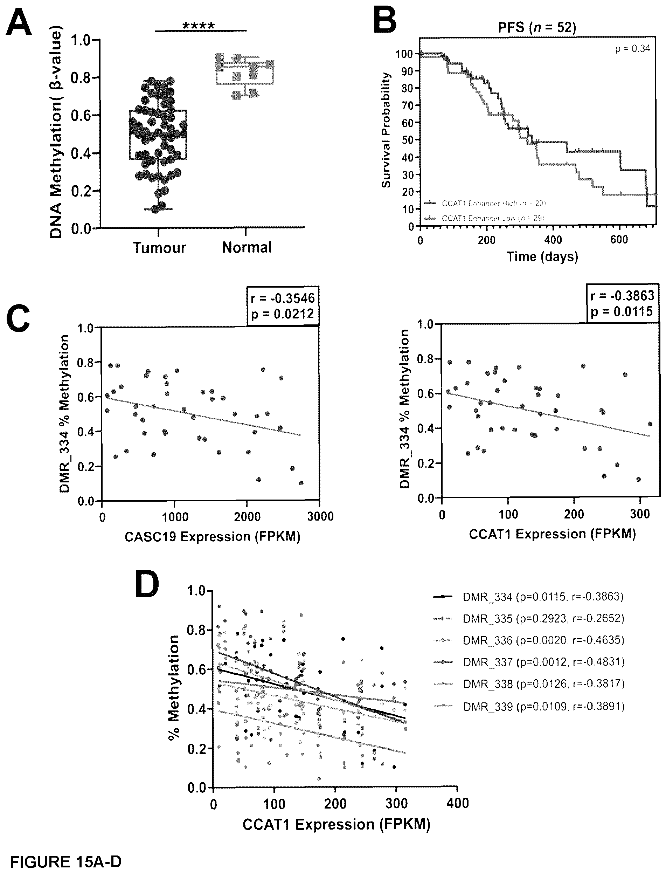

Figure 15A-D demonstrates the activity of the essential enhancer, DMR_334 in the Angiopredict mCRC cohort. Figure 15A shows DMR_334 β methylation levels in tumor and normal samples. Figure 15B shows a Kaplan-Meier survival curve of PFS probability of discovery cohort patients with high and low enhancer methylation, dichotomized by median methylation levels. Figure 15C shows a Pearson correlation of matched *CASC19* and *CCAT1* gene expression and DMR_334 methylation data from the Angiopredict mCRC cohort. The y-axis shows the β methylation value, while the x-axis shows the FPKM value. Figure 15D shows a Pearson correlation of *CCAT1* gene expression and all signature DMRs tiled alongside DMR_334.

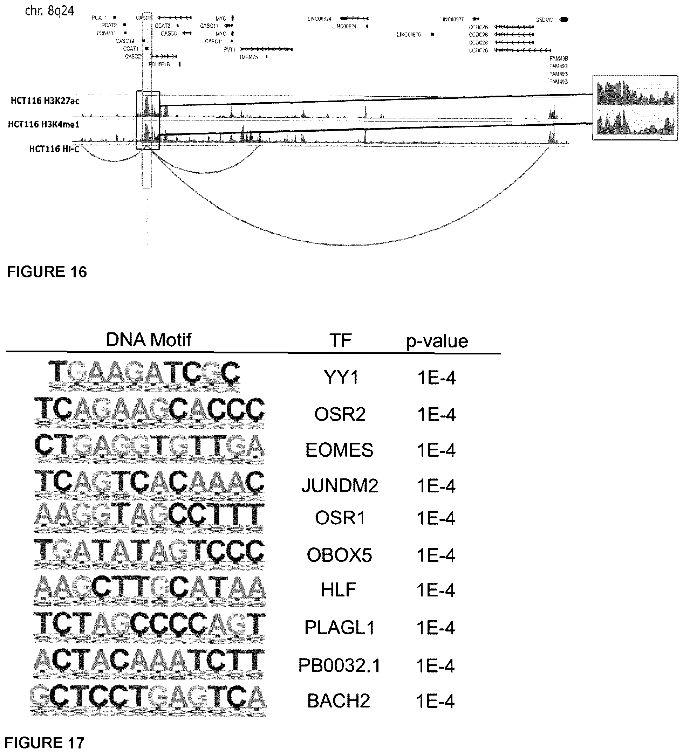

Figure 16 demonstrates 3D chromatin architecture and histone modifications present at the essential enhancer of interest (DMR_334). The yellow line surrounded by a red box indicates the enhancer of interest. Interactions obtained from HCT116 Hi-C data (GSE158007) are shown by the purple loops. ChlP-Seq peaks for H3K27ac and H3k4me1 in HCT116 cells are also shown (obtained from GSE77737). Data was visualized using the Washu Epigenome Browser.
Figure 17 demonstrates a TF motif analysis for the essential enhancer DMR_334. Selected motifs with the most significant enrichment are shown (p ≤ 0.05). For each, the forward consensus DNA binding sequence and predicted binding TF are shown.

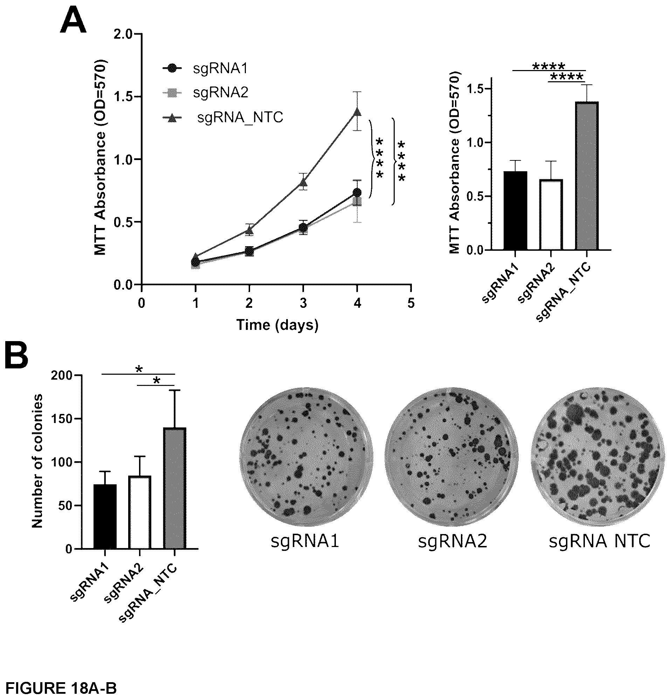

Figure 18 demonstrates CRISPRi-mediated repression of essential enhancer DMR_334 in HCT116 cells using individual sgRNAs. Figure 18A shows an MTT assay; following puromycin selection, an MTT assay was carried out at 24, 48, 72 and 96 hours on cells transfected with targeting and non-targeting control (NTC) sgRNAs. Significance was determined using a student's t-test on the 4 day timepoint only. (B) Colony formation assay of HCT116 enhancer knockdown cells 14 days post-selection. Representative images are shown. Error bars represent SEM. All *in vitro* experiments represent three independent experiments (n=3).

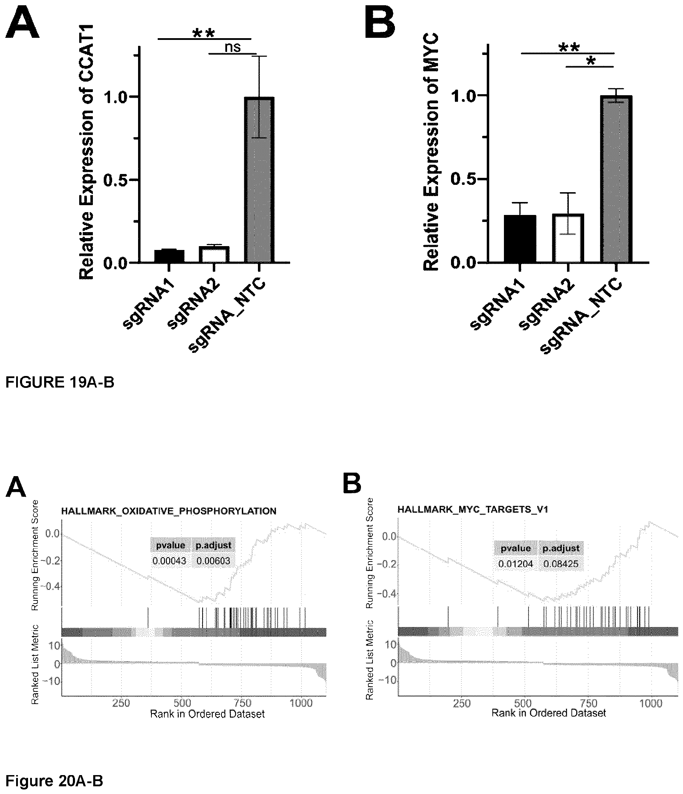

Figure 19 demonstrates alterations in *CCAT1* and *MYC* mRNA levels following enhancer repression. Two sgRNAs targeting the essential enhancer and a NTC sgRNA were transfected to HCT116 cells *via* lentiviral delivery. For days post selection, mRNA levels of (Figure 19A) *CCAT1* and (Figure 19B) *MYC* were detected by RT-qPCR. Results were normalized to *GAPDH* expression and samples were normalized to the NTC. Error bars represent SEM of three independent experiments (n=3). Significance was calculated using a student's t-test.
Figure 20 shows GSEA of hallmarks altered after enhancer knockdown using sgRNA1. Enrichment plots of (Figure 20A) *oxidative phosphorylation* and (Figure 20B) *MYC targets* (v1) hallmarks downregulated after enhancer repression. The enrichment score shown by the green line is calculated by GSEA and depicts the degree of over-representation of genes in the geneset at the top or bottom of the entire input ranked gene list.

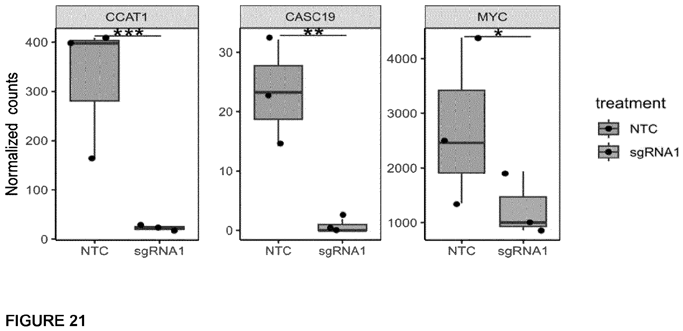

Figure 21 demonstrates mRNA expression of genes located proximally to the essential enhancer identified in RNA-Seq data. Boxplots of normalized counts of genes proximal to the enhancer. Significance values depict p-values before adjustment.

### Experimental Data

### Experiment 1 - Determination of the role enhancers in mCRC pathogenesis

To investigate the role of enhancers in mCRC pathogenesis, the present inventors assessed DNA methylation alterations in formalin fixed paraffin embedded tissue derived from retrospective clinical cohort of 55 stage IV mCRC primary tumours and 10 matched normal tissue samples using a targeted bisulfite sequencing approach. The targeted bisulfite sequence capture panel encompassed all the 376-loci contained within the DNA methylation signature of the present invention. These cohorts include stage III CRC patients who exhibit stable disease - "Non-progressors" (n=100) and stage III CRC patients who develop metastasis - "Progressors" (n=100). The inventors applied this capture panel to bisulfite converted DNA libraries generated from all tumour samples extracted from the two aforementioned cohorts using well-established experimental pipelines [17, 18]. Data generated was analysed using established bioinformatic pipeline, which allows generation of single-base pair resolution, high-confidence methylation calls across both strands of the DNA [17, 18, 20]. This led to the identification of a robust tumour-specific DNA methylation signature comprising of 376-differentially methylated loci (DMLs), which consisted predominantly (>90%) of gene enhancer regions (Figure 1). The inventors have ascertained the precise methylation status of each loci in stage IV mCRC patients (Figure 1).

The signature was further stratified into three distinct clusters (NMF1,2,3) that showed significant correlation with the recently published consensus molecular subtypes (CMS) in colorectal cancer [11, 12]. Using RNAsequencing data available for 48 out of the 55 mCRC patients, the present inventors determined the consensus molecular sub-types (CMS) present within this cohort. Interestingly, the present inventors showed that NMF1 and NMF2 clusters, as defined in the signature of the present invention, significantly overlapped (p=0.008, Fisher's-exact test) with CMS2 (Wnt-MYC-p53 signalling pathway sub-type) and CMS4 (Epithelial mesenchymal transition subtype), respectively (Figure 1). This data thus strongly suggests the likely impact of the novel DNA methylation signature of the present invention, or a subset thereof, on processes underpinning mCRC development.

### Experiment 2 - Determination if the loci can be used as a predictive approach to differentiate between mCRC tumour and normal samples

As a proof of concept, the inventors have evaluated a shallow artificial neural network-based (ANN) algorithm to the 376-loci methylation signature of the present invention (Figure 2) in order to understand if these loci can be used as a predictive approach to differentiate between mCRC tumour and normal samples within the same clinical cohort. The inventors used a two-layer feedforward neural network in combination with a sigmoid hidden layer and softmax output neurons. The network was trained by scale conjugate gradient backpropagation (Figure 2). These preliminary data show that the neural network demonstrated an accuracy of 98.5% in distinguishing tumour from normal samples.

### Experiment 3 - Application of the transcription factor binding analysis TRANSFAC to the two identified patient tumour clusters

The inventors further showed that applying this ANN approach to the DNA methylation signature of the present invention, generated three distinctive clusters - normal only cluster, patient tumour cluster 1, and patient tumour cluster 2 (Figure 3), with a >90% overlap with the NMF clusters identified using conventional clustering methods. It has been widely reported that the landscape of disease-associated alterations is largely orchestrated through modulation of transcription factors. To this end, application of the transcription factor binding analysis *TRANSFAC* (GeneXplain platform) [13, 14][1, 2] to the two patient tumour clusters, showed that these loci were enriched for transcription factor binding sites (TFBS) of key transcription factors such as HIF1-α or CREBP1 (p<0.001, over background). Intriguingly, this analysis showed that these TFBS are unique to each patient cohort, largely suggesting that there are likely different TF mediated functions that determine heterogeneity between the two patient cohorts (Figure 3).

### Experiment 4: Determination of the diagnostic potential of the novel DNA methylation signature

The DNA methylation signature consisting of 376 differentially methylated regions (DMRs) itself clusters patients into 3 distinct clusters (denoted as non-matrix factorization: NMF- 1, 2, 3). Below is a table (Table 2) that shows correlation of the cluster with clinical variables associated with the metastatic colorectal cancer (mCRC) patient cohort. Notably, here NMF2 and 3 cluster show significant correlation with widely known concensus molecular subtype (CMS) - CMS 2 and 4 respectively.

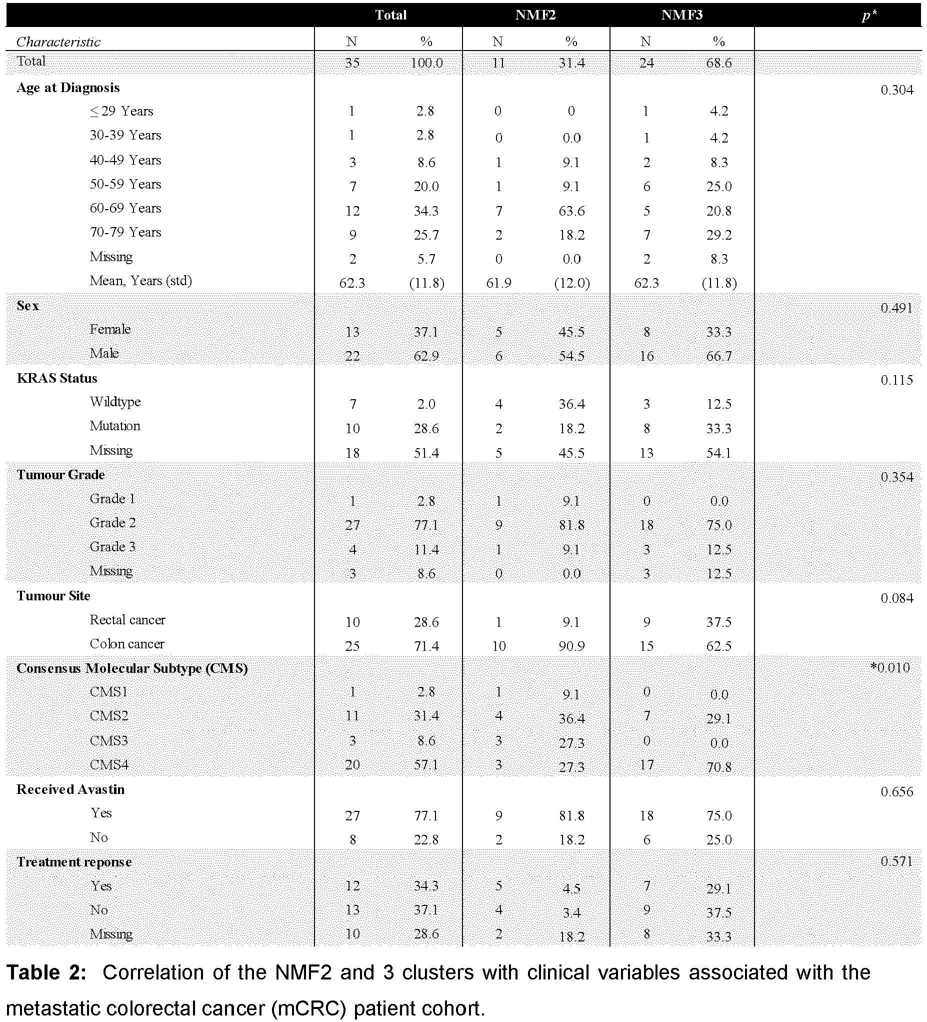

Subsequently, survival analysis was carried out between NMF2 and NMF3 patients with progression-free survival (PFS) as the endpoint. For this, full censoring was applied prior to analysis. Interestingly, it was found that NMF3 patients had a lower progression-free survival (PFS) probability compared to NMF2 patients (p = 0.028).

The inventors demonstrated that the NMF3 patient methylation cluster have significantly worse PFS compared to NMF2 (Figure 4).

### Experiment 5: Determination of the prognostic relevance of the novel DNA methylation signature

To investigate the prognostic relevance of the methylation signature of the present invention, univariate analysis was first carried out for all 376 DMR % methylation values with progression-free survival (PFS) as the endpoint. Once again, full censoring was applied and matched normal samples were removed from the analysis, as well as patients without survival data. Interestingly, 11 DMRs were significantly associated with PFS. Specifically, the hazard ratio (HR) was less than 1 for all DMRs, suggesting higher methylation at these loci is associated with a higher PFS. Further inspection revealed all 11 DMRs are hypomethylated in the mCRC patients compared to normal, suggesting this loss of methylation may be associated with a greater risk (Figure 5). Surprisingly, the DMR_302, identified as a gene enhancer, exhibited greatest association with PFS (p=0.001).

### Experiment 6: Stratification and validation of the best performing prognostic predictors

To further stratify and validate the best performing prognostic predictors, a LASSO-penalised regression model was constructed to again identify which of the 376 DMRs were most predictive of PFS (progression-free survival). LASSO is a popular technique in biomarker discovery and works by shrinking regression coefficients, thereby providing better predictions and preventing overfitting. Following LASSO analysis, two DMRs were found to promote the risk of poor PFS: namely DMR_302 and DMR_75, which were originally identified in univariate analysis (Figure 6A-B). Methylation of both DMRs was divided into three groups - low, medium and high methylation, to explore the effect on PFS. Survival analysis revealed that low methylation, i.e. hypomethylation, was significantly associated with lower PFS probability for DMR_302 (p=0.022) and DMR_75 (p= 0.049).

Given that DMR_302 and DMR_75 were both candidate predictors of PFS in the univariate and LASSO analysis, a new prediction model was built with the selected loci using linear regression. Receiver operating characteristic (ROC) curve generation indicated excellent accuracy in PFS prediction (AUC = 0.807) (Figure 7). Next, a risk score was generated for each mCRC patient based on β methylation at the two DMRs and regression coefficients previously obtained. To assess the impact of the combined risk score on mCRC patient PFS in comparison to other clinical variables, a univariate and multivariate analysis was performed. Univariate analysis indicated that the risk score was the strongest predictor (p=0.001) of PFS (Figure 7B). Compared to age, gender, tumor location and grade, only DMR risk score was significantly associated with PFS in multivariate analysis (p=0.001) (Figure 7C).

### Experiment 7 - Identification of loci with the greatest diagnostic utility

Having identified DMRs from the methylation signature associated with PFS, the inventors were next interested in identifying loci with greatest diagnostic utility, i.e. the DMRs best able to differentiate normal from tumour samples. To build the diagnostic model, the Angiopredict mCRC patients were first split into a training (70%) group, to train a random forest classifier, and a test group (30%) to evaluate how the model performs. Application of the model to the training set yielded an overall accuracy of 95.7% (Figure 8A) and an accuracy of 94.4% in the test set indicating excellent sample classification (Figure 8B). Next, the random forest algorithm was used to identify top important DMRs for diagnostic classification. DMRs with greatest mean decrease Gini scores, which represent predictor importance, are shown in Figure 8C, while DMRs with top mean decrease accuracy scores, which indicate greater ability of a DMR to reduce the model classification error, are shown in Figure 8D.

To evaluate the diagnostic accuracy of all 376 DMRs in the training and test groups, ROC curves were generated. In both groups, the signature DMRs had a similar AUC of 0.85 and 0.82 in the training and test group respectively (Figure 9A-B). As with the prognostic model, LASSO-penalised regression was next carried out to further pinpoint DMRs with the greatest diagnostic utility. In total, five DMRs were common to both the random forest and LASSO regression: DMR_240, DMR_236, DMR_148, DMR_276, DMR_33 (Figure 9C). Subsequently, a diagnostic prediction model was constructed based on the five DMRs and the resulting ROC curve had an AUC = 0.92, indicating the five DMR model predictions were 92% correct (Figure 9D).

### Experiment 8 - Identification of CpG sites commonly present between the microarrays and the signature of the present invention

It is important to state here that each of these 376-DMR's which are 500 bp in size indeed consists of multiple CpG sites, wherein the methylation alterations primarily occur. Unfortunately, no whole-genome bisulfite sequencing CRC datasets of adequate sample size were available at the time of this analysis so the inventors instead opted for 450K and EPIC array (both microarray platforms) derived CRC methylation datasets. To begin, the CpG sites present in the 376 DMRs were overlapped with 450K and EPIC array CpGs, revealing 197 CpGs commonly present between the microarrays and signature (Figure 10A). This translated to a coverage of 96 out of the 376 DMRs (25%) (Figure 10B).

### Experiment 9 - Evaluation of the prognostic role of overlapping CpG's

Although only 25% of the CpG's from within the 376-DMRs of the signature are covered on the EPIC/450K array, despite this, individual CpG sites have been well studied as biomarkers and are often regarded as better suited for examining the fraction of methylation in tissue⁽¹⁹⁾. Thus to evaluate the potential prognostic role of the 25% overlapping CpG's, in collaboration with the Stratification of Colorectal Cancer (S:CORT) consortium, the inventors utilized EPIC array methylation data from the de novo stage IV FOCUS cohort of patients (n=385) previously enrolled in the FOCUS clinical trial (OREC 15/EE/0241).

Univariate analysis revealed 34 CpGs significantly associated with OS (overall survival) and 10 CpGs associated with DFS (disease free survival) in the mCRC cohort (Figure 11A). Amongst these, 5 CpGs were commonly associated with both OS and DFS, namely *cg01775550, cg19308222, cg06531379, cg10248357, cg06481168* (Figure 11A-B). LASSO-penalised regression analysis of all CpG sites thus identified CpG loci: *cg01175550* as the best predictor of OS and DFS which is located within DMR_312. In the FOCUS cohort, low methylation at cg01175550 was significantly associated with lower OS (p=0.005) and lower DFS (p=0.007) (Figure 11C).

### Experiment 10 - Determination if CpGs are differentially methylated between normal, adenoma and CRC patients

The inventors next explored whether any of the CpGs from the 376-DMRs are differentially methylated between the three stages of normal, adenoma and CRC, for potential early diagnostic biomarker discovery. To that end, the inventors utilized 450K array methylation data from the GEO dataset GSE48684, which consisted of 41 normal, 42 adenoma and 64 CRC patients. First, the inventors derived the subset methylation data for CpGs overlapping between the 450K array and the 376 DMRs: 201 CpGs in total (Figure 12A). Next, using the ChaMP R package, differential methylation analysis was carried out between normal, adenoma and CRC patients. ChAMP is one of the most widely used pipelines for analysis of Illumina microarray data, and calculates differential methylation using linear model for microarray data (LIMMA) functions⁽²⁰⁾. The analysis revealed that 86% of the 201 CpGs were confirmed as differentially methylated between normal and CRC (p.adj ≤ 0.05). Specifically, 123 CpGs were hypermethylated and 50 were hypomethylated. Additionally, 116 CpGs were hypermethylated and 56 CpGs were hypomethylated between adenoma and normal. A smaller percentage of sites were differentially methylated between CRC and adenoma, with 24 hypermethylated and 3 hypomethylated (Figure 12B). Interestingly, 4 differentially methylated positions (DMPs) were found between the three analyses, namely cg21406271, cg19432993, cg00303183 and cg26704293. This included 3 hypermethylated and 1 hypomethylated CpG (Figure 12C). Cumulatively, the results suggest that even at a single DMP level, the vast majority of CpGs present in the 376 DMRs exhibit differential methylation between normal and CRC, and some of these may become more hypomethylated or hypermethylated in the progression of normal to adenoma to CRC.

### Experiment 11 - To determine if any of the 201 CpGs overlapping with the 376 DMRs differentiate between adenoma-H and adenoma-L subgroups

The inventors noticed that the patient hierarchal clustering performed for heatmap visualization contained adenoma patients that clustered with both CRC and others that clustered with normal. One landmark study, that also utilized this dataset, identified two subgroups of adenoma patients with contained varying methylation profiles, termed adenoma-high (adenoma-H) and adenoma-low (adenoma-L)⁽²¹⁾. To address this question, the inventors carried out clustering of the adenoma patients, followed by differential methylation analysis to explore whether any of the 201 CpGs overlapping with the 376 DMRs differentiate between adenoma-H and adenoma-L subgroups. Following this, the inventors found that 165 out of the 201 CpGs (82%) were differentially methylated between both subgroups (Figure 13A) including 120 hypermethylated CpGs and 45 hypomethylated CpGs in adenoma-H compared to adenoma-L patients (p.adj ≤ 0.05). Given that the majority of the 201 CpGs overlapping with the 376 DMRs are hypermethylated in CRC, the inventors identified adenoma-H as the subgroup that clustered with CRC patients, while, adenoma-L patients clustered with normal (Figure 13B). Overall, these findings suggest that methylation alteration at several DMPs from within the signature of the present invention identified in patients with adenoma-H polyps may enact as predictive precursors to CRC.

### Experiment 12 - Demonstration of the therapeutic potential of the novel DNA signature of the present invention

In order to demonstrate the therapeutic potential of the novel DNA methylation signature of the present invention, the inventors carried out a CRISPRi *loss-of-function* (LOF) screen in a HCT116 cell line and identified several loci whose perturbation impacted growth of this specific cell line. From the 2,274 genomic regions of interest included in the CRISPRi screen, 31 regions significantly essential for HCT116 proliferation were identified (p.adjust ≤ 0.05). Importantly, these included 13 depleted regions, which following CRISPR-mediated silencing caused a significant decrease in HCT116 proliferation and 18 whose silencing resulted in a significant increase in HCT116 proliferation.

Specifically, these 31 essential regions encompassed: one gene promoter, one p53-binding site and 29 enhancers. Of the 29 essential enhancers, three were derived from the 376-DMR signature of the present invention, while 17 and 7 variable enhancer loci (VELs) were gained and lost respectively in mCRC cell lines as well one enhancer was a VEL lost in all CRC cell lines and one enhancer was identified as a VEL gained in all CRC cells.

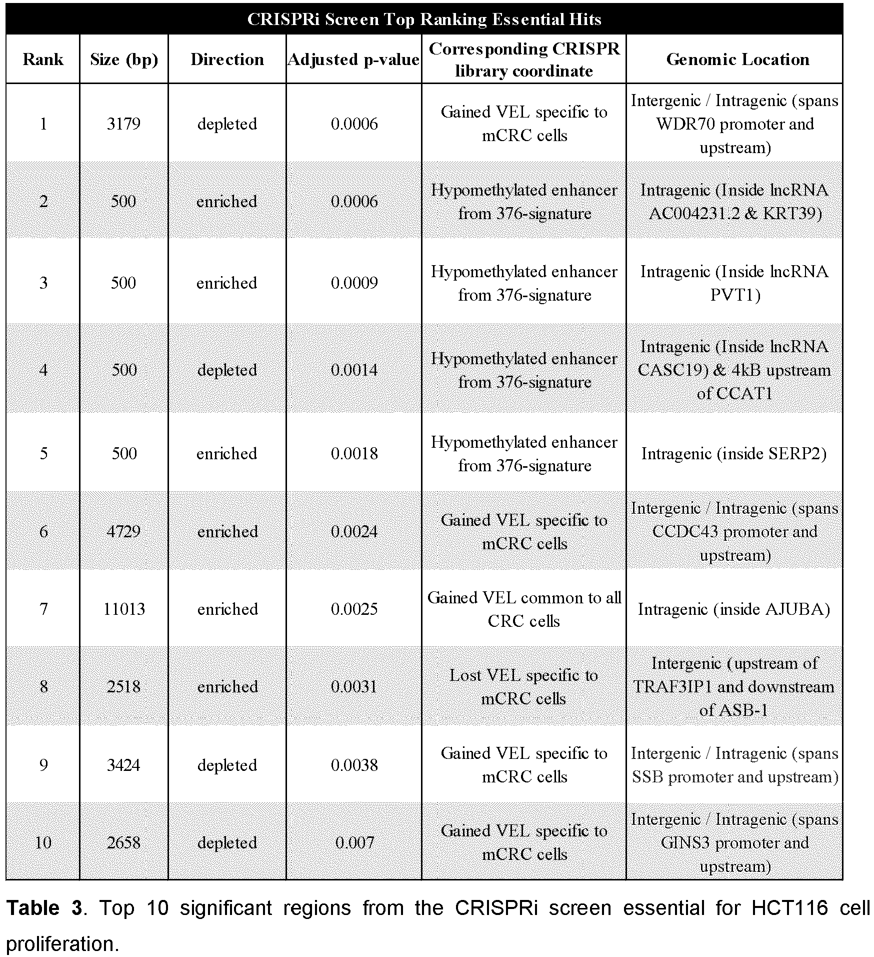

### Experiment 13 - Determination of regions likely to have the strongest effects on HCT116 cell proliferation

To select regions likely to have the strongest effects on HCT116 cell proliferation, the inventors proceeded with regions that decreased or increased proliferation by ≥ 1-fold. Intriguingly, only the 13 depleted regions remained after filtering (Figure 14A). This included one DMR from the methylation signature, which ranked as the 2^{nd} highest significantly depleted loci in the CRISPR screen (p=0.001, log2foldchange=-4.3). Furthermore, all six targeting sgRNAs were significantly depleted at day 21 for this DMR, which was identified as a gene enhancer (Figure 14B). In summary, multiple enhancers essential for HCT116 proliferation were identified using a CRISPRi LOF screen, which warrant further investigation to elucidate their function. As this project was based on a 376-DMR methylation signature, we proceeded with the sole depleted essential enhancer DMR for further examination.

### Experiment 14 - investigation of the function of the essential signature enhancer DMR 334

To further understand the function of the essential signature enhancer (DMR_334) identified from the CRISPRi screen, we returned to data generated from the mCRC discovery cohort described earlier, which was used to identify the original 376-DMR signature. It was found that this particular enhancer, which is located on chromosome 8, is hypomethylated in the primary tumour of mCRC patients in comparison to normal colon samples (q=2.2e-5) (Figure 15A). While patients with lower levels of methylation did not appear to have significantly different (progression-free survival) PFS in comparison to those with higher methylation levels (p=0.34) (Figure 15B), loss of methylation was associated with higher expression of two genes identified from proximity-based correlation analysis. This included *CASC19*, which has multiple isoforms in the vicinity of the enhancer (p=0.02) and *CCAT1*, which is approximately 4kb upstream of the enhancer (p=0.01) (Figure 15C). Furthermore, it is highly likely that the enhancer is a part of a much larger super-enhancer, given that DMRs were originally tiled to only 500bp for identification. Without wishing to be bound by theory, the inventors submit this for two reasons. Firstly, hypomethylation at five other DMRs located directly beside the enhancer of interest was additionally associated with a significant increase in *CCAT1* expression (Figure 15D) and secondly because two previous studies have identified a super-enhancer spanning this 500bp region in CRC cell lines and patients.

Further examination of patients with higher or lower methylation levels at the enhancer revealed that patients with low methylation at this loci were predominantly from the NMF3 cluster (p=0.007). While no significant relationship between enhancer methylation levels and CMS were found, a trend of CMS4 patients having low enhancer methylation levels was noted (p=0.08) (Table 4).

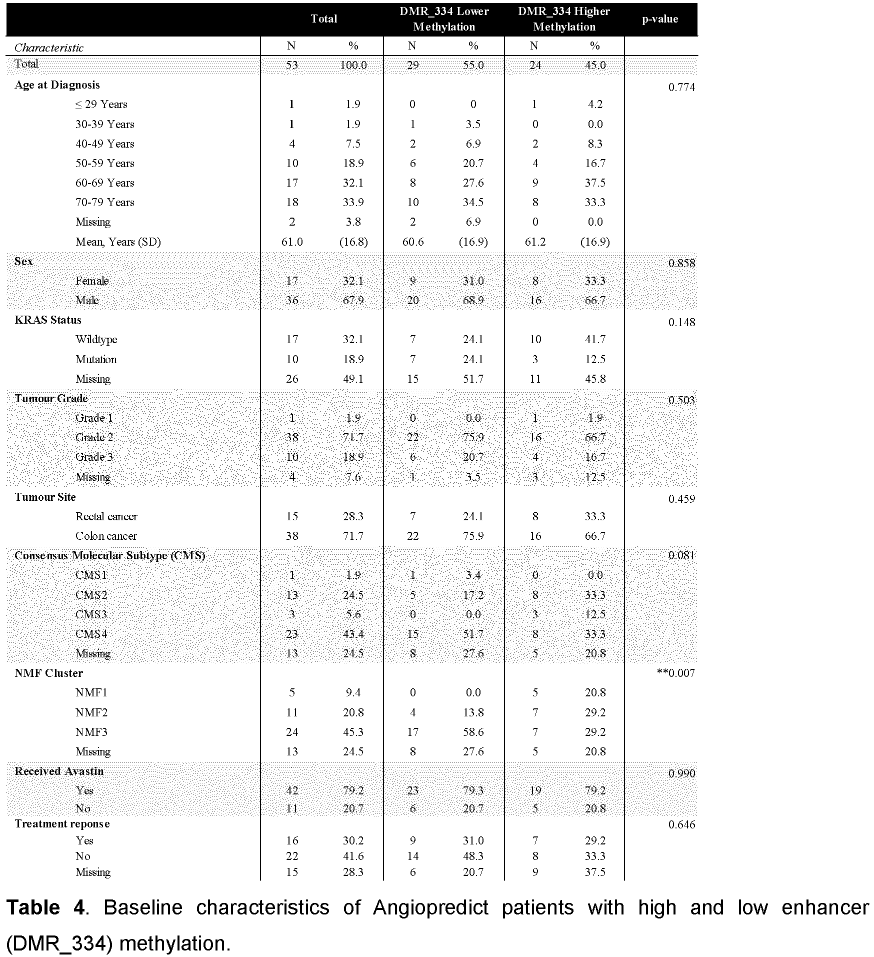

### Experiment 15 - Determination of the the role of the enhancer DMR 334 in mCRC

To further elucidate the role of the enhancer DMR_334 in mCRC, the inventors investigated specific histone marks of active enhancers within the chr.8q24 region which contains the enhancer region of interest. Intriguingly, Chr8.q24 is commonly known as a "*gene desert*" of non-coding transcriptional activity, with many genes in this region conferring susceptibility to different cancer types. sing publicly available HCT116 ChlP-Seq data for H3K27ac and H3K4me1 marks (GSE77737) the inventors found that both histone mark levels are enriched specifically at this enhancer region, suggesting that the enhancer is highly active in mCRC (Figure 16).

To identify potential genes directly interacting with the enhancer through enhancer-promoter loops, the inventors also examined HCT116 Hi-C data Unfortunately, no consistent interactions were found among either three or two biological replicates, however it is noted that interactions with *TMEM75*, *CCDC26* and *PVT1* were observed in one replicate as shown in Figure 16. Through use of the UCSC Genome Browser, the inventors found that the 500bp enhancers contains two SNPs; rs3839855 and rs35813501. Only rs35813501 has been associated with breast cancer risk previously.

### Experiment 16 - Determination of the TF binding motifs enriched within the DMR 334 enhancer

To determine the TF binding motifs enriched within the 500bp DMR_334 enhancer, the inventors utilized query motif predictions previously generated, using Homer software. Overall, 30 significantly enriched motifs were located within this enhancer site. This included Yin Yang-1 (YY1), which was the top TF predicted to bind to this enhancer site (p=1e-4). YY1 is highly expressed in CRC and, despite controversial roles, is considered as an oncogenic TF (Figure 17). Similarly, Odd-Skipped Related TF 2 (ORS2) was the 2^{nd} ranking predicted motif.

Together the results from *in silico* analysis suggest that the enhancer DMR that caused a profound reduction in HCT116 cell proliferation upon repression, is a highly active hypomethylated enhancer that is part of a much larger partially differentially methylated super-enhancer. Furthermore, the enhancer is associated with *CCAT1* and CASC19 expression and enriched for multiple oncogenic TFs.

### Experiment 17 - Validation of the anti-proliferative effect on the DMR 334 enhancer observed upon enhancer repression

In order to validate the anti-proliferative effect observed upon enhancer repression, the inventors next selected two individual sgRNAs to knockdown the DMR_334 enhancer using CRISPRi. The HCT116-dCas9-KRAB-MeCP2 cell line was subsequently transfected with lentivirus transiently expressing each individual sgRNA. After puromycin selection was complete, MTT viability assays were performed at 24 hr intervals for four days to compare proliferation between targeting sgRNAs and NTC. Successful knockdown was confirmed by death of all cells transfected with the TC sgRNA. After four days, potent inhibition of HCT116 proliferation was induced by targeting sgRNA1 (p=1.8e-7) and sgRNA2 (p=9.7e-8) compared to the sgRNA NTC (Figure 18A). To examine the impact of enhancer repression on anchorage independent growth, colony formation assays were also carried out. The number of HCT116 colonies was significantly lower in cells transfected with targeting sgRNA1 (p=0.02) and sgRNA2 (p=0.01) compared to the NTC (Figure 18B). Importantly, for both phenotypic experiments, no significant difference between each targeting sgRNA was found.

### Experiment 18 - Examination of the expression levels of the DMR 334 gene after 4 days of enhancer repression

As *CCAT1* is the most proximal gene to the DMR_334 enhancer of interest, and correlation analysis suggested that expression of this gene is associated with hypomethylation at this region, the inventors examined the expression levels of this gene after 4 days of enhancer repression. Enhancer silencing using sgRNA1 resulted in potent inhibition of *CCAT1* expression (p=0.003), while a trend in *CCAT1* downregulation was noted with sgRNA2 (p=0.09) (Figure 19A). As *MYC* transcription is knowingly regulated by long-range chromatin interactions with *CCAT1*, the expression of this gene was also examined. The inventors found that upon enhancer repression, both sgRNA1 (p=0.004) and sgRNA2 (p=0.012) induced significant downregulation of *MYC* expression (Figure 19B). In summary, silencing of the essential enhancer (DMR_334) in HCT116 cells using two alternative sgRNAs resulted in potent inhibition of HCT116 proliferation, colony formation ability and expression of known oncogenes *CCAT1* and *MYC*, suggesting potential utility of the enhancer as a therapeutic target for CRC.

### Experiment 19 - Evaluating the effect of the essential enhancer (DMR 334) on the CRC transcriptome

To further understand the effect of the essential enhancer (DMR_334) identified and validated from the CRISPRis screen on the CRC transcriptome, the inventors performed RNA-Seq on HCT116 cells four days after repression of the enhancer. Differential gene expression analysis next revealed that enhancer silencing using sgRNA1 resulted in 1,096 differentially expressed genes (DEGs), while unexpectedly only 16 genes were significantly differentially expressed by sgRNA2 in comparison to the NTC sgRNA (p.adjust ≤ 0.05). In total, 8 genes were commonly upregulated by sgRNA1 and sgRNA2 and 4 genes were commonly downregulated (Table 4). Interestingly, the 12 DEGs common to both sgRNAs harbored diverse functions including two solute carrier family genes: SLC2A1 and *SLC1A3.* SLC family genes, which encode membrane transfer proteins, are involved in glycometabolism and are often dysregulated in cancers such as CRC.

**Table 5. Genes differentially expressed following enhancer repression in HCT116 cells common to both targeting sgRNAs.**

| **Gene** | **Gene Name** | **Function** | **Direction** |
|---|---|---|---|
| PLEKHA2 | Pleckstrin Homology Domain Containing A2 | positive regulation of cell-matrix adhesion | Upregulated |
| DYNLL2 | Dynein Light Chain LC8-Type 2 | dynein light intermediate chain binding activity | Upregulated |
| SLC2A1 | Solute Carrier Family 2 Member 1 | glucose transporter | Upregulated |
| ISPD | CDP-L-Ribitol Pyrophosphorylase A | Encodes Isoprenoid synthase domain-containing protein | Upregulated |
| KLK10 | Kallikrein Related Peptidase 10 | Diverse functions - carcinogenesis | Upregulated |
| CEMIP | Cell Migration Inducing Hyaluronidase 1 | clathrin heavy chain / hyaluronic acid binding activity and protein phosphorylation | Upregulated |
| SUSD2 | Sushi Domain-Containing Protein 2 | negative regulation of cell cycle G1/S phase transition and division | Upregulated |
| KRT13 | Keratin 13 | Epithelial cell structural integrity | Upregulated |
| NFIA | Nuclear Factor I A | Encodes NF family transcription factors | Downregulated |
| ST6GAL1 | ST6 Beta-Galactoside Alpha-2,6-Sialyltransferase 1 | encodes glycosyltransferase family 29 member | Downregulated |
| ACSM3 | Acyl-CoA Synthetase Medium Chain Family Member 3 | Ulcerative colitis biomarker | Downregulated |
| SLC1A3 | Solute Carrier Family 1 Member 3 | Amino acid and glutamate binding | Downregulated |

### Experiment 20 - Investigation of gene pathway alterations induced by sgRNA1 only

Given the variance in sgRNA2 replicates, coupled with the lack of genes significantly differentially expressed following enhancer repression with this guide, the inventors decided to proceed with investigation of gene pathway alterations induced by sgRNA1 only. From the DEG list, it is known that many genes altered by sgRNA1 were also altered by sgRNA2, however, these did not reach significance after p-value adjustment for sgRNA2, likely due to the stronger effect of sgRNA1 in comparison.

For a more focused understanding of the genome-wide alterations induced by DMR_334 repression, the inventors investigated whether any hallmark genesets were dysregulated. The hallmark collection was developed by the Molecular Signatures Database (MSigDB) in order to provide a refined delineation of biological processes for use with Gene Set Enrichment Analysis (GSEA) software. While no positively regulated hallmarks were identified, interestingly however genes downregulated by sgRNA1 displayed significant enrichment for *oxidative phosphorylation* (p.adjust=0.006) (Figure 20A). Furthermore, the inventors also noted a trend in downregulation of *MYC* target genes (p.adjust=0.08) despite not reaching significance (Figure 20B).

### Experiment 21 - Examination of the RNA-Seq DEG lists to explore the regulation of MYC and any other genes proximal to the enhancer

As MYC targets (v1) was noted as the second ranking enriched hallmark, the inventors examined the RNA-Seq DEG lists to explore the regulation of MYC and any other genes proximal to the enhancer on chromosome 8. From this, a trend in reduced expression of *CASC19*, *CCAT1* and *MYC* was observed upon knockdown with sgRNA1, however, these were only significant before p-value adjustment (Figure 21).

Various modifications and variations to the described embodiments of the inventions will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes of carrying out the invention which are obvious to those skilled in the art are intended to be covered by the present invention.

### References

1.Colorectal Cancer Facts & Figures 2017-2019. In. Atlanta, Ga: American Cancer Society; 2017: 2017.
2.Biller LH, Schrag D. Diagnosis and Treatment of Metastatic Colorectal Cancer: A Review. JAMA 2021.
3.Cancer Genome Atlas N. Comprehensive molecular characterization of human colon and rectal cancer. Nature 2012.
4. Smeets D et al.,Copy number load predicts outcome of metastatic colorectal cancer patients receiving bevacizumab combination therapy. Nat Commun 2018.
5. van Dijk E et al., Loss of Chromosome 18q11.2-q12.1 Is Predictive for Survival in Patients With Metastatic Colorectal Cancer Treated With Bevacizumab. J Clin Oncol 2018.
6. Brenner H et al.,. Colorectal cancer. Lancet 2014.
7. Lordick F. Changing paradigms in adjuvant treatment of colorectal cancer. Lancet Gastroenterol Hepatol 2018.
8. Loberg M, Holme O, Bretthauer M, Kalager M. Colorectal adenomas, surveillance, and cancer. Lancet Oncol 2017; 18: e427.
9. Dy GK et al., Long-term survivors of metastatic colorectal cancer treated with systemic chemotherapy alone: a North Central Cancer Treatment Group review of 3811 patients, N0144. Clin Colorectal Cancer 2009.
10. Hu Z, Tee WW. Enhancers and chromatin structures: regulatory hubs in gene expression and diseases. Biosci Rep 2017;37.
11. Dienstmann R et al.,Consensus molecular subtypes and the evolution of precision medicine in colorectal cancer. Nat Rev Cancer 2017; 17: 79-92.
12. Guinney J et al., The consensus molecular subtypes of colorectal cancer. Nat Med 2015.
13. Matys V et al.,TRANSFAC and its module TRANSCompel: transcriptional gene regulation in eukaryotes. Nucleic Acids Res 2006.
14. Wingender E et al., TFClass: expanding the classification of human transcription factors to their mammalian orthologs. Nucleic Acids Res 2018.
15. Eberhard J et al., A cohort study of the prognostic and treatment predictive value of SATB2 expression in colorectal cancer. Br J Cancer 2012.
16. Iwaya M et al., Colitis-associated colorectal adenocarcinomas are frequently associated with non-intestinal mucin profiles and loss of SATB2 expression. Mod Pathol 2019.
17. Ward E et al., Epigenome-wide SRC-1-Mediated Gene Silencing Represses Cellular Differentiation in Advanced Breast Cancer. Clin Cancer Res 2018.
18. Moran B, Das S et al., Assessment of concordance between fresh-frozen and formalin-fixed paraffin embedded tumor DNA methylation using a targeted sequencing approach. Oncotarget 2017.
19. Tsai PC, Bell JT. Power and sample size estimation for epigenome-wide association scans to detect differential DNA methylation. Int J Epidemiol 2015.
20. Vareslija D, et al., Transcriptome Characterization of Matched Primary Breast and Brain Metastatic Tumors to Detect Novel Actionable Targets. J Natl Cancer Inst 2018.
21. Sokolov A et al.,Pathway-Based Genomics Prediction using Generalized Elastic Net. PLoS Comput Biol 2016.
22. Luo H et al., Circulating tumor DNA methylation profiles enable early diagnosis, prognosis prediction, and screening for colorectal cancer. Sci Transl Med 2020.
23. Sui J et al., Discovery and validation of methylation signatures in blood-based circulating tumor cell-free DNA in early detection of colorectal carcinoma: a case-control study. Clin Epigenetics 2021.
24. Jin S et al., Efficient detection and post-surgical monitoring of colon cancer with a multi-marker DNA methylation liquid biopsy. Proc Natl Acad Sci U S A 2021.
25. Bi F et al., Circulating tumor DNA in colorectal cancer: opportunities and challenges. Am J Transl Res 2020.

## Claims

1. A method for predicting the progression of colorectal cancer in a subject and/or screening for an increased likelihood of progression of colorectal cancer in a subject, the method comprising:
- providing a biological sample which has been obtained from the subject;
- testing the biological sample for the presence or absence of a gene expression signature, or a subset thereof;
wherein the gene expression signature, or a subset thereof, comprises or consists of one or more of the differentially methylated regions set forth in Table 1;
wherein the presence of the gene expression signature, or a subset thereof, indicates an increased likelihood of disease progression.

2. The method of claim 1, wherein the one or more differentially methylated regions comprise or consist of the nucleic acid sequences of SEQ ID NO:1 to SEQ ID NO:367 or a functionally active fragment or variant thereof; or any combination thereof.

3. The method of claim 1 or claim 2, wherein the one or more differentially methylated regions are hypomethylated.

4. The method of claim 1 or 2, wherein the gene expression signature comprises or consists of one or more differentially methylated regions selected from the group comprising or consisting of DMR_240, DMR_236, DMR_148, DMR_276, DMR_33, DMR_312, DMR_302, DMR_75 or DMR_334 or any combination thereof.

5. The method of claim 4, wherein
(i) the differentially methylated region DMR_240 comprises or consists of the nucleic acid sequence of SEQ ID NO:240 or a functionally active fragment or variant thereof;
(ii) the differentially methylated region DMR_236 comprises or consists of the nucleic acid sequence of SEQ ID NO:236 or a functionally active fragment or variant thereof;
(iii) the differentially methylated region DMR_148 comprises or consists of the nucleic acid sequence of SEQ ID NO: 148 or a functionally active fragment or variant thereof;
(iv) the differentially methylated region DMR_276 comprises or consists of the nucleic acid sequence of SEQ ID NO:276 or a functionally active fragment or variant thereof;
(v) the differentially methylated region DMR_33 comprises or consists of the nucleic acid sequence of SEQ ID NO:33 or a functionally active fragment or variant thereof;
(vi) the differentially methylated region DMR_312 comprises or consists of the nucleic acid sequence of SEQ ID NO:312 or a functionally active fragment or variant thereof;
(vii) the differentially methylated region DMR_302 comprises or consists of the nucleic acid sequence of SEQ ID NO:302 or a functionally active fragment or variant thereof;
(viii) the differentially methylated region DMR_75 comprises or consists of the nucleic acid sequence of SEQ ID NO:75 or a functionally active fragment or variant thereof; or
(ix) the differentially methylated region DMR_334 comprises or consists of the nucleic acid sequence of SEQ ID NO:334 or a functionally active fragment or variant thereof.

6. The method of claim 4, wherein the one or more differentially methylated regions comprise or consist of DMR_240, DMR_236, DMR_148, DMR_276 and/or DMR_33 or combinations thereof and wherein,
(i) the differentially methylated region DMR_240 comprises or consists of the nucleic acid sequence of SEQ ID NO:240 or a functionally active fragment or variant thereof.
(ii) the differentially methylated region DMR_236 comprises or consists of the nucleic acid sequence of SEQ ID NO:236 or a functionally active fragment or variant thereof.
(iii) the differentially methylated region DMR_148 comprises or consists of the nucleic acid sequence of SEQ ID NO:148 or a functionally active fragment or variant thereof.
(v) the differentially methylated region DMR_276 comprises or consists of the nucleic acid sequence of SEQ ID NO:276 or a functionally active fragment or variant thereof and/or
(vi) the differentially methylated region DMR_33 comprises or consists of the nucleic acid sequence of SEQ ID NO:33 or a functionally active fragment or variant thereof.

7. The method of claim 4, wherein the one or more differentially methylated regions comprise or consist of DMR_302, DMR_75 and/or DMR_312 or combinations thereof and wherein
(i) the differentially methylated region DMR_302 comprises or consists of the nucleic acid sequence of SEQ ID NO:302 or a functionally active fragment or variant thereof;
(ii) the differentially methylated region DMR_75 comprises or consists of the nucleic acid sequence of SEQ ID NO:75 or a functionally active fragment or variant thereof;
(iii) the differentially methylated region DMR_312 comprises or consists of the nucleic acid sequence of SEQ ID NO:312 or a functionally active fragment or variant thereof.

8. One or more biomarkers for use in vitro diagnosis of colorectal cancer or for predicting the likelihood of colorectal cancer progressing to metastatic colorectal cancer, wherein the biomarkers comprise or consist of the nucleic acid sequences of the one or more differentially methylated regions listed in Table 1.

9. The one or more biomarkers as claimed in claim 8, wherein the biomarkers comprise or consist of one or more of the nucleic acid sequences of SEQ ID NO:1 to 376 or a functionally active fragment or variant thereof.

10. The one or more biomarkers of claims 8 or 9, wherein the one or more differentially methylated region is DMR_334, DMR_312, DMR_302, DMR_75 or combinations thereof.

11. The one or more biomarkers of claims 8 to 10, wherein the nucleic acid sequence of one or more differentially methylated regions is hypomethylated.

12. The one or more biomarkers of claims 8 or 9, wherein the one or more biomarkers comprise or consist of all of the hypomethylated differentially methylated regions in Table 1.

13. A method of screening for the presence of or diagnosing colorectal cancer in a subject, comprising
(a) obtaining a biological sample from a subject; and
(b) determining a methylation status of one or more differential methylated regions (DMR) selected from the group listed in Table 1;
wherein an alteration in methylation of said DMR in said sample relative to the level of methylation of said DMR in normal colorectal cells in indicative of colorectal cancer in said subject.

14. A biomarker for use in the treatment of colorectal cancer, wherein the biomarker comprises or consists of one or more differential methylated regions (DMR) selected from the group listed in Table 1; optionally wherein the biomarker comprises or consists of one or more of the nucleic acid sequences of SEQ ID NO:1 to 376 or a functionally active fragment or variant thereof.

15. The biomarker of claim 14, wherein the differentially methylated region is DMR_334 and wherein the differentially methylated region DMR_334 comprises or consists of the nucleic acid sequence of SEQ ID NO:334 or a functionally active fragment or variant thereof.
